# EUROPEAN PATENT SPECIFICATION

(11) **EP 2 513 313 B1**
(45) Date of publication and mention of the grant of the patent: **11.02.2015**
(21) Application number: 10796348.0
(22) Date of filing: 14.12.2010
(51) Int. Cl.: C12N 15/62, C07K 14/50, C12N 9/48, A61K 38/00

(54) **Substances and methods for the treatment of lysosomal storage diseases**
Substanzen und Verfahren zur Behandlung von lysosomalen Speicherkrankheiten
Substances et procédés pour le traitement de maladies liées au stockage lysosomal

(30) Priority: 14.12.2009 EP 09179047
(43) Date of publication of application: 24.10.2012
(73) Proprietor: Steinfeld, Robert, 37077 Göttingen (DE)
(72) Inventor: Steinfeld, Robert, 37077 Göttingen (DE)
(74) Representative: Vossius & Partner
(86) International application number: PCT/EP2010/069649
(87) International publication number: WO 2011/073199

(56) References cited:
- WO-A2-2007/014156
- WO-A2-2007/091159
- WO-A2-2008/049058
- US-B1- 6 302 685
- REILAND JANE ET AL: "Heparan sulfate proteoglycan and FGF receptor target basic FGF to different intracellular destinations", JOURNAL OF CELL SCIENCE, vol. 105, no. 4, 1993, pages 1085-1093, XP002621758, ISSN: 0021-9533
- GOLABEK ADAM A ET AL: "Glycosaminoglycans modulate activation, activity, and stability of tripeptidyl-peptidase I in vitro and in vivo", JOURNAL OF BIOLOGICAL CHEMISTRY, vol. 280, no. 9, March 2005 (2005-03), pages 7550-7561, XP002621759, ISSN: 0021-9258
- JANS DAVID A: "Nuclear signaling pathways for polypeptide ligands and their membrane receptors?", FASEB JOURNAL, vol. 8, no. 11, 1994, pages 841-847, XP002621757, ISSN: 0892-6638
- GUHANIYOGI JAYITA ET AL: "Crystal Structure and Autoactivation Pathway of the Precursor Form of Human Tripeptidyl-peptidase 1, the Enzyme Deficient in Late Infantile Ceroid Lipofuscinosis", JOURNAL OF BIOLOGICAL CHEMISTRY, vol. 284, no. 6, February 2009 (2009-02), pages 3985-3997, XP002582830, ISSN: 0021-9258
- BEGLEY DAVID J ET AL: "Lysosomal storage diseases and the blood-brain barrier", CURRENT PHARMACEUTICAL DESIGN, vol. 14, no. 16, June 2008 (2008-06), pages 1566-1580, XP002582829, ISSN: 1381-6128
- ZHU H ET AL: "GLU-96 OF BASIC FIBROBLAST GROWTH FACTOR IS ESSENTIAL FOR HIGH AFFINITY RECEPTOR BINDING", JOURNAL OF BIOLOGICAL CHEMISTRY, AMERICAN SOCIETY FOR BIOCHEMISTRY AND MOLECULAR BIOLOGY, INC, US, vol. 270, no. 37, 15 September 1995 (1995-09-15), pages 21869-21874, XP002073993, ISSN: 0021-9258, DOI: DOI:10.1074/JBC.270.37.21869
- CHANG MICHAEL ET AL: "Intraventricular enzyme replacement improves disease phenotypes in a mouse model of late infantile neuronal ceroid lipofuscinosis.", MOLECULAR THERAPY : THE JOURNAL OF THE AMERICAN SOCIETY OF GENE THERAPY APR 2008 LNKD- PUBMED:18362923, vol. 16, no. 4, April 2008 (2008-04), pages 649-656, XP002582833, ISSN: 1525-0024
- KOHLSCHUTTER A ET AL: "Towards understanding the neuronal ceroid lipofuscinoses", BRAIN AND DEVELOPMENT, AMSTERDAM, NL, vol. 31, no. 7, 1 August 2009 (2009-08-01) , pages 499-502, XP026252136, ISSN: 0387-7604, DOI: DOI:10.1016/J.BRAINDEV.2008.12.008 [retrieved on 2009-02-04]
- ARAUJO RITA ET AL: "Proteolytic Enzyme Engineering: A Tool for Wool", BIOMACROMOLECULES, vol. 10, no. 6, June 2009 (2009-06), pages 1655-1661, ISSN: 1525-7797

## Description

### FIELD OF THE INVENTION

This invention is in the field of biology and medicine in particular human therapeutics, more in particular in the field of lysosomal storage diseases (LSDs) which are a group of approximately 40 rare inherited metabolic disorders that result from defects in lysosomal function. Lysosomal storage diseases result when a specific component of lysosomes which are organelles in the body's cells malfunctions.

### BACKGROUND OF THE INVENTION

Lysosomal storage diseases (LSDs) are a group of approximately 40 rare inherited metabolic disorders that result from defects in lysosomal function.

Tay-Sachs disease was the first of these disorders to be described, in 1881, followed by Gaucher disease in 1882 and Fabry disease in 1898. In the late 1950s and early 1960s, de Duve and colleagues, using cell fractionation techniques, cytological studies and biochemical analyses, identified and characterized the lysosome as a cellular organelle responsible for intracellular digestion and recycling of macromolecules. Pompe disease was the first disease to be identified as a LSD in 1963 (α-glucosidase deficiency).

Lysosomal storage disorders are caused by lysosomal dysfunction usually as a consequence of deficiency of a single enzyme required for the metabolism of lipids, proteins or carbohydrates. Worldwilde, individual LSDs occur with incidences of less than 1:100.000, however, as a group the incidence is about 1:5000 -1:10.000. Lysosomal disorders are caused by partial or complete loss of function of lysosomal proteins, mostly lysosomal enzymes. When this happens, substances accumulate in the cell. In other words, when the lysosome doesn't function normally, excess products destined for breakdown and recycling are stored in the cell.

Lysosomal storage diseases affect mostly children and they often die at a young and unpredictable age, many within a few months or years of birth. Many other children die of this disease following years of suffering from various symptoms of their particular disorder. The symptoms of lysosomal storage disease vary, depending on the particular disorder and other environmental and genetic factors. Usually, early onset forms are associated with a severe phenotype whereas late onset forms show a milder phenotype. Typical symptoms can include developmental delay, movement disorders, seizures, dementia, deafness and/or blindness. Some people with lysosomal storage disease have enlarged livers (hepatomegaly) and enlarged spleens (splenomegaly), pulmonary and cardiac problems, and bones that grow abnormally.

The lysosomal storage diseases are generally classified by the nature of the primary stored material involved, and can be broadly broken into the following: (ICD-10 codes are provided), (i) (E75) lipid storage disorders, mainly sphingolipidoses (including Gaucher's and Niemann-Pick diseases), (ii) (E75.0-E75.1) gangliosidosis (including Tay-Sachs disease), (iii) (E75.2) leukodystrophies, (iv) (E76.0) mucopolysaccharidoses (including Hunter syndrome and Hurler disease), (v) (E77) glycoprotein storage disorders and (vi) (E77.0-E77.1) mucolipidoses.

Alternatively to the protein targets, lysosomal storage diseases may be classified by the type of protein that is deficient and is causing build-up.

| **Type of defect protein** | **Disease examples** | **Deficient protein** |
|---|---|---|
| lysosomal hydrolases primarily | Sphingolipidoses(e.g., gangliosidoses, like GM1- and GM2-gangliosidoses, Gaucher's disease, Fabry disease, Niemann-Pick disease, like Niemann-Pick disease type A and B) | Various |
| Posttranstational modification of enzymes | Multiple sulfatase deficiency | Multiple sulfatases |
| Membrane transport proteins | Mucolipidosis type II and IIIA | N-acetylglucosamine-1-phosphate transferase |
| Enzyme protecting proteins | Galactosialidosis | Cathepsin A |
| Soluble nonenzymatic proteins | GM2-AP deficiency, variant AB | GM2-AP |
| Transmembrane proteins | SAP deficiency | Sphingolipid activator proteins |
| | Niemann-Pick disease, type C | NPC1 and NPC2 |
| | Salla disease | Sialin |

There are no cures for lysosomal storage diseases especially for those with brain involvement and treatment is mostly symptomatic, although bone marrow transplantation and enzyme replacement therapy (ERT) have been tried with some success (Clarke JT, Iwanochko RM (2005) "Enzyme replacement therapy of Fabry disease". Mol. Neurobiol. 32 (1): 43-50 and Bruni S, Loschi L, Incerti C, Gabrielli O, Coppa GV (2007) "Update on treatment of lysosomal storage diseases", Acta Myol 26 (1): 87-92). In addition, umbilical cord blood transplantation is being performed at specialized centres for a number of these diseases. Further, substrate reduction therapy, a method used to decrease the accumulation of storage material, is currently being evaluated for some of these diseases. Also enzyme replacement therapy is being attempted however; success rates are low because the enzymes are poorly internalized in particular by neurons.

WO 2007/091159 discloses chimeric polypeptides comprising a lysosomal peptide and a cell-penetrating peptide for use in treating lysosomal storage disorders. Reiland (Journal of Cell Science 105 (1993), 1085-93) used basic Fibroblast Growth Factor (bFGF) conjugated to saporin (bFGF-saporin) as a probe to study targeting of bFGF by bFGF binding receptors.

Hence, there is a great need for a therapy for treating individuals that have a lysosomal storage disease, preferably for those lysosomal storage diseases with brain involvement. Such an approach would need to overcome in particular the problem of poor internalization as it was recently shown for example that in rat brain the turnover of mannose-6-phosphate is much lower in the central nervous system than in other tissues.

To improve their internalization and lysosomal targeting beyond the amount mediated by the mannose-6-phosphate pathway the lysosomal proteins have to be modified. This modification can be undertaken by chemical or genetic fusion of the lysosomal protein with other molecules. The resulting chimeric molecules are much better internalized and targeted to the lysosomal compartment than the original, unmodified lysosomal proteins.

The successful fusion of lysosomal enzymes is greatly facilitated by the knowledge of the three-dimensional structure and enzymatic properties of the enzyme. The structures of a couple of lysosomal enzymes have been resolved recently. Among those is tripeptidyl peptidase 1 (TPP1) that has been crystallized as enzymatically active, completely glycosylated full-length protein (s. Pal et al., 2009, structure of tripeptidyl-peptidase I provides insight into the molecular basis of late infantile neuronal ceroid lipofuscinosis. J Biol Chem 284 (6): 3976-84).

### SUMMARY OF THE INVENTION

The inventors of the present invention have astonishingly found that certain chimeric molecules can solve the above mentioned problem. The present invention therefore, relates to a chimeric molecule comprising
(i) a targeting moiety that binds to heparin or heparan sulfate proteoglycans; and
(ii) a lysosomal peptide or protein, wherein the lysosomal peptide or protein is tripeptidyl-peptidase 1,
   wherein the targeting moiety is Basic Fibroblast Growth Factor (bFGF) comprising the amino acid sequence according to SEQ ID NO. 4 (GHFKDPKRLYCKNGGF).

Disclosed herein is also a chimeric molecule, comprising (i) a targeting moiety that binds to heparin or heparan sulfate proteoglycans, (ii) a lysosomal peptide or protein and (iii) wherein the targeting moiety is a neurotrophic growth factor and/or, wherein the targeting moiety comprises one of the following consensus sequences BBXB, BXBB, BBXXB, BXXBB, BBXXXB or BXXXBB and wherein B represents an arginine, lysine or histidine amino acid and X represents any amino acid, with the proviso that the targeting moiety is at least thirteen amino acids long.

The invention also relates to a polynucleotide encoding the chimeric molecule according to the invention as well as a pharmaceutical composition comprising the chimeric molecule according to the invention. The chimeric molecule according to the invention is also claimed for the use in the treatment of a disease. In one aspect of the invention the disease is a lysosomal storage disease.

Herein, a "chimeric molecule" is a molecule (preferably a biopolymer) containing molecule portions derived from two different origins, in a preferred embodiment, e.g. from two different genes.

Herein, a "mutant" sequence is defined as DNA, RNA or amino acid sequence differing from but having sequence identity with the native or disclosed sequence. Depending on the particular sequence, the degree of sequence identity between the native or disclosed sequence and the mutant sequence is preferably greater than 50% (e.g. 60%, 70%, 80%, 90%, 95%, 99% or more, calculated using the Smith-Waterman algorithm known by those skilled in the art (Smith & Waterman, 1981). As used herein, an "allelic variant" of a nucleic acid molecule, or region, for which nucleic acid sequence is provided herein is a nucleic acid molecule, or region, that occurs essentially at the same locus in the genome of another or second isolate, and that, due to natural variation caused by, for example, mutation or recombination, has a similar but not identical nucleic acid sequence. A coding region allelic variant typically encodes a protein having similar activity to that of the protein encoded by the gene to which it is being compared. An allelic variant can also comprise an alteration in the 5' or 3' untranslated regions of the gene, such as in regulatory control regions (e.g. see US patent 5,753,235).

### FIGURE CAPTIONS

**Fig. 1**
   Purification of the TPP1-FGF2 fusion protein: Coomassie-stained PAGE gel with the 86 kDa TPP1 fusion protein in lane 2 after cation exchange chromatography.
**Fig. 2**
   Autocatalytic processing of the TPP1-FGF2 fusion protein: **A** Coomassie-stained PAGE gel demonstrating the pH-dependent auto-processing of the 86 kDa TPP1-FGF2 fusion protein after 10 min (10') and 90 min (90'), respectively. **B** Activity of the TPP1-FGF2 fusion protein during auto-processing.
**Fig. 3**

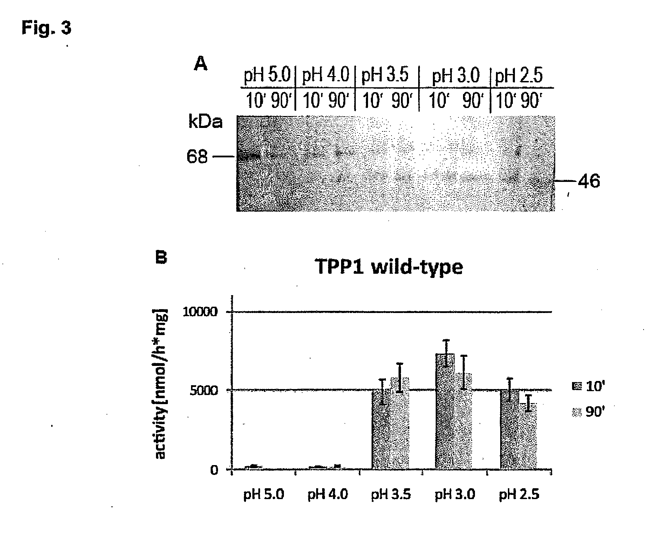

   Fig. 3 illustrates the respective auto-processing of the TPP1 wild-type. Interestingly, the TPP1-FGF2 fusion proteins showed a three times higher enzymatic activity than the processed TPP1 wild-type. Since after 10 min of incubation the N-terminal part of TPP1 is preferably cleaved off while the C-terminal part comprising the FGF2 tag is unaffected, it is concluded that the FGF2 tag improves the TPP1 activity. After 90 minutes incubation at room temperature the FGF2 tag is largely cleaved off and the activity is comparable to that of the TPP1 wild-type.
   The TPP1-FGF2 fusion protein is significantly more active at pH of 4.0 after a 10 minute (10 times higher) or a 90 minute incubation (5 times higher), respectively, than the TPP1 wild-type. This implies that the FGF2-Tag increases the TPP1 auto-processing at natural Lysosomal pH environment (pH 4-5). The *in vitro* auto-activation at pH 3.5 is not physiological and does not represent the *in vivo* conditions. *In vivo*, other interacting compounds such as glycosaminoglycans may increase auto-processing at higher pH (pH 4-5).
**Fig. 4**
   Cellular uptake and intracellular activation of the TPP1-FGF2 fusion protein (**A**) and the TPP1 wild-type (TPP1-WT) protein (**B**), respectively. After 48 h of incubation with 0.4 to 0.5 µM TPP1-FGF2 fusion protein or TPP1 wild-type protein, respectively, the activity in the cell lysates of human NT2 cells was determined. TPP1-FGF2 fusion protein treated cells had a six times higher activity than the TPP1 wild-type treated NT2 cells. By adding 1 mM heparin (H) the cellular uptake was reduced to less than 30 %, whereas the addition of mannose-6-phosphate (MP) led to a 50% reduction of the uptake of the TPP1-FGF2 fusion protein. The combined addition of H and MP (HMP) led to a reduction to 16% of the cellular uptake/activity of the TPP1-FGF2 fusion protein. For the TPP1 wild-type protein, the highest reduction was observed for MP alone.
**Fig. 5**
   Survival times of *tpp1-*/*-* mice under intraventricular injections of either 10 µg TPP1 wild-type or TPP1-FGF2 fusion protein once per week, respectively. Injections were performed from the 30th day of life of the mice.
**Fig. 6**

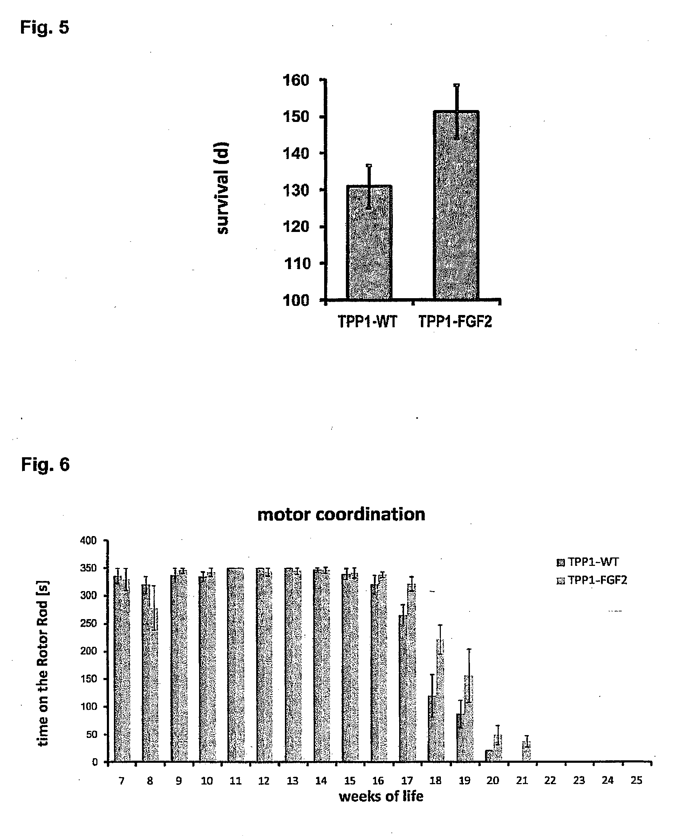

   Fig. 6 shows testing of the motor coordination of TPP1 wild-type (TPP1-WT) and TPP1-FGF2 fusion protein treated *tpp1-*/*-* mice. The time the mice spent on the Rotor Rod before falling down is plotted.

### DETAILED DESCRIPTION OF THE INVENTION

The inventors of the present invention have astonishingly found that certain chimeric molecules can solve the above mentioned problem. The present invention therefore, relates to a chimeric molecule comprising
(i) a targeting moiety that binds to heparin or heparan sulfate proteoglycans; and
(ii) a lysosomal peptide or protein, wherein the lysosomal peptide or protein is tripeptidyl-peptidase 1,
   wherein the targeting moiety is Basic Fibroblast Growth Factor (bFGF) comprising the amino acid sequence according to SEQ ID NO. 4 (GHFKDPKRLYCKNGGF).

Disclosed herein is also a chimeric molecule, comprising (i) a targeting moiety that binds to heparin or heparan sulfate proteoglycans, (ii) a lysosomal peptide or protein and (iii) wherein the targeting moiety is a neurotrophic growth factor and/or, wherein the targeting moiety comprises one of the following consensus sequences BBXB, BXBB, BBXXB, BXXBB, BBXXXB or BXXXBB and wherein B represents an arginine, lysine or histidine amino acid and X represents any amino acid, with the proviso that the targeting moiety is at least thirteen amino acids long.

Preferably, the targeting moiety contains at least 7 basic amino acids selected from arginine, lysine and histidine.

It was astonishingly found that chimeric polypeptides, such as TPP1-FGF1 fusion proteins showed a significantly higher life expectancy in mice (*tpp1*-/- mice) as compared to mice treated with the TPP1 wild-type protein.

Moreover, *tpp1-*/*-* mice treated with TPP1-FGF2-fusion proteins showed a delayed course of illness in comparison to *tpp1-*/*-* mice treated with the TPP1 wild-type. Also motor coordination with the so called Rotor Rod was greatly improved in mice treated with the TPP1-FGF2 fusion protein.

The chimeric molecule of the invention comprises as the targeting moiety bFGF comprising the amino acid sequence according to SEQ ID NO. 4 (GHFKDPKRLYCKNGGF).

Disclosed herein is also a chimeric molecule which comprises a targeting moiety selected from the group of
(i) annexin II comprising the amino acid sequence according to SEQ ID NO. 1 (KIRSEFKKKYGKSLYY),
(ii) vitronectin comprising the amino acid sequence according to SEQ ID NO. 2 (QRFRHRNRKGYRSQRG),
(iii) ApoB comprising the amino acid sequence according to SEQ ID NO. 3 (KFIIPSPKRPVKLLSG),
(iv) NCAM comprising the amino acid sequence according to SEQ ID NO. 5 (DGGSPIRHYLIKYKAK),
(v) Protein C inhibitor comprising the amino acid sequence according to SEQ ID NO. 6 (GLSEKTLRKWLKMFKK),
(vi) AT-III comprising the amino acid sequence according to SEQ ID NO. 7 (KLNCRLYRKANKSSKL),
(vii) ApoE comprising the amino acid sequence according to SEQ ID NO. 8 (SHLRKLRKRLLRDADD),
(viii) Fibrin comprising the amino acid sequence according to SEQ ID NO. 9 (GHRPLDKKREEAPSLR),
(ix) hGDNF comprising the amino acid sequence according to SEQ ID NO. 10 (SRGKGRRGQRGKNRG),
(x) B-thromboglobulin comprising the amino acid sequence according to SEQ ID NO. 11 (PDAPRIKKIVQKKLAG)
(xi) Insulin-like growth factor-binding protein-3 comprising the amino acid sequence according to SEQ ID NO. 12 (DKKGFYKKKQCRPSKG),
(xii) Antp comprising the amino acid sequence according to SEQ ID NO. 13 (RQIKIWFQNRRMKWKK) and
(xiii) human clock comprising the amino acid sequence according to SEQ ID NO. 14 (KRVSRNKSEKKRR).

In one embodiment the growth factor is modified and lysosomal targeting is improved.

In a preferred embodiment the chimeric molecule of the invention is a molecule wherein the targeting moiety and the lysosomal protein or peptide (also referred herein as the enzyme moiety; the two terms may are used interchangeable throughout the whole application) are covalently linked to each other.

Ideally, the chimeric molecule is a single polypeptide chain.

Expression systems for such peptide chains are for example those used with mammalian cells, baculoviruses, and plants.

### Mammalian Systems

Mammalian expression systems are known in the art. A mammalian promoter is any DNA sequence capable of binding mammalian RNA polymerase and initiating the downstream (3') transcription of a coding sequence (e.g. structural gene) into mRNA. A promoter will have a transcription initiating region, which is usually placed proximal to the 5' end of the coding sequence, and a TATA box, usually located 25-30 base pairs (bp) upstream of the transcription initiation site. The TATA box is thought to direct RNA polymerase II to begin RNA synthesis at the correct site. A mammalian promoter will also contain an upstream promoter element, usually located within 100 to 200 bp upstream of the TATA box. An upstream promoter element determines the rate at which transcription is initiated and can act in either orientation (Sambrook et al. (1989) "Expression of Cloned Genes in Mammalian Cells." In Molecular Cloning: A Laboratory Manual, 2nd ed.).

Mammalian viral genes are often highly expressed and have a broad host range; therefore sequences encoding mammalian viral genes provide particularly useful promoter sequences. Examples include the SV40 early promoter, mouse mammary tumor virus LTR promoter, adenovirus major late promoter (Ad MLP), and herpes simplex virus promoter. In addition, sequences derived from non-viral genes, such as the murine metallotheionein gene, also provide useful promoter sequences. Expression may be either constitutive or regulated (inducible), depending on the promoter can be induced with glucocorticoid in hormone-responsive cells. The presence of an enhancer element (enhancer), combined with the promoter elements described above, will usually increase expression levels. An enhancer is a regulatory DNA sequence that can stimulate transcription up to 1000-fold when linked to homologous or heterologous promoters, with synthesis beginning at the normal RNA start site. Enhancers are also active when they are placed upstream or downstream from the transcription initiation site, in either normal or flipped orientation, or at a distance of more than 1000 nucleotides from the promoter (Maniatis et al. (1987) Science 236: 1237; Alberts et al. (1989) Molecular Biology of the Cell, 2nd ed.). Enhancer elements derived from viruses may be particularly useful, because they usually have a broader host range. Examples include the SV40 early gene enhancer (Dijkema et al (1985) EMBO J. 4: 761) and the enhancer/promoters derived from the long terminal repeat (LTR) of the Rous Sarcoma Virus (Gorman et al. (1982b) Proc. Natl. Acad. Sci. 79: 6777) and from human cytomegalovirus (Boshart et al. (1985) Cell 41: 521). Additionally, some enhancers are regulatable and become active only in the presence of an inducer, such as a hormone or metal ion (Sassone-Corsi and Borelli (1986) Trends Genet. 2: 215; Maniatis et al. (1987) Science 236: 1237). A DNA molecule may be expressed intracellularly in mammalian cells. A promoter sequence may be directly linked with the DNA molecule, in which case the first amino acid at the N-terminus of the recombinant protein will always be a methionine, which is encoded by the ATG start codon. If desired, the N-terminus may be cleaved from the protein by in vitro incubation with cyanogen bromide. Alternatively, foreign proteins can also be secreted from the cell into the growth media by creating chimeric DNA molecules that encode a fusion protein comprised of a leader sequence fragment that provides for secretion of the foreign protein in mammalian cells. Preferably, there are processing sites encoded between the leader fragment and the foreign gene that can be cleaved either in vivo or In vitro. The leader sequence fragment usually encodes a signal peptide comprised of hydrophobic amino acids which direct the secretion of the protein from the cell. The adenovirus triparite leader is an example of a leader sequence that provides for secretion of a foreign protein in mammalian cells. Usually, transcription termination and polyadenylation sequences recognized by mammalian cells are regulatory regions located 3' to the translation stop codon and thus, together with the promoter elements, flank the coding sequence. The 3' terminus of the mature mRNA is formed by site-specific post-transcriptional cleavage and polyadenylation (Birnstiel et al. (1985) Cell 41: 349; Proudfoot and Whitelaw (1988) "Termination and 3' end processing of eukaryotic RNA" in Transcription and splicing (ed. B.D. Hames and D.M. Glover); Proudfoot (1989) Trends Biochem. Sci. 14: 105). These sequences direct the transcription of an mRNA which can be translated into the polypeptide encoded by the DNA. Examples of transcription terminator/polyadenylation signals include those derived from SV40 (Sambrook et al (1989) "Expression of cloned genes in cultured mammalian cells." In Molecular Cloning: A Laboratory Manual). Usually, the above described components, comprising a promoter, polyadenylation signal, and transcription termination sequence are put together into expression constructs. Enhancers, introns with functional splice donor and acceptor sites, and leader sequences may also be included in an expression construct, if desired. Expression constructs are often maintained in a replicon, such as an extrachromosomal element (e.g. plasmids) capable of stable maintenance in a host, such as mammalian cells or bacteria. Mammalian replication systems include those derived from animal viruses, which require trans-acting factors to replicate. For example, plasmids containing the replication systems of papovaviruses, such as SV40 (Gluzman (1981) Cell 23: 175) or polyomavirus, replicate to extremely high copy number in the presence of the appropriate viral T antigen. Additional examples of mammalian replicons include those derived from bovine papillomavirus and Epstein-Barr virus. Additionally, the replicon may have two replication systems, thus allowing it to be maintained, for example, in mammalian cells for expression and in a prokaryotic host for cloning and amplification. Examples of such mammalian-bacteria shuttle vectors include pMT2 (Kaufman et al. (1989) Mol. Cell. Biol. 9: 9469) and pHEBO (Shimizu et al. (1986) Mol. Cell. Biol. 6: 1074). The transformation procedure used depends upon the host to be transformed. Methods for introduction of heterologous polynucleotides into mammalian cells are known in the art and include dextran-mediated transfection, calcium phosphate precipitation, polybrene mediated transfection, protoplast fusion, electroporation, encapsulation of the polynucleotide (s) in liposomes, and direct microinjection of the DNA into nuclei. Mammalian cell lines available as hosts for expression are known in the art and include many immortalized cell lines available from the American Type Culture Collection (ATCC), including but not limited to, Chinese hamster ovary (CHO) cells, HeLa cells, baby hamster kidney (BHK) cells, monkey kidney cells (COS), human hepatocellular carcinoma cells (e.g. Hep G2), and a number of other cell lines.

### Baculovirus Systems

The polynucleotide encoding the protein can also be inserted into a suitable insect expression vector, and is operably linked to the control elements within that vector. Vector construction employs techniques which are known in the art. Generally, the components of the expression system include a transfer vector, usually a bacterial plasmid, which contains both a fragment of the baculovirus genome, and a convenient restriction site for insertion of the heterologous gene or genes to be expressed; a wild type baculovirus with a sequence homologous to the baculovirus-specific fragment in the transfer vector (this allows for the homologous recombination of the heterologous gene in to the baculovirus genome); and appropriate insect host cells and growth media. After inserting the DNA sequence encoding the protein into the transfer vector, the vector and the wild type viral genome are transfected into an insect host cell where the vector and viral genome are allowed to recombine. The packaged recombinant virus is expressed and recombinant plaques are identified and purified. Materials and methods for baculovirus/insect cell expression systems are commercially available in kit form from, inter alia, Invitrogen, San Diego CA ("MaxBac" kit). These techniques are generally known to those skilled in the art and fully described in Summers and Smith, Texas Agricultural Experiment Station Bulletin No. 1555 (1987) (hereinafter "Summers and Smith").

Prior to inserting the DNA sequence encoding the protein into the baculovirus genome, the above described components, comprising a promoter, leader (if desired), coding sequence, and transcription termination sequence, are usually assembled into an intermediate transplacement construct (transfer vector). This may contain a single gene and operably linked regulatory elements; multiple genes, each with its owned set of operably linked regulatory elements; or multiple genes, regulated by the same set of regulatory elements. Intermediate transplacement constructs are often maintained in a replicon, such as an extra-chromosomal element (e.g. plasmids) capable of stable maintenance in a host, such as a bacterium. The replicon will have a replication system, thus allowing it to be maintained in a suitable host for cloning and amplification. Currently, the most commonly used transfer vector for introducing foreign genes into AcNPV is pAc373. Many other vectors, known to those of skill in the art, have also been designed. These include, for example, pVL985 (which alters the polyhedrin start codon from ATG to ATT, and which introduces a BamHI cloning site 32 basepairs downstream from the ATT; see Luckow and Summers, Virology (1989) 17:31. The plasmid usually also contains the polyhedrin polyadenylation signal (Miller et al. (1988) Ann. Rev. Microbiol., 42: 177) and a prokaryotic ampicillin-resistance (amp) gene and origin of replication for selection and propagation in *E*. *coli*. Baculovirus transfer vectors usually contain a baculovirus promoter. A baculovirus promoter is any DNA sequence capable of binding a baculovirus RNA polymerase and initiating the downstream (5' to 3') transcription of a coding sequence (e.g. structural gene) into mRNA. A promoter will have a transcription initiation region which is usually placed proximal to the 5' end of the coding sequence. This transcription initiation region usually includes an RNA polymerase binding site and a transcription initiation site. A baculovirus transfer vector may also have a second domain called an enhancer, which, if present, is usually distal to the structural gene. Expression may be either regulated or constitutive.

Structural genes, abundantly transcribed at late times in a viral infection cycle, provide particularly useful promoter sequences. Examples include sequences derived from the gene encoding the viral polyhedron protein, Friesen et al., (1986) "The Regulation of Baculovirus Gene Expression," in: The Molecular Biology of Baculoviruses (ed. Walter Doerfler); EPO Publ. Nos. 127 839 and 155 476; and the gene encoding the p10 protein, Vlak et al., (1988), J. Gen. Virol. 69:765. DNA encoding suitable signal sequences can be derived from genes for secreted insect or baculovirus proteins, such as the baculovirus polyhedrin gene (Carbonell et al. (1988) Gene, 73:409). Alternatively, since the signals for mammalian cell posttranslational modifications (such as signal peptide cleavage, proteolytic cleavage, and phosphorylation) appear to be recognized by insect cells, and the signals required for secretion and nuclear accumulation also appear to be conserved between the invertebrate cells and vertebrate cells, leaders of non-insect origin, such as those derived from genes encoding human-interferon, Maeda et al., (1985), Nature 315:592; human gastrin-releasing peptide, Lebacq-Verheyden et al., (1988), Molec. Cell. Biol. 8: 3129; human IL-2, Smith et al., (1985) Proc. Natl. Acad. Sci. USA, 82:8404; mouse IL-3, (Miyajima et al., (1987) Gene 58:273; and human glucocerebrosidase, Martin et al. (1988) DNA, 7:99, can also be used to provide for secretion in insects. A recombinant polypeptide or polyprotein may be expressed intracellularly or, if it is expressed with the proper regulatory sequences, it can be secreted. Good intracellular expression of non-fused foreign proteins usually requires heterologous genes that ideally have a short leader sequence containing suitable translation initiation signals preceding an ATG start signal. If desired, methionine at the N-terminus may be cleaved from the mature protein by in vitro incubation with cyanogen bromide.

Alternatively, recombinant polyproteins or proteins which are not naturally secreted can be secreted from the insect cell by creating chimeric DNA molecules that encode a fusion protein comprised of a leader sequence fragment that provides for secretion of the foreign protein in insects. The leader sequence fragment usually encodes a signal peptide comprised of hydrophobic amino acids which direct the translocation of the protein into the endoplasmic reticulum.

After insertion of the DNA sequence and/or the gene encoding the expression product precursor of the protein, an insect cell host is co-transformed with the heterologous DNA of the transfer vector and the genomic DNA of wild type baculovirus - usually by co-transfection. The promoter and transcription termination sequence of the construct will usually comprise a 2-5kb section of the baculovirus genome. Methods for introducing heterologous DNA into the desired site in the baculovirus virus are known in the art. (See Summers and Smith supra; Ju et al. (1987); Smith et al., Mol. Cell. Biol. (1983) 3: 2156; and Luckow and Summers (1989)). For example, the insertion can be into a gene such as the polyhedrin gene, by homologous double crossover recombination; insertion can also be into a restriction enzyme site engineered into the desired baculovirus gene. Miller et al., (1989), Bioessays 4: 91.The DNA sequence, when cloned in place of the polyhedrin gene in the expression vector, is flanked both 5' and 3' by polyhedrin-specific sequences and is positioned downstream of the polyhedrin promoter. The newly formed baculovirus expression vector is subsequently packaged into an infectious recombinant baculovirus. Homologous recombination occurs at low frequency (between about 1 % and about 5%); thus, the majority of the virus produced after cotransfection is still wild-type virus. Therefore, a method is necessary to identify recombinant viruses. An advantage of the expression system is a visual screen allowing recombinant viruses to be distinguished. The polyhedrin protein, which is produced by the native virus, is produced at very high levels in the nuclei of infected cells at late times after viral infection. Accumulated polyhedrin protein forms occlusion bodies that also contain embedded particles. These occlusion bodies, up to 15 m in size, are highly refractile, giving them a bright shiny appearance that is readily visualized under the light microscope. Cells infected with recombinant viruses lack occlusion bodies. To distinguish recombinant virus from wild-type virus, the transfection supernatant is plaqued onto a monolayer of insect cells by techniques known to those skilled in the art. Namely, the plaques are screened under the light microscope for the presence (indicative of wild-type virus) or absence (indicative of recombinant virus) of occlusion bodies. "Current Protocols in Microbiology" Vol. 2 (Ausubel et al. eds) at 16.8 (Supp. 10, 1990); Summers and Smith, supra; Miller et al. (1989). Recombinant baculovirus expression vectors have been developed for infection into several insect cells. For example, recombinant baculoviruses have been developed for, inter alia: Aedes aegypti, Autographa californica, Bombyx mori, Drosophila melanogaster, Spodoptera frugiperda, and Trichoplusia ni (WO 89/046699; Carbonell et al., (1985) J. Virol. 56: 153; Wright (1986) Nature 321: 718; Smith et al., (1983) Mol. Cell. Biol. 3: 2156; and see generally, Fraser, et al. (1989) In Vitro Cell. Dev. Biol. 25: 225). Cells and cell culture media are commercially available for both direct and fusion expression of heterologous polypeptides in a baculovirus/expression system; cell culture technology is generally known to those skilled in the art. See, e.g. Summers and Smith supra. The modified insect cells may then be grown in an appropriate nutrient medium, which allows for stable maintenance of the plasmid (s) present in the modified insect host. Where the expression product gene is under inducible control, the host may be grown to high density, and expression induced. Alternatively, where expression is constitutive, the product will be continuously expressed into the medium and the nutrient medium must be continuously circulated, while removing the product of interest and augmenting depleted nutrients. The product may be purified by such techniques as chromatography, e.g. HPLC, affinity chromatography, ion exchange chromatography, etc.; electrophoresis; density gradient centrifugation; solvent extraction, etc. As appropriate, the product may be further purified, as required, so as to remove substantially any insect proteins which are also present in the medium, so as to provide a product which is at least substantially free of host debris, e.g. proteins, lipids and polysaccharides. In order to obtain protein expression, recombinant host cells derived from the transformants are incubated under conditions which allow expression of the recombinant protein encoding sequence. These conditions will vary, dependent upon the host cell selected. However, the conditions are readily ascertainable to those of ordinary skill in the art, based upon what is known in the art.

### Plant Systems

There are many plant cell culture and whole plant genetic expression systems known in the art. Exemplary plant cellular genetic expression systems include those described in patents, such as: US 5,693,506; US 5,659,122; and US 5,608,143. Additional examples of genetic expression in plant cell culture has been described by Zenk, Phytochemistry 30: 3861-3863 (1991). Typically, using techniques known in the art, a desired polynucleotide sequence is inserted into an expression cassette comprising genetic regulatory elements designed for operation in plants. The expression cassette is inserted into a desired expression vector with companion sequences upstream and downstream from the expression cassette suitable for expression in a plant host. The companion sequences will be of plasmid or viral origin and provide necessary characteristics to the vector to permit the vectors to move DNA from an original cloning host, such as bacteria, to the desired plant host. The basic bacterial/plant vector construct will preferably provide a broad host range prokaryote replication origin; a prokaryote selectable marker; and, for Agrobacterium transformations, T DNA sequences for Agrobacterium-mediated transfer to plant chromosomes. Where the heterologous gene is not readily amenable to detection, the construct will preferably also have a selectable marker gene suitable for determining if a plant cell has been transformed. A general review of suitable markers, for example for the members of the grass family, is found in Wilmink and Dons, 1993, Plant Mol. Biol. Reptr, 11 (2):165-185. Sequences suitable for permitting integration of the heterologous sequence into the plant genome are also recommended. These might include transposon sequences and the like for homologous recombination as well as Ti sequences which permit random insertion of a heterologous expression cassette into a plant genome. Suitable prokaryote selectable markers include resistance toward antibiotics such as ampicillin or tetracycline. Other DNA sequences encoding additional functions may also be present in the vector, as is known in the art. The nucleic acid molecules of the subject invention may be included into an expression cassette for expression of the protein (s) of interest. Usually, there will be only one expression cassette, although two or more are feasible. The recombinant expression cassette will contain in addition to the heterologous protein encoding sequence the following elements, a promoter region, plant 5' untranslated sequences, initiation codon depending upon whether or not the structural gene comes equipped with one, and a transcription and translation termination sequence. Unique restriction enzyme sites at the 5' and 3' ends of the cassette allow for easy insertion into a pre-existing vector. A heterologous coding sequence may be for any protein relating to the present invention. The sequence encoding the protein of interest will encode a signal peptide which allows processing and translocation of the protein, as appropriate, and will usually lack any sequence which might result in the binding of the desired protein of the invention to a membrane. Since, for the most part, the transcriptional initiation region will be for a gene which is expressed and translocated during germination, by employing the signal peptide which provides for translocation, one may also provide for translocation of the protein of interest. In this way, the protein(s) of interest will be translocated from the cells in which they are expressed and may be efficiently harvested. Typically secretion in seeds are across the aleurone or scutellarepithelium layer into the endosperm of the seed. While it is not required that the protein be secreted from the cells in which the protein is produced, this facilitates the isolation and purification of the recombinant protein. Since the ultimate expression of the desired gene product will be in a eucaryotic cell it is desirable to determine whether any portion of the cloned gene contains sequences which will be processed out as introns by the host's splicosome machinery. If so, site-directed mutagenesis of the "intron" region may be conducted to prevent losing a portion of the genetic message as a false intron code, Reed and Maniatis, Cell 41:95-105, 1985. The vector can be microinjected directly into plant cells by use of micropipettes to mechanically transfer the recombinant DNA. Crossway, Mol. Gen. Genet, 202:179-185,1985. The genetic material may also be transferred into the plant cell by using polyethylene glycol, Krens, et al., Nature, 296, 72-74, 1982. Another method of introduction of nucleic acid segments is high velocity ballistic penetration by small particles with the nucleic acid either within the matrix of small beads or particles, or on the surface, Klein, et al., Nature, 327, 70-73, 1987 and Knudsen and Muller, 1991, Planta, 185:330-336 teaching particle bombardment of barley endosperm to create transgenic barley. Yet another method of introduction would be fusion of protoplasts with other entities, either minicells, cells, lysosomes or other fusible lipidsurfaced bodies, Fraley, et al., Proc. Natl. Acad. Sci. USA, 79, 1859-1863, 1982. The vector may also be introduced into the plant cells by electroporation. (Fromm et al., Proc. Natl Acad. Sci. USA 82: 5824, 1985). In this technique, plant protoplasts are electroporated in the presence of plasmids containing the gene construct. Electrical impulses of high field strength reversibly permeabilize biomembranes allowing the introduction of the plasmids. Electroporated plant protoplasts reform the cell wall, divide, and form plant callus. All plants from which protoplasts can be isolated and cultured to give whole regenerated plants can be transformed by the present invention so that whole plants are recovered which contain the transferred gene. It is known that practically all plants can be regenerated from cultured cells or tissues, including but not limited to all major species of sugarcane, sugar beet, cotton, fruit and other trees, legumes and vegetables. Some suitable plants include, for example, species from the genera Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersion, Nicotiana, Solanum, Petunia, Digitalis, Majorana, Cichorium, Helianthus, Lactuca, Bromus, Asparagus, Antirrhinum, Hererocallis, Nemesia, Pelargonium, Panicum, Pennisetum, Ranunculus, Senecio, Salpiglossis, Cucumis, Browaalia, Glycine, Lolium, Zea, Triticum, Sorghum, and Datura. Means for regeneration vary from species to species of plants, but generally a suspension of transformed protoplasts containing copies of the heterologous gene is first provided. Callus tissue is formed and shoots may be induced from callus and subsequently rooted. Alternatively, embryo formation can be induced from the protoplast suspension. These embryos germinate as natural embryos to form plants. The culture media will generally contain various amino acids and hormones, such as auxin and cytokinins. It is also advantageous to add glutamic acid and proline to the medium, especially for such species as corn and alfalfa. Shoots and roots normally develop simultaneously. Efficient regeneration will depend on the medium, on the genotype, and on the history of the culture. If these three variables are controlled, then regeneration is fully reproducible and repeatable.

In some plant cell culture systems, the desired protein of the invention may be excreted or alternatively, the protein may be extracted from the whole plant. Where the desired protein of the invention is secreted into the medium, it may be collected. Alternatively, the embryos and embryoless-half seeds or other plant tissue may be mechanically disrupted to release any secreted protein between cells and tissues. The mixture may be suspended in a buffer solution to retrieve soluble proteins. Conventional protein isolation and purification methods will be then used to purify the recombinant protein. Parameters of time, temperature pH, oxygen, and volumes will be adjusted through routine methods to optimize expression and recovery of heterologous protein.

Particular molecules as disclosed herein are listed in Tables 1 to 7 below. In one embodiment the following tags are added at the C-terminus of the lysosomal proteins (see Table 1). Ideally, they contain linker sequences between the lysosomal protein and the tag. The C-terminal tags are fused with the lysosomal proteins in such a way that the tags replace the stop codon of the lysosomal proteins. In case C-terminal amino acids are omitted it is indicated.

**Table 1 - Antp and CLOCK**

| | | |
|---|---|---|
| Linker preceding the tags "Antp" and "CLOCK" | AGATCCCCCGGG | SEQ ID NO. 15 |
| Linker preceding the tags "Antp" and "CLOCK" | GGATCCCCCGGG | SEQ ID NO.16 |
| Antp cDNA of tag; | | SEQ ID NO.17 |
| Antp amino acid sequence; | RQIKIWFQNRRMKWKK | SEQ ID NO.18 |
| Human CLOCK cDNA tag; | | SEQ ID NO.19 |
| Human CLOCK amino acid sequence tag; | KRVSRNKSEKKRR | SEQ ID NO.20 |

The following tags are derived from the human basic fibroblast growth factor (FGF2) and possess an N-terminal linker (AGATCCGTCGACATCGAAGGTAGAGGCATT (SEQ ID NO. 21) or GGATCCGTCGACATCGAAGGTAGAGGCATT (SEQ ID NO. 23)) containing the factor Xa cleavage site "IEGR" (Table 2). The N-terminal linker may be mutated within the Xa cleavage site (IEGR) so that a base change through a mutation at bp 24 of SEQ ID NO. 21 or 22 eliminates the factor Xa cleavage site "IEGR" by replacing the "R" by "S". In context of the present invention the sequences within the fusion proteins encoded by a nucleic acid sequence according to SEQ ID NO. 21 or SEQ ID NO. 22 may be exchanged by a peptide sequence encoded by a nucleic acid sequence according to SEQ ID NO. 73 or SEQ ID NO. 74, respectively. Furthermore, the sequences according to SEQ ID NO. 21 or SEQ ID NO. 23 within the nucleotide sequences according to the present invention may be exchanged by a nucleotide sequence according to SEQ ID NO. 73 or SEQ ID NO. 74, respectively.

**Table 2 - FGF2 and variants thereof**

| | | |
|---|---|---|
| N-terminal linker | AGATCCGTCGACATCGAAGGTAGAGGCATT | SEQ ID NO.21 |
| N-terminal linker | GGATCCGTCGACATCGAAGGTAGAGGCATT | SEQ ID NO.22 |
| N-terminal linker with mutated Xa cleavage site | AGATCCGTCGACATCGAAGGTAGCGGCATT | SEQ ID NO. 73 |
| N-terminal linker with mutated Xa cleavage site | GGATCCGTCGACATCGAAGGTAGCGGCATT | SEQ ID NO.74 |
| FGF2 variant 1 (base substitution G206C and G260C (small letter) leading to amino acid substitution s C69S and C87S) CDNA: | | SEQ ID NO.23 |
| Amino acid sequence of FGF2 variant 1 (base substitution G206C and G260C (small letter) leading to amino acid substitution s C69S and C87S) | | SEQ ID NO.24 |
| FGF2 variant 2 (same as variant 1 plus reduced FGFR binding ) cDNA | | SEQ ID NO.25 |
| | | |
| FGF2 variant 2 amino acid sequence; | | SEQ ID NO.26 |
| FGF2 variant 3 (same as variant 1 plus reduced nuclear translocation n) CDNA; | | SEQ ID NO.27 |
| FGF2 variant 3 amino acid sequence; | | SEQ ID NO.28 |
| FGF2 variant 4 (same as variant 1 plus reduced FGFR binding and reduced nuclear translocatio n) cDNA: | | SEQ ID NO.29 |
| FGF2 variant 4 amino acid sequence | | SEQ ID NO.30 |

The following sequences demonstrate the C-terminal tags fused to the cDNA of human tripeptidyl peptidase 1 (TPP1) (Table 3).

**Table 3 - TPP1/Antp, TPP1/CLOCK and TPP1/FGF2 and variants thereof**

| | | |
|---|---|---|
| TPP1-Antp construct CDNA; | | SEQ ID NO.31 |
| | | |
| TPP1-Antp amino acid sequence; | | SEQ ID NO.32 |
| TPP1-CLOCK cDNA: | | SEQ ID NO. 33 |
| | | |
| TPP1-CLOCK amino acid sequence: | | SEQ ID NO. 34 |
| | | |
| TPP1-FGF2 variant 1 cDNA: | | SEQ ID NO.35 |
| | | |
| TPP1-FGF2 variant 1 amino acid sequence; | | SEQ ID NO.36 |
| TPP1-FGF2 variant 2 cDNA; | | SEQ ID NO.37 |
| | | |
| | | |
| TPP1-FGF2 variant 2 amino acid sequence; | | SEQ ID NO.38 |
| TPP1-FGF2 variant 3 cDNA; | | SEQ ID NO.39 |
| | | |
| | | |
| TPP1-FGF2 variant 3 amino acid sequence; | | SEQ ID NO.40 |
| TPP1-FGF2 variant 4 cDNA; | | SEQ ID NO.41 |
| | | |
| TPP1-FGF2 variant 4 amino acid sequence; | | SEQ ID NO.42 |
| | | |

The following tags (Table 4) are derived from the human heparin-binding epidermal growth factor (HB-EGF). They are added at the N-terminus of the lysosomal proteins and replace the signal peptide of the lysosomal proteins.

Two different HB-EGF tags were designed. The last nucleotide "T" of HB1 and HB2 is alternatively replaced by "C":

**Table 4 HB1 and HB2 tags**

| | | |
|---|---|---|
| HB1 cDNA | | SEQ ID NO.43 |
| HB2 cDNA | | SEQ ID NO.44 |
| HB1 amino acid sequence | | SEQ ID NO. 71 |
| HB2 amino acid sequence | | SEQ ID NO. 72 |

The following sequences (Table 5) disclose the N-terminal tags fused to the cDNA of human sulfamidase (hSGSH)

**Table 5 - HB1/SGSH and HB2/SGSH**

| | | |
|---|---|---|
| HB1-SGSH cDNA: | | SEQ ID NO.45 |
| | | |
| HB1-SGSH amino acid sequence; | | SEQ ID NO.46 |
| HB2-SGSH CDNA: | | SEQ ID NO.47 |
| | | |
| HB2-SGSH amino acid sequence; | | SEQ ID NO.48 |

Combined N-terminal and C-terminal heparin/heparan sulfate binding tags were constructed correspondingly. The combined N-terminal and C-terminal tag is demonstrated for human sulfamidase (SGSH) (Table 6).

**Table 6 - HB2/SGSH/Antp**

| | | |
|---|---|---|
| HB2-SGSH-Antp cDNA; | | SEQ ID NO.49 |
| HB2-SGSH-Antp amino acid sequence | | SEQ ID NO.50 |

The following lysosomal proteins were fused to the above described heparin/heparin sulfate binding tags (Table 7).

**Table 7 - TPP1, CTSD, PPT1, SGSH, IDUA, IDS, ARSA, GALC, GBA and GLA**

| | | |
|---|---|---|
| Human tripeptidyl peptidase 1 (TPP1) cDNA: | | SEQ ID NO.51 |
| | | |
| Human tripeptidyl peptidase 1 (TPP1) amino acid sequence; | | SEQ ID NO.52 |
| Human cathepsin D (CTSD) cDNA; | | SEQ ID NO.53 |
| | | |
| Human cathepsin D (CTSD) amino acid sequence; | | SEQ ID NO.54 |
| Human palmitoyl protein thioesterase 1 (PPT1) CDNA; | | SEQ ID NO.55 |
| | | |
| Human palmitoyl protein thioesterase 1 (PPT1) amino acid sequence; | | SEQ ID NO.56 |
| Human sulfamidase (SGSH) CDNA; | | SEQ ID NO.57 |
| | | |
| Human sulfamidase (SGSH) amino acid sequence; | | SEQ ID NO.58 |
| Human alpha-L-iduronidase (IDUA) CDNA; | | SEQ ID NO.59 |
| | | |
| | | |
| Human alpha-L-iduronidase (IDUA) amino acid sequence; | | SEQ ID NO.60 |
| Human iduronate-2-sulfatase (IDS) CDNA; | | SEQ ID NO.61 |
| | | |
| Human iduronate-2-sulfatase (IDS) amino acid sequence; | | SEQ ID NO.62 |
| Human arylsulfatas e A (ARSA) CDNA. Remark: for the C-terminal tags the sequence "CATGCC" immediately before the stop codon was omitted (small letters); | | SEQ ID NO.63 |
| Human arylsulfatase A (ARSA) amino acid sequence. Remark: for the C-terminal tags the last two amino acids "HA" were omitted; | | SEQ ID NO.64 |
| | | |
| Human galactocereb rosidase (GALC) CDNA. Remark: for the C-terminal tags the sequence "CGC" immediately before the stop codon was omitted (small letters); | | SEQ ID NO.65 |
| | | |
| Human galactocereb rosidase (GALC) amino acid sequence. Remark: for the C-terminal tags the last amino acid "R" was omitted; | | SEQ ID NO.66 |
| Human acid | | SEQ ID NO.67 |
| beta-glucosidase= beta-glucocerebro sidase (GBA) CDNA. Remark: substitution of 3 cysteine by serine residues; | | |
| Human acid beta-glucosidase= beta-glucocerebro sidase (GBA) amino acid sequence. Remark: substitution of 3 "C" by "S" (C165S, C287S, C381S); | | SEQ ID NO.68 |
| Human alpha galactosidas e (GLA) cDNA; | | SEQ ID NO.69 |
| | | |
| Human alpha galactosidas e (GLA) amino acid sequence; | | SEQ ID NO.70 |

A targeting moiety selected from the group of Antp, CLOCK, FGF2, HB1 and HB2 including the variants as outlined above is disclosed herein.

Preferably the targeting moiety and the enzyme moiety are linked via a peptide linker as encoded by one of the following sequences SEQ ID NO. 15 or 16. SEQ ID NO. 21 or 22 are preferably N-terminal to FGF2 variants. SEQ ID NO. 43 or 44 are preferably N-terminal to the lysosomal proteins.

In one embodiment the peptide linker comprises a protease cleavage site. Such a site may be a site recognized by factor Xa, a caspase, thrombin, trypsin, papain and plasmin. For FGF2 variant constructs this is preferred.

In particular, a, lysosomal enzyme selected from the group consisting of β-galactocerebrosidase, arylsulfatase A (sulfatidase), α-iduronidase, sulfaminidase, α-N-acetylglucosaminidase, acetyl-CoA:α-glucosaminide-N-Ac-transferase, N-acetylglucosamine-6-sulfatase, tripeptidyl-peptidase 1, palmitoyl-protein thioesterase, β-galactosidase, sphingomyelinase, β-hexosaminidase A, β-hexosaminidase B, ceramidase, α-mannosidase, β-mannosidase, α-fucosidase, sialidase, α-N-acetylgalactosaminidase, α-L-iduronidase, iduronate-2-sulfatase, sulfamidase (heparan N-sulftase), α-N-acetylglucosaminidase, N-acetylgalactosamin-6-sulfatase, arylsulfatase B, β-glucuronidase, α-L-fucosidase, aspartylglucosaminidase, α-neuraminidase (sialidase), cathepsin A, β-hexosaminidase A + B, arylsulfatase A, cerebroside-β-galactosidase, β-glucocerebrosidase, α-galactosidase A (ceramide trihexosidase), acid α-glucosidase (acid maltase), CLN5-protein, acid lipase, steroid sulfatase (arylsulfatase C) and cathepsin D is disclosed herein.

More particular, a lysosomal enzyme selected from the group consisting of tripeptidyl-peptidase 1 (TPP1), Human cathepsin D (CTSD), Human palmitoyl protein thioesterase 1 (PPT1), Human sulfamidase (SGSH), Human alpha-L-iduronidase (IDUA), Human iduronate-2-sulfatase (IDS), Human arylsulfatase A (ARSA), Human acid beta-glucosidase=beta-glucocerebrosidase (GBA) and Human alpha-galactosidase (GLA) is disclosed herein.

Preferably the targeting moiety is a polypeptide having a sequence according to any one of SEQ ID NO. 18, 20, 24, 26, 27, 30, 71, 72 or as encoded by 23, 25, 27, 29, 43, and 44 or the other nucleic acid sequences encoding the respective peptides.

Polypeptides with reduced nuclear translocation are preferred, such as SEQ ID NO. 28.

Polypeptides with reduced FGF receptor binding are preferred, such as SEQ ID NO. 26.

Also polypeptides with both above mentioned activity reductions are preferred such as SEQ ID NO. 30.

Preferably the enzyme moiety is a polypeptide having a sequence according to any one of SEQ ID NO. 52, 54, 56, 58, 60, 62, 64, 66, 68 and 70.

Preferably chimeric molecule polypeptide has a sequence according to any one of the SEQ ID NO. 32, 34, 36, 38, 40, 42, 46, 48 and 50.

The present invention also relates to sequence variants, allelic variants or mutants of the chimeric molecule described herein, and nucleic acid sequences that encode them. Sequence variants of the invention preferably share at least 90%, 91%, 92%, 93% or 94% identity with a polypeptide of the invention or with a nucleic acid sequence that encodes it. More preferably, a sequence variant shares at least 95%, 96%, 97% or 98% identity at the amino acid or nucleic acid level. Most preferably, a sequence variant shares at least 99%, 99.5%, 99.9% or more identity with a polypeptide of the invention or a nucleic acid sequence that encodes it.

Accordingly, the present invention provides an isolated chimeric protein comprising a sequence that is at least 80%, 85%, 90%, 92%, 94%, 95%, 96%, 97%, 98%, 99%, 99.5%, 99.9% or 100% identical to the sequence of the chimeric molecule of the invention.

The chimeric molecules may be pegylated. The term "pegylation," "polyethylene glycol" or "PEG" includes a polyalkylene glycol compound or a derivative thereof, with or without coupling agents or derivatization with coupling or activating moieties (e.g., with thiol, triflate, tresylate, azirdine, oxirane, or preferably with a maleimide moiety, e.g., PEG-maleimide). Other appropriate polyalkylene glycol compounds include, but are not limited to, maleimido monomethoxy PEG, activated PEG polypropylene glycol, but also charged or neutral polymers of the following types: dextran, colominic acids, or other carbohydrate based polymers, polymers of amino acids, and biotin and other affinity reagent derivatives.

The chimeric molecules may be incorporated into nanoparticles, solid polymeric molecules of 1 - 1000 nm diameters. These nanoparticles may comprise poly butyl cyanoacrylate, poly lactic acid or similar compounds and can be coated with polysorbate 80 and polysorbate 20 or similar non-ionic surfactant and emulsifier.

The chimeric molecules may be incorporated into virus like particles that consist of recombinantly produced viral envelope proteins. The chimeric molecules are packaged into these viral envelope proteins and taken up by cells via viral cell surface receptors and released from the viral envelope proteins within the target cells.

The invention also relates to a polynucleotide encoding the chimeric molecule according to the invention.

Particular polynucleotides as disclosed herein are selected from the group of the SEQ ID NO. 31, 33, 35, 37, 39, 41, 45, 47 and 49.

The nucleic acid may differ from the sequence outlined above, in particular due to the degeneracy of the genetic code.

The invention also relates to a pharmaceutical composition comprising a chimeric molecule according to the invention.

The invention relates to the chimeric molecule according to the invention for the use in the treatment of a disease.

The disease is preferably a lysosomal storage disease, preferably with brain involvement.

Preferably the lysosomal storage disease is selected from the group consisting of the neuronal ceroid lipofuscinoses (NCL), infantile NCL (CLN1-defect), late infantile NCL (CLN2-defect), late infantile NCL (CLN5-defect), NCL caused by cathepsin D deficiency (CLN10-defect), mucopolysaccharidosis type I, mucopolysaccharidosis type II, mucopolysaccharidosis type IIIA, mucopolysaccharidosis type IIIB, mucopolysaccharidosis type IIIC, mucopolysaccharidosis type IIID, mucopolysaccharidosis type IVA, mucopolysaccharidosis type IVB, mucopolysaccharidosis type VI, mucopolysaccharidosis type VII, fucosidosis, α-mannosidosis, β-mannosidosis, aspartylglucosaminuria, Schindler's disease, sialidosis (mucolipidosis I), galactosialidosis, GM₁-gangliosidosis / mucopolysaccharidosis type IVB, GM₂-gangliosidosis, Sandhoff disease, Tay-Sachs disease, metachromatic leukodystrophy, Krabbe disease, Gaucher disease, Fabry disease, Niemann-Pick disease type A+B), glycogen storage disease type II (Pompe disease), Faber's syndrome, Wolman disease, X-linked ichthyosis.

Brain involvement in context of the present invention refers to diseases related to neurological and/or psychiatric symptoms, i.e. to any abnormality related to the central nervous system and may manifest as neurological or psychiatric symptoms (e.g. mental retardation), as neuropysiological abnormality (e.g. signs of epileptic discharges in the electroencephalography) or as abnormal brain imaging (e.g. atrophy of the grey matter).

In one embodiment of the present invention lysosomal storage diseases with brain involvement are selected from the group consisting of neuronal ceroid lipofuscinoses (NCL), infantile NCL (CLN1-defect), late infantile NCL (CLN2-defect), late infantile NCL (CLN5-defect), NCL caused by cathepsin D deficiency (CLN10-defect), mucopolysaccharidosis type I, mucopolysaccharidosis type II, mucopolysaccharidosis type IIIA, mucopolysaccharidosis type IIIB, mucopolysaccharidosis type IIIC, mucopolysaccharidosis type IIID, mucopolysaccharidosis type IVB, mucopolysaccharidosis type VII, fucosidosis, α-mannosidosis, β-mannosidosis, aspartylglucosaminuria, Schindler's disease, sialidosis (mucolipidosis I), galactosialidosis, GM₁-gangliosidosis mucopolysaccharidosis type IVB, GM₂-gangliosidosis, Sandhoff disease, Tay-Sachs disease, metachromatic leukodystrophy, Krabbe disease, Gaucher disease, Fabry disease, Niemann-Pick disease (type A+B), Faber's syndrome, Wolman disease.

In one embodiment the lysosomal storage disease is the late infantile form of neuronal ceroid lipofuscinosis and the enzyme moiety comprises lysosomal tripeptidyl peptidase 1 (TPP1).

Combinations of disease names and enzyme defects are given in table 8 below.

**Table 8**

| **DISEASE** | **ENZYME/PROTEIN DEFECT** |
|---|---|
| Mucopolysaccharidosis type I | α-L-iduronidase |
| Mucopolysaccharidosis type II | Iduronat-2-Sulfatase |
| Mucopolysaccharidosis type IIIA | Sulfamidase (Heparan N-Sulfatase) |
| Mucopolysaccharidosis type IIIB | α-N-Acetylglucosaminidase |
| Mucopolysaccharidosis type IIIC | Glucosamin-N-Acetyltransferase |
| Mucopolysaccharidosis type IIID | N-Acetylglucosamin-6-Sulfatase |
| Mucopolysaccharidosis type IVA | N-Acetylgalactosamin-6-Sulfatase |
| Mucopolysaccharidosis type IVB | β-Galactosidase |
| Mucopolysaccharidosis type VI | Arylsulfatase B |
| Mucopolysaccharidosis type VII | β-Glucuronidase |
| Fucosidosis | α-L-Fucosidase |
| α-Mannosidosis | α-Mannosidase |
| β-Mannosidosis | β-Mannosidase |
| Aspartylglucosaminuria | Aspartylglucosaminidase |
| M. Schindler | α-N-Acetylgalactosaminidase |
| Sialidosis (Mucolipidosis Type I) | α-Neuraminidase (Sialidase) |
| Galactosialidosis | Cathepsin A |
| GM₁-Gangliosidosis / MPS IVB | β-Galactosidase |
| GM₂-Gangliosidosis | |
| M. Sandhoff | β-Hexosaminidase A + B |
| M. Tay-Sachs | β-Hexosaminidase A |
| Metachromatic Leukodystrophy | Arylsulfatase A |
| M. Krabbe | Cerebrosid-β-Galactosidase |
| M. Gaucher | β-Glucocerebrosidase |
| M. Fabry | α-Galactosidase A (Ceramidtrihexosidase) |
| M. Niemann-Pick Type =A+B | Sphingomyelinase |
| Glycogen storage disease type II (M. Pompe) | Acid α-Glucosidase (Acid Maltase) |
| Infantile NCL (CLN1-defect) | Palmitoyl-Protein Thioesterase 1 (PPT1) |
| Late Infantile NCL (CLN2-defect) | Tripeptidyl-Peptidase 1 (TPP1) |
| Late Infantile NCL (CLN5-defect) | CLN5-Protein |
| Cathepsin D deficient NCL (CLN10-defect) | Cathepsin D |
| M. Faber | Ceramidase |
| M. Wolman | acid Lipase |
| X-chromosomal Ichthyosis | Steroidsulfatase (Arylsulfatase C) |

In a preferred embodiment the chimeric molecule for use in the treatment of a disease is administered intraventricularly, prefereably by use of an Ommaya reservoir or a Rickham capsule.

In one embodiment the invention relates to the use of the chimeric molecule according to the invention for the manufacture of a medicament.

The invention also relates to a method of treating a lysosomal storage disease comprising the administration of a therapeutically effective amount of a chimeric molecule according to the invention. In a preferred embodiment of the present invention the lysosomal storage disease is a lysosomal storage disease with brain involvement.

In a first aspect the present invention relates to a chimeric molecule comprising
(i) a targeting moiety that binds to heparin or heparan sulfate proteoglycans; and
(ii) a lysosomal peptide or protein, wherein the lysosomal peptide or protein is tripeptidyl-peptidase 1,
   wherein the targeting moiety is Basic Fibroblast Growth Factor (bFGF) comprising the amino acid sequence according to SEQ ID NO. 4 (GHFKDPKRLYCKNGGF).

In a second aspect the present invention relates to a chimeric molecule according to the first aspect, wherein the growth factor is modified and lysosomal targeting is improved.

In a third aspect the present invention relates to a chimeric molecule according to the first or the second aspect, wherein the targeting moiety and the enzyme moiety are covalently linked to each other.

In a fourth aspect the present invention relates to a chimeric molecule according to any one of the aspects from the first to the third aspect, wherein the chimeric molecule is a single polypeptide chain.

In a fifth aspect the present invention relates to a chimeric molecule according to any one of the aspects from the first to the fourth aspect, wherein the targeting moiety and the enzyme moiety are linked via a peptide linker.

In a sixth aspect the present invention relates to the chimeric molecule according to any one of the aspects from the first to the fifth aspect, wherein the peptide linker comprises a protease cleavage site.

In an seventh aspect the present invention relates to a chimeric molecule according to any one of the aspects from the first to the sixth aspect, wherein the protease cleavage site is that of a protease selected from the group consisting of factor Xa, thrombin, trypsin, papain and plasmin.

In a seventh aspect the present invention relates to a chimeric molecule according to any one of aspects from the first to the eighth aspect, wherein the targeting moiety is a polypeptide having a sequence according to any one of SEQ ID NO. 24, 26, 28 and 30.

In an eighth aspect the present invention relates to a molecule according to any one of the aspects from the first to the seventh aspect, wherein the enzyme moiety (lysosomal protein or peptide) is a polypeptide having a sequence according to SEQ ID NO. 52.

In a ninth aspect the present invention relates to a chimeric molecule according to the seventh or the eighth aspect, wherein the polypeptide has a sequence according to any one of the SEQ ID NO. 36, 38, 40 and 42.

In a tenth aspect the present invention relates to a polynucleotide encoding the chimeric molecule according to any one of the aspects from the first to the ninth aspect.

In an eleventh aspect the invention relates to a polynucleotide according to thirteenth aspect having the sequence according to any one of the SEQ ID NO. 35, 37, 39 and 41.

In a twelfth aspect the present invention relates to a pharmaceutical composition comprising a chimeric molecule according to any one of the aspect from the first to the ninth aspect.

In a thirteenth aspect the present invention relates to a chimeric molecule according to any one of the aspects from the first to the ninth aspect for the use in the treatment of a disease.

In a fourteenth aspect the present invention relates to a chimeric molecule for the use in the treatment of a disease according to the thirteenth aspect, wherein the disease is a lysosomal storage disease.

In a fifteenth aspect the present invention relates to a chimeric molecule for the use in the treatment of a disease according to the fourteenth aspect, wherein the lysosomal storage disease is selected from the group consisting of the neuronal ceroid lipofuscinoses (NCL), infantile NCL (CLN1-defect), late infantile NCL (CLN2-defect), late infantile NCL (CLN5-defect), NCL caused by cathepsin D deficiency (CLN10-defect), mucopolysaccharidosis type I, mucopolysaccharidosis type II, mucopolysaccharidosis type IIIA, mucopolysaccharidosis type IIIB, mucopolysaccharidosis type IIIC, mucopolysaccharidosis type IIID, mucopolysaccharidosis type IVA, mucopolysaccharidosis type IVB, mucopolysaccharidosis type VI, mucopolysaccharidosis type VII, fucosidosis, α-mannosidosis, β-mannosidosis, aspartylglucosaminuria, Schindler's disease, Sialidosis (Mucolipidosis I), galaktosialidosis, GM₁-gangliosidosis / mucopolysaccharidosis type IVB, GM₂-gangliosidosis, Sandhoff disease, Tay-Sachs disease, metachromatic leukodystrophy, Krabbe disease, Gaucher disease, Fabry disease, Niemann-Pick disease typeA+B, glycogen storage disease type II (Pompe disease), Faber's syndrome, Wolman disease, X-linked ichthyosis.

In a sixteenth aspect the present invention relates to a chimeric molecule for the use in the treatment of a disease according to the fifteenth aspect, wherein the lysosomal storage disease is the late infantile form of neuronal ceroid lipofuscinosis and the enzyme moiety comprises lysosomal tripeptidyl peptidase 1 (TPP1).

In a seventeenth aspect the present invention relates to a chimeric molecule for the use in the treatment of a disease according to any one of the aspects from the thirteenth to the sixteenth aspect, wherein the chimeric molecule is administered intraventricularly, by use of an Ommaya reservoir, a Rickham capsule or a similar device known by those skilled in the art.

In an eighteenth aspect the present invention relates to the use of the chimeric molecule according to any one of the aspects from the first to the ninth aspect for the manufacture of a medicament.

### EXAMPLES

### Example 1

The medium to be purified is adjusted to a pH-value of 6.0 using a phosphate buffer (final concentration 20mM; stock solution: KH₂PO₄, 1 M, pH 4.5 and K₂HPO₄ 1 M pH 9). After centrifugation for 10 min at 40.000 g and 4 °C, the medium is filtrated through a 0.2 µm filter and then degassed. The supernatant, having a maximum NaCl concentration of 100 mM, is applied to a cation exchange column (for example Resource S). The flow-through is collected.

The column is then washed with 10 column volumes of a 20 mM phosphate buffer (pH 6, 100 mM NaCl). A further washing step using an intermediate gradient of 100 to 150 mM NaCl over 5 column volumes is applied. Elution is achieved by applying a linear gradient of 150 to 500 mM NaCl over 20 column volumes (1 ml fractions are collected). A final step of 1 M NaCl over 10 column volumes is applied. UV and salt gradient are monitored during the entire elution process.

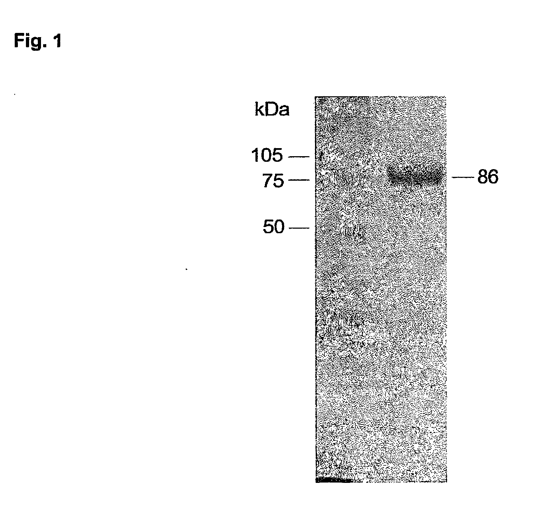

Fractions containing the fusion protein are pooled and adjusted to pH 7.5 using phosphate buffer. Fig. 1 shows a purified sample of the TPP1-FGF2 fusion protein.

### Example 2

The medium is adjusted to a pH of 7.5 using a 20 mM phosphate buffer, centrifuged for 10 min at 40.000 g and 4°C, filtrated through a 0.2 µm filter and then degassed. The supernatant is diluted with 1 volume of 20 mM phosphate buffer (pH 7.5) so that the diluted supernatant has a maximum NaCl concentration of 80 mM. The diluted supernatant is then applied to an anion exchange column (for example Resource Q). The column is subsequently washed with 10 column volumes of phosphate buffer (pH 7.5; 80 mM NaCl) followed by an intermediate NaCl gradient of 80 to 150 mM NaCl over 10 column volumes. For elution, the a linear gradient of 150-500 mM NaCl over 20 column volumes is applied (1 ml fractions are collected, peak between 200-300 mM NaCl) with a subsequent adjustment to 1 M NaCl over 10 column volumes. UV and salt gradient are monitored during the entire elution process.

### Example 3

The medium is adjusted to a pH of 7.5 using 20 mM phosphate buffer. The final NaCl concentration is adjusted to 800 mM NaCl. The medium is then centrifuged for 10 min at 40.000 g and 4 °C, followed by filtration through a 0.2 µm filter and subsequent degassing. The filtered supernatant is then applied to a Heparin-Sepharose-column (flow rate 1 ml/min), the flow-through is collected.

Purification is continued by applying 10 column volumes of 20 mM phosphate buffer (pH 7.5, 800 mM NaCl). For elution a linear gradient of 0.8 - 2 M NaCl over 20 column volumes is applied (1 ml fractions are collected, peak between 1.5 and 1.8 M NaCl), followed by a 2 M NaCl step over 10 column volumes. UV and salt gradient are monitored during the entire elution process.

After subsequent desalting and buffer exchange to PBS (pH 7.5) using gel filtration or ultrafiltration, the TPP1-FGF2 fusion proteins are aliquoted and stored at -70 °C.

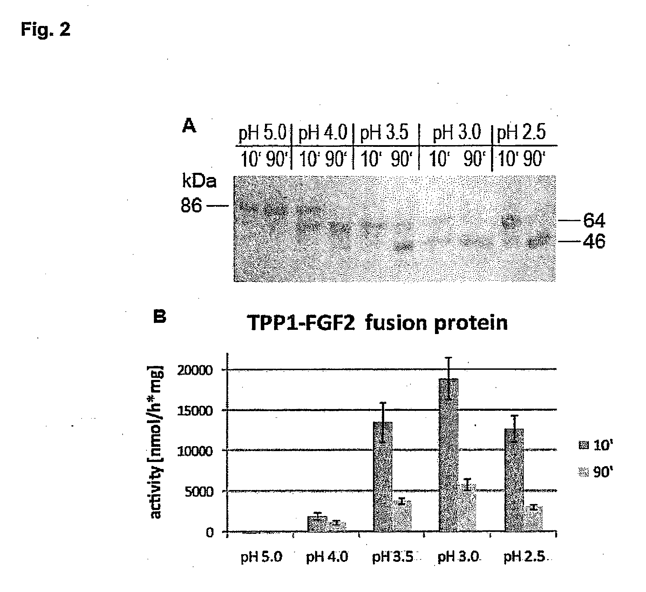

For further characterisation of the fusion proteins, the enzymatic activities are examined by a standardized enzyme assay. The pH dependent auto-activation of the TPP1-FGF2 proproteins was comparable to that of the TPP1 wild-type. Fig. 2 illustrates the-auto-processing of a TPP1-FGF2 fusion protein.

### Example 4

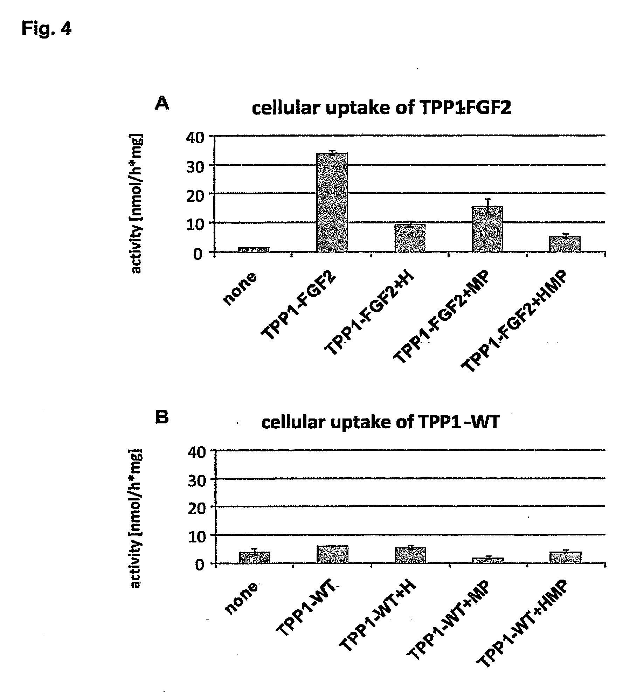

Furthermore, endocytosis into human neuronal progenitor cells (NT2 cells) was compared for TPP1-FGF2 fusion proteins and the TPP1 wild-type. At a final concentration of 0.4-0.5 µM TPP1-FGF2 fusion protein or TPP1 wild-type protein, respectively, was added to the medium. After 48 hours of incubation the intracellular TPP1-activity was measured (see Fig. 4). TPP1-activity was six times higher in cell lysates of the NT2-cells which were treated with TPP1-FGF2 fusion proteins than for the TPP1 wild-type protein. It was possible to inhibit the intracellular TPP1-activity by heparin either alone or in combination with mannose-6-phosphate. The results show that the cellular uptake of the TPP1-FGF2 fusion protein is mainly mediated by cell surface HSPG.

### Example 5

Finally, the effect of the TPP1-FGF1 fusion proteins was also examined in an animal model, namely *tpp1-*/*-* mice. In weekly intervals, the *tpp1*-/- mice were injected intraventricularly with 10 µg of TPP1-FGF2 fusion protein or TPP1 wild-type protein, respectively. Mice treated with TPP1-FGF2 showed a significantly higher life expectancy as compared to mice treated with the TPP1 wild-type protein (Fig. 5).

Moreover, *tpp1-*/*-* mice treated with TPP1-FGF2-fusion proteins showed a delayed course of illness in comparison to *tpp1-*/*-* mice treated with the TPP1 wild-type. This result was tested by checking the motor coordination with a so called Rotor Rod (a rotating pole) (Fig. 6). As of the 17^{th} week, *tpp1*-/- mice treated with the TPP1-FGF2 fusion protein were able to stay longer on the Rotor Rod than the *tpp1-*/*-* mice treated with the TPP1 wild-type.

## Claims

1. A chimeric molecule comprising
(i) a targeting moiety that binds to heparin or heparan sulfate proteoglycans; and
(ii) a lysosomal peptide or protein, wherein the lysosomal peptide or protein is tripeptidyl-peptidase 1,
wherein the targeting moiety is Basic Fibroblast Growth Factor (bFGF) comprising the amino acid sequence according to SEQ ID NO. 4 (GHFKDPKRLYCKNGGF).

2. Chimeric molecule according to claim 1, wherein the targeting moiety and the enzyme moiety are covalently linked to each other.

3. Chimeric molecule according to claim 1 or 2, wherein the chimeric molecule is a single polypeptide chain.

4. Chimeric molecule according to any one of claims 1 to 3, wherein the targeting moiety and the enzyme moiety are linked via a peptide linker.

5. Chimeric molecule according to any one of claims 1 to 4, wherein the targeting moiety is a polypeptide having a sequence according to any one of SEQ ID NO. 24, 26, 28 and 30.

6. Chimeric molecule according to any one of claims 1 to 5, wherein the enzyme moiety is a polypeptide having a sequence according to SEQ ID NO. 52.

7. Chimeric molecule according to claims 5 or 6, wherein the polypeptide has a sequence according to any one of the SEQ ID NO. 36, 38, 40, and 42.

8. Polynucleotide encoding the chimeric molecule according to any one of claim 1 to 7.

9. Polynucleotide according to claim 8 having the sequence according to any one of the SEQ ID NO. 35, 37, 39, and 41.

10. Pharmaceutical composition comprising a chimeric molecule according to any one of the claims 1 to 9.

11. Chimeric molecule according to any one of the claims 1 to 7 for the use in the treatment of a disease.

12. Chimeric molecule for the use in the treatment of a disease according to claim 11, wherein the disease is a lysosomal storage disease.

13. Chimeric molecule for the use in the treatment of a lysosomal storage disease according to claim 12, wherein the lysosomal storage disease is late infantile neuronal ceroid lipofuscinose (CLN2-defect).

14. The use of the chimeric molecule according to any one of the claims 1 to 7 for the manufacture of a medicament.

## Patentansprüche

1. Chimäres Molekül, umfassend
(i) eine Targeting-Einheit, die an Heparin oder Heparansulfat-Proteoglykane bindet; und
(ii) ein lysosomales Peptid oder Protein, wobei das lysosomale Peptid oder Protein Tripeptidyl-Peptidase 1 ist,
wobei die Targeting-Einheit basischer Fibroblasten-Wachstumsfaktor (Basic Fibroblast Growth Factor; bFGF) ist, der die Aminosäuresequenz nach SEQ ID NO:4 (GHFKDPKRLYCKNGGF) umfasst.

2. Chimäres Molekül nach Anspruch 1, wobei die Targeting-Einheit und die Enzymeinheit kovalent miteinander verbunden sind.

3. Chimäres Molekül nach Anspruch 1 oder 2, wobei das chimäre Molekül eine einzelne Polypeptidkette ist.

4. Chimäres Molekül nach einem der Ansprüche 1 bis 3, wobei die Targeting-Einheit und die Enzymeinheit über einen Peptidlinker verbunden sind.

5. Chimäres Molekül nach einem der Ansprüche 1 bis 4, wobei die Targeting-Einheit ein Polypeptid ist, das eine Sequenz nach einer der SEQ ID NOs:24, 26, 28 und 30 hat.

6. Chimäres Molekül nach einem der Ansprüche 1 bis 5, wobei die Enzymeinheit ein Polypeptid ist, das eine Sequenz nach SEQ ID NO:52 hat.

7. Chimäres Molekül nach Anspruch 5 oder 6, wobei das Polypeptid eine Sequenz nach einer der SEQ ID NOs:36, 38, 40 und 42 hat.

8. Polynucleotid, das ein chimäres Molekül nach einem der Ansprüche 1 bis 7 codiert.

9. Polynucleotid nach Anspruch 8, das eine Sequenz nach einer der SEQ ID NOs:35, 37, 39 und 41 hat.

10. Pharmazeutische Zusammensetzung, die ein chimäres Molekül nach einem der Ansprüche 1 bis 9 umfasst.

11. Chimäres Molekül nach einem der Ansprüche 1 bis 7 zur Verwendung in der Behandlung einer Erkrankung.

12. Chimäres Molekül zur Verwendung in der Behandlung einer Erkrankung nach Anspruch 11, wobei die Erkrankung eine lysosomale Speicherkrankheit ist.

13. Chimäres Molekül zur Verwendung in der Behandlung einer lysosomalen Speicherkrankheit nach Anspruch 12, wobei die lysosomale Speicherkrankheit spätinfantile neuronale Lipofuszinose (CLN2-Defekt) ist.

14. Verwendung des chimären Moleküls nach einem der Ansprüche 1 bis 7 zur Herstellung eines Medikaments.

## Revendications

1. Molécule chimérique comprenant
(i) un fragment de ciblage qui se lie à l'héparine ou à des protéoglycanes à sulfate d'héparane ; et
(ii) un peptide ou une protéine lysosomal(e), où le peptide ou la protéine lysosomal(e) est la tripeptidyl-peptidase 1,
où le fragment de ciblage est le facteur de croissance des fibroblastes basique (bFGF) comprenant la séquence d'acides aminés selon SEQ ID NO: 4 (GHFKDPKRLYCKNGGF).

2. Molécule chimérique selon la revendication 1, dans laquelle le fragment de ciblage et le fragment enzymatique sont liés l'un à l'autre de façon covalente.

3. Molécule chimérique selon la revendication 1 ou 2, où la molécule chimérique est une seule chaîne polypeptidique.

4. Molécule chimérique selon l'une quelconque des revendications 1 à 3, dans laquelle le fragment de ciblage et le fragment enzymatique sont liés via une séquence de liaison peptidique.

5. Molécule chimérique selon l'une quelconque des revendications 1 à 4, dans laquelle le fragment de ciblage est un polypeptide ayant une séquence selon l'une quelconque des SEQ ID NO: 24, 26, 28 et 30.

6. Molécule chimérique selon l'une quelconque des revendications 1 à 5, dans laquelle le fragment enzymatique est un polypeptide ayant une séquence selon SEQ ID NO: 52.

7. Molécule chimérique selon les revendications 5 ou 6, dans laquelle le polypeptide possède une séquence selon l'une quelconque des SEQ ID NO: 36, 38, 40 et 42.

8. Polynucléotide codant pour la molécule chimérique selon l'une quelconque des revendications 1 à 7.

9. Polynucléotide selon la revendication 8 ayant la séquence selon l'une quelconque des SEQ ID NO: 35, 37, 39 et 41.

10. Composition pharmaceutique comprenant une molécule chimérique selon l'une quelconque des revendications 1 à 9.

11. Molécule chimérique selon l'une quelconque des revendications 1 à 7 destinée à être utilisée dans le traitement d'une maladie.

12. Molécule chimérique destinée à être utilisée dans le traitement d'une maladie selon la revendication 11, où la maladie est une maladie de surcharge lysosomale.

13. Molécule chimérique destinée à être utilisée dans le traitement d'une maladie de surcharge lysosomale selon la revendication 12, où la maladie de surcharge lysosomale est la céroïde-lipofuscinose neuronale infantile tardive (défaut de CLN2).

14. Utilisation de la molécule chimérique selon l'une quelconque des revendications 1 à 7 pour la fabrication d'un médicament.


### SEQUENCE LISTING

<110> Steinfeld, Robert
<120> SUBSTANCES AND METHODS FOR THE TREATMENT OF LYSOSMAL STORAGE DISEASES
<130> R2546 PCT BLN
<150> EP 09 17 9047.7
   <151> 2009-12-14
<160> 74
<170> PatentIn version 3.5
<210> 1
   <211> 16
   <212> PRT
   <213> Homo sapiens
<400> 1
   Lys Ile Arg Ser Glu Phe Lys Lys Lys Tyr Gly Lys Ser Leu Tyr Tyr
   1               5                   10                  15
<210> 2
   <211> 16
   <212> PRT
   <213> Homo sapiens
<400> 2
   Gln Arg Phe Arg His Arg Asn Arg Lys Gly Tyr Arg Ser Gln Arg Gly
   1               5                   10                  15
<210> 3
   <211> 16
   <212> PRT
   <213> Homo sapiens
<400> 3
   Lys Phe Ile Ile Pro Ser Pro Lys Arg Pro Val Lys Leu Leu Ser Gly
   1               5                   10                  15
<210> 4
   <211> 16
   <212> PRT
   <213> Homo sapiens
<400> 4
   Gly His Phe Lys Asp Pro Lys Arg Leu Tyr Cys Lys Asn Gly Gly Phe
   1 5 10 15
<210> 5
   <211> 16
   <212> PRT
   <213> Homo sapiens
<400> 5
   Asp Gly Gly Ser Pro Ile Arg His Tyr Leu Ile Lys Tyr Lys Ala Lys
   1 5 10 15
<210> 6
   <211> 16
   <212> PRT
   <213> Homo sapiens
<400> 6
   Gly Leu Ser Glu Lys Thr Leu Arg Lys Trp Leu Lys Met Phe Lys Lys
   1 5 10 15
<210> 7
   <211> 16
   <212> PRT
   <213> Homo sapiens
<400> 7
   Lys Leu Asn Cys Arg Leu Tyr Arg Lys Ala Asn Lys Ser Ser Lys Leu
   1 5 10 15
<210> 8
   <211> 16
   <212> PRT
   <213> Homo sapiens
<400> 8
   Ser His Leu Arg Lys Leu Arg Lys Arg Leu Leu Arg Asp Ala Asp Asp
   1 5 10 15
<210> 9
   <211> 16
   <212> PRT
   <213> Homo sapiens
<400> 9
   Gly His Arg Pro Leu Asp Lys Lys Arg Glu Glu Ala Pro Ser Leu Arg
   1 5 10 15
<210> 10
   <211> 15
   <212> PRT
   <213> Homo sapiens
<400> 10
   Ser Arg Gly Lys Gly Arg Arg Gly Gln Arg Gly Lys Asn Arg Gly
   1 5 10 15
<210> 11
   <211> 16
   <212> PRT
   <213> Homo sapiens
<400> 11
   Pro Asp Ala Pro Arg Ile Lys Lys Ile Val Gln Lys Lys Leu Ala Gly
   1 5 10 15
<210> 12
   <211> 16
   <212> PRT
   <213> Homo sapiens
<400> 12
   Asp Lys Lys Gly Phe Tyr Lys Lys Lys Gln Cys Arg Pro Ser Lys Gly
   1 5 10 15
<210> 13
   <211> 16
   <212> PRT
   <213> Homo sapiens
<400> 13
   Arg Gln Ile Lys Ile Trp Phe Gln Asn Arg Arg Met Lys Trp Lys Lys
   1 5 10 15
<210> 14
   <211> 13
   <212> PRT
   <213> Homo sapiens
<400> 14
   Lys Arg Val Ser Arg Asn Lys Ser Glu Lys Lys Arg Arg
   1 5 10
<210> 15
   <211> 12
   <212> DNA
   <213> Homo sapiens
<400> 15
   agatcccccg gg 12
<210> 16
   <211> 12
   <212> DNA
   <213> Homo sapiens
<400> 16
   ggateccccg gg 12
<210> 17
   <211> 51
   <212> DNA
   <213> Homo sapiens
<400> 17
   cgccagataa agatttggtt ccagaatcgg cgcatgaagt ggaagaagta a 51
<210> 18
   <211> 16
   <212> PRT
   <213> Homo sapiens
<400> 18
   Arg Gln Ile Lys Ile Trp Phe Gln Asn Arg Arg Met Lys Trp Lys Lys
   1 5 10 15
<210> 19
   <211> 42
   <212> DNA
   <213> Homo sapiens
<400> 19
   aaaagagtat ctagaaacaa atctgaaaag aaacgtagat aa 42
<210> 20
   <211> 13
   <212> PRT
   <213> Homo sapiens
<400> 20
   Lys Arg Val Ser Arg Asn Lys Ser Glu Lys Lys Arg Arg
   1 5 10
<210> 21
   <211> 30
   <212> DNA
   <213> Homo sapiens
<400> 21
   agatccgtcg acatcgaagg tagaggcatt 30
<210> 22
   <211> 30
   <212> DNA
   <213> Homo sapiens
<400> 22
   ggatccgtcg acatcgaagg tagaggcatt 30
<210> 23
   <211> 441
   <212> DNA
   <213> Homo sapiens
<400> 23
   cccgccttgc ccgaggatgg cggcagcggc gccttcccgc ccggccactt caaggacccc      60
   aagcggctgt actgcaaaaa cgggggcttc ttcctgcgca tccaccccga cggccgagtt     120
   gacggggtcc gggagaagag cgaccctcac atcaagctac aacttcaagc agaagagaga     180
   ggagttgtgt ctatcaaagg agtgtctgct aaccgttacc tggctatgaa ggaagatgga     240
   agattactgg cttctaaatc tgttacggat gagtgtttct tttttgaacg attggaatct     300
   aataactaca atacttaccg gtcaaggaaa tacaccagtt ggtatgtggc actgaaacga     360
   actgggcagt ataaacttgg ctccaaaaca ggacctgggc agaaagctat actttttctt     420
   ccaatgtctg ctaagagctg a                                               441
<210> 24
   <211> 145
   <212> PRT
   <213> Homo sapiens
<400> 24
   Pro Ala Leu Pro Glu Asp Gly Gly Ser Gly Ala Phe Pro Pro Gly His
   1               5                   10                  15
   Phe Lys Asp Pro Lys Arg Leu Tyr Cys Lys Asn Gly Gly Phe Phe Leu
               20                  25                  30
   Arg Ile His Pro Asp Gly Arg Val Asp Gly Val Arg Glu Lys Ser Asp
           35                  40                  45
   Pro His Ile Lys Leu Gln Leu Gln Ala Glu Glu Arg Gly Val Val Ser
       50                  55                  60
   Ile Lys Gly Val Ser Ala Asn Arg Tyr Leu Ala Met Lys Glu Asp Gly
   65                  70                  75                  80
   Arg Leu Leu Ala Ser Lys Ser Val Thr Asp Glu Cys Phe Phe Phe Glu
                   85                  90                  95
   Arg Leu Glu Ser Asn Asn Tyr Asn Thr Tyr Arg Ser Arg Lys Tyr Thr
               100                 105                 110
   Ser Trp Tyr Val Ala Leu Lys Arg Thr Gly Gln Tyr Lys Leu Gly Ser
           115                 120                 125
   Lys Thr Gly Pro Gly Gln Lys Ala Ile Leu Phe Leu Pro Met Ser Ala
       130                 135                 140
   Lys
   145
<210> 25
   <211> 441
   <212> DNA
   <213> Homo sapiens
<400> 25
   cccgccttgc ccgaggatgg cggcagcggc gccttcccgc ccggccactt caaggacccc 60
   aagcggctgt actgcaaaaa cgggggcttc ttcctgcgca tccaccccga cggccgagtt 120
   gacggggtcc gggagaagag cgaccctcac atcaagctac aacttcaagc agaagagaga 180
   ggagttgtgt ctatcaaagg agtgtctgct aaccgttacc tggctatgaa ggaagatgga 240
   agattactgg cttctaaatc tgttacggat gagtgtttct tttttgcacg attggaatct 300
   aataactaca atacttaccg gtcaaggaaa tacaccagtt ggtatgtggc actgaaacga 360
   actgggcagt ataaacttgg ctccaaaaca ggacctgggc agaaagctat actttttctt 420
   ccaatgtctg ctaagagctg a 441
<210> 26
   <211> 146
   <212> PRT
   <213> Homo sapiens
<400> 26
   Pro Ala Leu Pro Glu Asp Gly Gly Ser Gly Ala Phe Pro Pro Gly His
   1               5                   10                  15
   Phe Lys Asp Pro Lys Arg Leu Tyr Cys Lys Asn Gly Gly Phe Phe Leu
               20                  25                  30
   Arg Ile His Pro Asp Gly Arg Val Asp Gly Val Arg Glu Lys Ser Asp
           35                  40                  45
   Pro His Ile Lys Leu Gln Leu Gln Ala Glu Glu Arg Gly Val Val Ser
       50                  55                  60
   Ile Lys Gly Val Ser Ala Asn Arg Tyr Leu Ala Met Lys Glu Asp Gly
   65                  70                  75                  80
   Arg Leu Leu Ala Ser Lys Ser Val Thr Asp Glu Cys Phe Phe Phe Ala
                   85                  90                  95
   Arg Leu Glu Ser Asn Asn Tyr Asn Thr Tyr Arg Ser Arg Lys Tyr Thr
               100                 105                 110
   Ser Trp Tyr Val Ala Leu Lys Arg Thr Gly Gln Tyr Lys Leu Gly Ser
           115                 120                 125
   Lys Thr Gly Pro Gly Gln Lys Ala Ile Leu Phe Leu Pro Met Ser Ala
       130                 135                 140
   Lys Ser
   145
<210> 27
   <211> 441
   <212> DNA
   <213> Homo sapiens
<400> 27
   cccgccttgc ccgaggatgg cggcagcggc gccttcccgc ccggccactt caaggacccc      60
   aagcggctgt actgcaaaaa cgggggcttc ttcctgcgca tccaccccga cggccgagtt     120
   gacgggacaa gggacaggag cgaccagcac attcagctgc agctcagtgc agaagagaga     180
   ggagttgtgt ctatcaaagg agtgtctgct aaccgttacc tggctatgaa ggaagatgga     240
   agattactgg cttctaaatc tgttacggat gagtgtttct tttttgaacg attggaatct     300
   aataactaca atacttaccg gtcaaggaaa tacaccagtt ggtatgtggc actgaaacga     360
   actgggcagt ataaacttgg ctccaaaaca ggacctgggc agaaagctat actttttctt     420
   ccaatgtctg ctaagagctg a                                               441
<210> 28
   <211> 146
   <212> PRT
   <213> Homo sapiens
<400> 28
   Pro Ala Leu Pro Glu Asp Gly Gly Ser Gly Ala Phe Pro Pro Gly His
   1               5                   10                  15
   Phe Lys Asp Pro Lys Arg Leu Tyr Cys Lys Asn Gly Gly Phe Phe Leu
               20                  25                  30
   Arg Ile His Pro Asp Gly Arg Val Asp Gly Thr Arg Asp Arg Ser Asp
           35                  40                  45
   Gln His Ile Gln Leu Gln Leu Ser Ala Glu Glu Arg Gly Val Val Ser
       50                  55                  60
   Ile Lys Gly Val Ser Ala Asn Arg Tyr Leu Ala Met Lys Glu Asp Gly
   65                  70                  75                  80
   Arg Leu Leu Ala Ser Lys Ser Val Thr Asp Glu Cys Phe Phe Phe Glu
                   85                  90                  95
   Arg Leu Glu Ser Asn Asn Tyr Asn Thr Tyr Arg Ser Arg Lys Tyr Thr
               100                 105                 110
   Ser Trp Tyr Val Ala Leu Lys Arg Thr Gly Gln Tyr Lys Leu Gly Ser
           115                 120                 125
   Lys Thr Gly Pro Gly Gln Lys Ala Ile Leu Phe Leu Pro Met Ser Ala
       130                 135                 140
   Lys Ser
   145
<210> 29
   <211> 441
   <212> DNA
   <213> Homo sapiens
<400> 29
   cccgccttgc ccgaggatgg cggcagcggc gccttcccgc ccggccactt caaggacccc      60
   aagcggctgt actgcaaaaa cgggggcttc ttcctgcgca tccaccccga cggccgagtt     120
   gacgggacaa gggacaggag cgaccagcac attcagctgc agctcagtgc agaagagaga     180
   ggagttgtgt ctatcaaagg agtgtctgct aaccgttacc tggctatgaa ggaagatgga     240
   agattactgg cttctaaatc tgttacggat gagtgtttct tttttgcacg attggaatct     300
   aataactaca atacttaccg gtcaaggaaa tacaccagtt ggtatgtggc actgaaacga     360
   actgggcagt ataaacttgg ctccaaaaca ggacctgggc agaaagctat actttttctt     420
   ccaatgtctg ctaagagctg a                                               441
<210> 30
   <211> 146
   <212> PRT
   <213> Homo sapiens
<400> 30
   Pro Ala Leu Pro Glu Asp Gly Gly Ser Gly Ala Phe Pro Pro Gly His
   1 5 10 15
   Phe Lys Asp Pro Lys Arg Leu Tyr Cys Lys Asn Gly Gly Phe Phe Leu
   20 25 30
   Arg Ile His Pro Asp Gly Arg Val Asp Gly Thr Arg Asp Arg Ser Asp
   35 40 45
   Gln His Ile Gln Leu Gln Leu Ser Ala Glu Glu Arg Gly Val Val Ser
   50 55 60
   Ile Lys Gly Val Ser Ala Asn Arg Tyr Leu Ala Met Lys Glu Asp Gly
   65 70 75 80
   Arg Leu Leu Ala Ser Lys Ser Val Thr Asp Glu Cys Phe Phe Phe Ala
   85 90 95
   Arg Leu Glu Ser Asn Asn Tyr Asn Thr Tyr Arg Ser Arg Lys Tyr Thr
   100 105 110
   Ser Trp Tyr Val Ala Leu Lys Arg Thr Gly Gln Tyr Lys Leu Gly Ser
   115 120 125
   Lys Thr Gly Pro Gly Gln Lys Ala Ile Leu Phe Leu Pro Met Ser Ala
   130 135 140
   Lys Ser
   145
<210> 31
   <211> 1752
   <212> DNA
   <213> Homo sapiens
<400> 31
   atgggactcc aagcctgcct cctagggctc tttgccctca tcctctctgg caaatgcagt 60
   tacagcccgg agcccgacca gcggaggacg ctgcccccag gctgggtgtc cctgggccgt 120
   gcggaccctg aggaagagct gagtctcacc tttgccctga gacagcagaa tgtggaaaga 180
   ctctcggagc tggtgcaggc tgtgtcggat cccagctctc ctcaatacgg aaaatacctg 240
   accctagaga atgtggctga tctggtgagg ccatccccac tgaccctcca cacggtgcaa 300
   aaatggctct tggcagccgg agcccagaag tgccattctg tgatcacaca ggactttctg 360
   acttgctggc tgagcatccg acaagcagag ctgctgctcc ctggggctga gtttcatcac 420
   tatgtgggag gacctacgga aacccatgtt gtaaggtccc cacatcccta ccagcttcca 480
   caggccttgg ccccccatgt ggactttgtg gggggactgc accgttttcc cccaacatca 540
   tccctgaggc aacgtcctga gccgcaggtg acagggactg taggcctgca tctgggggta 600
   accccctctg tgatccgtaa gcgatacaac ttgacctcac aagacgtggg ctctggcacc 660
   agcaataaca gccaagcctg tgcccagttc ctggagcagt atttccatga ctcagacctg 720
   gctcagttca tgcgcctctt cggtggcaac tttgcacatc aggcatcagt agcccgtgtg 780
   gttggacaac agggccgggg ccgggccggg attgaggcca gtctagatgt gcagtacctg 840
   atgagtgctg gtgccaacat ctccacctgg gtctacagta gccctggccg gcatgaggga 900
   caggagccct tcctgcagtg gctcatgctg ctcagtaatg agtcagccct gccacatgtg 960
   catactgtga gctatggaga tgatgaggac tccctcagca gcgcctacat ccagcgggtc 1020
   aacactgagc tcatgaaggc tgccgctcgg ggtctcaccc tgctcttcgc ctcaggtgac 1080
   agtggggccg ggtgttggtc tgtctctgga agacaccagt tccgccctac cttccctgcc 1140
   tccagcccct atgtcaccac agtgggaggc acatccttcc aggaaccttt cctcatcaca 1200
   aatgaaattg ttgactatat cagtggtggt ggcttcagca atgtgttccc acggccttca 1260
   taccaggagg aagctgtaac gaagttcctg agctctagcc cccacctgcc accatccagt 1320
   tacttcaatg ccagtggccg tgcctaccca gatgtggctg cactttctga tggctactgg 1380
   gtggtcagca acagagtgcc cattccatgg gtgtccggaa cctcggcctc tactccagtg 1440
   tttgggggga tcctatcctt gatcaatgag cacaggatcc ttagtggccg cccccctctt 1500
   ggctttctca acccaaggct ctaccagcag catggggcag gactctttga tgtaacccgt 1560
   ggctgccatg agtcctgtct ggatgaagag gtagagggcc agggtttctg ctctggtcct 1620
   ggctgggatc ctgtaacagg ctggggaaca cccaacttcc cagctttgct gaagactcta 1680
   ctcaacccca gatcccccgg gcgccagata aagatttggt tccagaatcg gcgcatgaag 1740
   tggaagaagt aa 1752
<210> 32
   <211> 583
   <212> PRT
   <213> Homo sapiens
<400> 32
   Met Gly Leu Gln Ala Cys Leu Leu Gly Leu Phe Ala Leu Ile Leu Ser
   1               5                   10                  15
   Gly Lys Cys Ser Tyr Ser Pro Glu Pro Asp Gln Arg Arg Thr Leu Pro
               20                  25                  30
   Pro Gly Trp Val Ser Leu Gly Arg Ala Asp Pro Glu Glu Glu Leu Ser
           35                  40                  45
   Leu Thr Phe Ala Leu Arg Gln Gln Asn Val Glu Arg Leu Ser Glu Leu
       50                  55                  60
   Val Gln Ala Val Ser Asp Pro Ser Ser Pro Gln Tyr Gly Lys Tyr Leu
   65                  70                  75                  80
   Thr Leu Glu Asn Val Ala Asp Leu Val Arg Pro Ser Pro Leu Thr Leu
                   85                  90                  95
   His Thr Val Gln Lys Trp Leu Leu Ala Ala Gly Ala Gln Lys Cys His
               100                 105                 110
   Ser Val Ile Thr Gln Asp Phe Leu Thr Cys Trp Leu Ser Ile Arg Gln
           115                 120                 125
   Ala Glu Leu Leu Leu Pro Gly Ala Glu Phe His His Tyr Val Gly Gly
       130                 135                 140
   Pro Thr Glu Thr His Val Val Arg Ser Pro His Pro Tyr Gln Leu Pro
   145                 150                 155                 160
   Gln Ala Leu Ala Pro His Val Asp Phe Val Gly Gly Leu His Arg Phe
                   165                 170                 175
   Pro Pro Thr Ser Ser Leu Arg Gln Arg Pro Glu Pro Gln Val Thr Gly
               180                 185                 190
   Thr Val Gly Leu His Leu Gly Val Thr Pro Ser Val Ile Arg Lys Arg
           195                 200                 205
   Tyr Asn Leu Thr Ser Gln Asp Val Gly Ser Gly Thr Ser Asn Asn Ser
   210 215 220
   Gln Ala Cys Ala Gln Phe Leu Glu Gln Tyr Phe His Asp Ser Asp Leu
   225 230 235 240
   Ala Gln Phe Met Arg Leu Phe Gly Gly Asn Phe Ala His Gln Ala Ser
   245 250 255
   Val Ala Arg Val Val Gly Gln Gln Gly Arg Gly Arg Ala Gly Ile Glu
   260 265 270
   Ala Ser Leu Asp Val Gln Tyr Leu Met Ser Ala Gly Ala Asn Ile Ser
   275 280 285
   Thr Trp Val Tyr Ser Ser Pro Gly Arg His Glu Gly Gln Glu Pro Phe
   290 295 300
   Leu Gln Trp Leu Met Leu Leu Ser Asn Glu Ser Ala Leu Pro His Val
   305 310 315 320
   His Thr Val Ser Tyr Gly Asp Asp Glu Asp Ser Leu Ser Ser Ala Tyr
   325 330 335
   Ile Gln Arg Val Asn Thr Glu Leu Met Lys Ala Ala Ala Arg Gly Leu
   340 345 350
   Thr Leu Leu Phe Ala Ser Gly Asp Ser Gly Ala Gly Cys Trp Ser Val
   355 360 365
   Ser Gly Arg His Gln Phe Arg Pro Thr Phe Pro Ala Ser Ser Pro Tyr
   370 375 380
   Val Thr Thr Val Gly Gly Thr Ser Phe Gln Glu Pro Phe Leu Ile Thr
   385 390 395 400
   Asn Glu Ile Val Asp Tyr Ile Ser Gly Gly Gly Phe Ser Asn Val Phe
   405 410 415
   Pro Arg Pro Ser Tyr Gln Glu Glu Ala Val Thr Lys Phe Leu Ser Ser
   420 425 430
   Ser Pro His Leu Pro Pro Ser Ser Tyr Phe Asn Ala Ser Gly Arg Ala
   435 440 445
   Tyr Pro Asp Val Ala Ala Leu Ser Asp Gly Tyr Trp Val Val Ser Asn
   450 455 460
   Arg Val Pro Ile Pro Trp Val Ser Gly Thr Ser Ala Ser Thr Pro Val
   465 470 475 480
   Phe Gly Gly Ile Leu Ser Leu Ile Asn Glu His Arg Ile Leu Ser Gly
   485 490 495
   Arg Pro Pro Leu Gly Phe Leu Asn Pro Arg Leu Tyr Gln Gln His Gly
   500 505 510
   Ala Gly Leu Phe Asp Val Thr Arg Gly Cys His Glu Ser Cys Leu Asp
   515 520 525
   Glu Glu Val Glu Gly Gln Gly Phe Cys Ser Gly Pro Gly Trp Asp Pro
   530 535 540
   Val Thr Gly Trp Gly Thr Pro Asn Phe Pro Ala Leu Leu Lys Thr Leu
   545 550 555 560
   Leu Asn Pro Arg Ser Pro Gly Arg Gln Ile Lys Ile Trp Phe Gln Asn
   565 570 575
   Arg Arg Met Lys Trp Lys Lys
   580
<210> 33
   <211> 1743
   <212> DNA
   <213> Homo sapiens
<400> 33
   atgggactcc aagcctgcct cctagggctc tttgccctca tcctctctgg caaatgcagt 60
   tacagcccgg agcccgacca gcggaggacg ctgcccccag gctgggtgtc cctgggccgt 120
   gcggaccctg aggaagagct gagtctcacc tttgccctga gacagcagaa tgtggaaaga 180
   ctctcggagc tggtgcaggc tgtgtcggat cccagctctc ctcaatacgg aaaatacctg 240
   accctagaga atgtggctga tctggtgagg ccatccccac tgaccctcca cacggtgcaa 300
   aaatggctct tggcagccgg agcccagaag tgccattctg tgatcacaca ggactttctg 360
   acttgctggc tgagcatccg acaagcagag ctgctgctcc ctggggctga gtttcatcac 420
   tatgtgggag gacctacgga aacccatgtt gtaaggtccc cacatcccta ccagcttcca 480
   caggccttgg ccccccatgt ggactttgtg gggggactgc accgttttcc cccaacatca 540
   tccctgaggc aacgtcctga gccgcaggtg acagggactg taggcctgca tctgggggta 600
   accccctctg tgatccgtaa gcgatacaac ttgacctcac aagacgtggg ctctggcacc 660
   agcaataaca gccaagcctg tgcccagttc ctggagcagt atttccatga ctcagacctg 720
   gctcagttca tgcgcctctt cggtggcaac tttgcacatc aggcatcagt agcccgtgtg 780
   gttggacaac agggccgggg ccgggccggg attgaggcca gtctagatgt gcagtacctg      840
   atgagtgctg gtgccaacat ctccacctgg gtctacagta gccctggccg gcatgaggga      900
   caggagccct tcctgcagtg gctcatgctg ctcagtaatg agtcagccct gccacatgtg      960
   catactgtga gctatggaga tgatgaggac tccctcagca gcgcctacat ccagcgggtc     1020
   aacactgagc tcatgaaggc tgccgctcgg ggtctcaccc tgctcttcgc ctcaggtgac     1080
   agtggggccg ggtgttggtc tgtctctgga agacaccagt tccgccctac cttccctgcc     1140
   tccagcccct atgtcaccac agtgggaggc acatccttcc aggaaccttt cctcatcaca     1200
   aatgaaattg ttgactatat cagtggtggt ggcttcagca atgtgttccc acggccttca     1260
   taccaggagg aagctgtaac gaagttcctg agctctagcc cccacctgcc accatccagt     1320
   tacttcaatg ccagtggccg tgcctaccca gatgtggctg cactttctga tggctactgg     1380
   gtggtcagca acagagtgcc cattccatgg gtgtccggaa cctcggcctc tactccagtg     1440
   tttgggggga tcctatcctt gatcaatgag cacaggatcc ttagtggccg cccccctctt     1500
   ggctttctca acccaaggct ctaccagcag catggggcag gactctttga tgtaacccgt     1560
   ggctgccatg agtcctgtct ggatgaagag gtagagggcc agggtttctg ctctggtcct     1620
   ggctgggatc ctgtaacagg ctggggaaca cccaacttcc cagctttgct gaagactcta     1680
   ctcaacccca gatcccccgg gaaaagagta tctagaaaca aatctgaaaa gaaacgtaga     1740
   taa                                                                   1743
<210> 34
   <211> 580
   <212> PRT
   <213> Homo sapiens
<400> 34
   Met Gly Leu Gln Ala Cys Leu Leu Gly Leu Phe Ala Leu Ile Leu Ser
   1               5                   10                  15
   Gly Lys Cys Ser Tyr Ser Pro Glu Pro Asp Gln Arg Arg Thr Leu Pro
               20                  25                  30
   Pro Gly Trp Val Ser Leu Gly Arg Ala Asp Pro Glu Glu Glu Leu Ser
           35                  40                  45
   Leu Thr Phe Ala Leu Arg Gln Gln Asn Val Glu Arg Leu Ser Glu Leu
       50                  55                  60
   Val Gln Ala Val Ser Asp Pro Ser Ser Pro Gln Tyr Gly Lys Tyr Leu
   65                  70                  75                  80
   Thr Leu Glu Asn Val Ala Asp Leu Val Arg Pro Ser Pro Leu Thr Leu
                   85                  90                  95
   His Thr Val Gln Lys Trp Leu Leu Ala Ala Gly Ala Gln Lys Cys His
   100 105 110
   Ser Val Ile Thr Gln Asp Phe Leu Thr Cys Trp Leu Ser Ile Arg Gln
   115 120 125
   Ala Glu Leu Leu Leu Pro Gly Ala Glu Phe His His Tyr Val Gly Gly
   130 135 140
   Pro Thr Glu Thr His Val Val Arg Ser Pro His Pro Tyr Gln Leu Pro
   145 150 155 160
   Gln Ala Leu Ala Pro His Val Asp Phe Val Gly Gly Leu His Arg Phe
   165 170 175
   Pro Pro Thr Ser Ser Leu Arg Gln Arg Pro Glu Pro Gln Val Thr Gly
   180 185 190
   Thr Val Gly Leu His Leu Gly Val Thr Pro Ser Val Ile Arg Lys Arg
   195 200 205
   Tyr Asn Leu Thr Ser Gln Asp Val Gly Ser Gly Thr Ser Asn Asn Ser
   210 215 220
   Gln Ala Cys Ala Gln Phe Leu Glu Gln Tyr Phe His Asp Ser Asp Leu
   225 230 235 240
   Ala Gln Phe Met Arg Leu Phe Gly Gly Asn Phe Ala His Gln Ala Ser
   245 250 255
   Val Ala Arg Val Val Gly Gln Gln Gly Arg Gly Arg Ala Gly Ile Glu
   260 265 270
   Ala Ser Leu Asp Val Gln Tyr Leu Met Ser Ala Gly Ala Asn Ile Ser
   275 280 285
   Thr Trp Val Tyr Ser Ser Pro Gly Arg His Glu Gly Gln Glu Pro Phe
   290 295 300
   Leu Gln Trp Leu Met Leu Leu Ser Asn Glu Ser Ala Leu Pro His Val
   305 310 315 320
   His Thr Val Ser Tyr Gly Asp Asp Glu Asp Ser Leu Ser Ser Ala Tyr
   325 330 335
   Ile Gln Arg Val Asn Thr Glu Leu Met Lys Ala Ala Ala Arg Gly Leu
   340 345 350
   Thr Leu Leu Phe Ala Ser Gly Asp Ser Gly Ala Gly Cys Trp Ser Val
   355 360 365
   Ser Gly Arg His Gln Phe Arg Pro Thr Phe Pro Ala Ser Ser Pro Tyr
   370 375 380
   Val Thr Thr Val Gly Gly Thr Ser Phe Gln Glu Pro Phe Leu Ile Thr
   385 390 395 400
   Asn Glu Ile Val Asp Tyr Ile Ser Gly Gly Gly Phe Ser Asn Val Phe
   405 410 415
   Pro Arg Pro Ser Tyr Gln Glu Glu Ala Val Thr Lys Phe Leu Ser Ser
   420 425 430
   Ser Pro His Leu Pro Pro Ser Ser Tyr Phe Asn Ala Ser Gly Arg Ala
   435 440 445
   Tyr Pro Asp Val Ala Ala Leu Ser Asp Gly Tyr Trp Val Val Ser Asn
   450 455 460
   Arg Val Pro Ile Pro Trp Val Ser Gly Thr Ser Ala Ser Thr Pro Val
   465 470 475 480
   Phe Gly Gly Ile Leu Ser Leu Ile Asn Glu His Arg Ile Leu Ser Gly
   485 490 495
   Arg Pro Pro Leu Gly Phe Leu Asn Pro Arg Leu Tyr Gln Gln His Gly
   500 505 510
   Ala Gly Leu Phe Asp Val Thr Arg Gly Cys His Glu Ser Cys Leu Asp
   515 520 525
   Glu Glu Val Glu Gly Gln Gly Phe Cys Ser Gly Pro Gly Trp Asp Pro
   530 535 540
   Val Thr Gly Trp Gly Thr Pro Asn Phe Pro Ala Leu Leu Lys Thr Leu
   545 550 555 560
   Leu Asn Pro Arg Ser Pro Gly Lys Arg Val Ser Arg Asn Lys Ser Glu
   565 570 575
   Lys Lys Arg Arg
   580
<210> 35
   <211> 2160
   <212> DNA
   <213> Homo sapiens
<400> 35
   atgggactcc aagcctgcct cctagggctc tttgccctca tcctctctgg caaatgcagt 60
   tacagcccgg agcccgacca gcggaggacg ctgcccccag gctgggtgtc cctgggccgt 120
   gcggaccctg aggaagagct gagtctcacc tttgccctga gacagcagaa tgtggaaaga 180
   ctctcggagc tggtgcaggc tgtgtcggat cccagctctc ctcaatacgg aaaatacctg 240
   accctagaga atgtggctga tctggtgagg ccatccccac tgaccctcca cacggtgcaa 300
   aaatggctct tggcagccgg agcccagaag tgccattctg tgatcacaca ggactttctg 360
   acttgctggc tgagcatccg acaagcagag ctgctgctcc ctggggctga gtttcatcac 420
   tatgtgggag gacctacgga aacccatgtt gtaaggtccc cacatcccta ccagcttcca 480
   caggccttgg ccccccatgt ggactttgtg gggggactgc accgttttcc cccaacatca 540
   tccctgaggc aacgtcctga gccgcaggtg acagggactg taggcctgca tctgggggta 600
   accccctctg tgatccgtaa gcgatacaac ttgacctcac aagacgtggg ctctggcacc 660
   agcaataaca gccaagcctg tgcccagttc ctggagcagt atttccatga ctcagacctg 720
   gctcagttca tgcgcctctt cggtggcaac tttgcacatc aggcatcagt agcccgtgtg 780
   gttggacaac agggccgggg ccgggccggg attgaggcca gtctagatgt gcagtacctg 840
   atgagtgctg gtgccaacat ctccacctgg gtctacagta gccctggccg gcatgaggga 900
   caggagccct tcctgcagtg gctcatgctg ctcagtaatg agtcagccct gccacatgtg 960
   catactgtga gctatggaga tgatgaggac tccctcagca gcgcctacat ccagcgggtc 1020
   aacactgagc tcatgaaggc tgccgctcgg ggtctcaccc tgctcttcgc ctcaggtgac 1080
   agtggggccg ggtgttggtc tgtctctgga agacaccagt tccgccctac cttccctgcc 1140
   tccagcccct atgtcaccac agtgggaggc acatccttcc aggaaccttt cctcatcaca 1200
   aatgaaattg ttgactatat cagtggtggt ggcttcagca atgtgttccc acggccttca 1260
   taccaggagg aagctgtaac gaagttcctg agctctagcc cccacctgcc accatccagt 1320
   tacttcaatg ccagtggccg tgcctaccca gatgtggctg cactttctga tggctactgg 1380
   gtggtcagca acagagtgcc cattccatgg gtgtccggaa cctcggcctc tactccagtg 1440
   tttgggggga tcctatcctt gatcaatgag cacaggatcc ttagtggccg cccccctctt 1500
   ggctttctca acccaaggct ctaccagcag catggggcag gactctttga tgtaacccgt 1560
   ggctgccatg agtcctgtct ggatgaagag gtagagggcc agggtttctg ctctggtcct 1620
   ggctgggatc ctgtaacagg ctggggaaca cccaacttcc cagctttgct gaagactcta 1680
   ctcaacccca gatccgtcga catcgaaggt agaggcattc ccgccttgcc cgaggatggc 1740
   ggcagcggcg ccttcccgcc cggccacttc aaggacccca agcggctgta ctgcaaaaac 1800
   gggggcttct tcctgcgcat ccaccccgac ggccgagttg acggggtccg ggagaagagc 1860
   gaccctcaca tcaagctaca acttcaagca gaagagagag gagttgtgtc tatcaaagga 1920
   gtgtctgcta accgttacct ggctatgaag gaagatggaa gattactggc ttctaaatct     1980
   gttacggatg agtgtttctt ttttgaacga ttggaatcta ataactacaa tacttaccgg     2040
   tcaaggaaat acaccagttg gtatgtggca ctgaaacgaa ctgggcagta taaacttggc     2100
   tccaaaacag gacctgggca gaaagctata ctttttcttc caatgtctgc taagagctga     2160
<210> 36
   <211> 719
   <212> PRT
   <213> Homo sapiens
<400> 36
   Met Gly Leu Gln Ala Cys Leu Leu Gly Leu Phe Ala Leu Ile Leu Ser
   1               5                   10                  15
   Gly Lys Cys Ser Tyr Ser Pro Glu Pro Asp Gln Arg Arg Thr Leu Pro
               20                  25                  30
   Pro Gly Trp Val Ser Leu Gly Arg Ala Asp Pro Glu Glu Glu Leu Ser
           35                  40                  45
   Leu Thr Phe Ala Leu Arg Gln Gln Asn Val Glu Arg Leu Ser Glu Leu
       50                  55                  60
   Val Gln Ala Val Ser Asp Pro Ser Ser Pro Gln Tyr Gly Lys Tyr Leu
   65                  70                  75                  80
   Thr Leu Glu Asn Val Ala Asp Leu Val Arg Pro Ser Pro Leu Thr Leu
                   85                  90                  95
   His Thr Val Gln Lys Trp Leu Leu Ala Ala Gly Ala Gln Lys Cys His
               100                 105                 110
   Ser Val Ile Thr Gln Asp Phe Leu Thr Cys Trp Leu Ser Ile Arg Gln
           115                 120                 125
   Ala Glu Leu Leu Leu Pro Gly Ala Glu Phe His His Tyr Val Gly Gly
       130                 135                 140
   Pro Thr Glu Thr His Val Val Arg Ser Pro His Pro Tyr Gln Leu Pro
   145                 150                 155                 160
   Gln Ala Leu Ala Pro His Val Asp Phe Val Gly Gly Leu His Arg Phe
                   165                 170                 175
   Pro Pro Thr Ser Ser Leu Arg Gln Arg Pro Glu Pro Gln Val Thr Gly
               180                 185                 190
   Thr Val Gly Leu His Leu Gly Val Thr Pro Ser Val Ile Arg Lys Arg
           195                 200                 205
   Tyr Asn Leu Thr Ser Gln Asp Val Gly Ser Gly Thr Ser Asn Asn Ser
       210                 215                 220
   Gln Ala Cys Ala Gln Phe Leu Glu Gln Tyr Phe His Asp Ser Asp Leu
   225                 230                 235                     240
   Ala Gln Phe Met Arg Leu Phe Gly Gly Asn Phe Ala His Gln Ala Ser
                   245                 250                 255
   Val Ala Arg Val Val Gly Gln Gln Gly Arg Gly Arg Ala Gly Ile Glu
               260                 265                 270
   Ala Ser Leu Asp Val Gln Tyr Leu Met Ser Ala Gly Ala Asn Ile Ser
           275                 280                 285
   Thr Trp Val Tyr Ser Ser Pro Gly Arg His Glu Gly Gln Glu Pro Phe
       290                 295                 300
   Leu Gln Trp Leu Met Leu Leu Ser Asn Glu Ser Ala Leu Pro His Val
   305                 310                 315                     320
   His Thr Val Ser Tyr Gly Asp Asp Glu Asp Ser Leu Ser Ser Ala Tyr
                   325                 330                 335
   Ile Gln Arg Val Asn Thr Glu Leu Met Lys Ala Ala Ala Arg Gly Leu
               340                 345                 350
   Thr Leu Leu Phe Ala Ser Gly Asp Ser Gly Ala Gly Cys Trp Ser Val
           355                 360                 365
   Ser Gly Arg His Gln Phe Arg Pro Thr Phe Pro Ala Ser Ser Pro Tyr
       370                 375                 380
   Val Thr Thr Val Gly Gly Thr Ser Phe Gln Glu Pro Phe Leu Ile Thr
   385                 390                 395                     400
   Asn Glu Ile Val Asp Tyr Ile Ser Gly Gly Gly Phe Ser Asn Val Phe
                   405                 410                 415
   Pro Arg Pro Ser Tyr Gln Glu Glu Ala Val Thr Lys Phe Leu Ser Ser
               420                 425                 430
   Ser Pro His Leu Pro Pro Ser Ser Tyr Phe Asn Ala Ser Gly Arg Ala
           435                 440                 445
   Tyr Pro Asp Val Ala Ala Leu Ser Asp Gly Tyr Trp Val Val Ser Asn
       450                 455                 460
   Arg Val Pro Ile Pro Trp Val Ser Gly Thr Ser Ala Ser Thr Pro Val
   465                 470                 475                 480
   Phe Gly Gly Ile Leu Ser Leu Ile Asn Glu His Arg Ile Leu Ser Gly
                   485                 490                 495
   Arg Pro Pro Leu Gly Phe Leu Asn Pro Arg Leu Tyr Gln Gln His Gly
               500                 505                 510
   Ala Gly Leu Phe Asp Val Thr Arg Gly Cys His Glu Ser Cys Leu Asp
           515                 520                 525
   Glu Glu Val Glu Gly Gln Gly Phe Cys Ser Gly Pro Gly Trp Asp Pro
       530                 535                 540
   Val Thr Gly Trp Gly Thr Pro Asn Phe Pro Ala Leu Leu Lys Thr Leu
   545                 550                 555                 560
   Leu Asn Pro Arg Ser Val Asp Ile Glu Gly Arg Gly Ile Pro Ala Leu
                   565                 570                 575
   Pro Glu Asp Gly Gly Ser Gly Ala Phe Pro Pro Gly His Phe Lys Asp
               580                 585                 590
   Pro Lys Arg Leu Tyr Cys Lys Asn Gly Gly Phe Phe Leu Arg Ile His
           595                 600                 605
   Pro Asp Gly Arg Val Asp Gly Val Arg Glu Lys Ser Asp Pro His Ile
       610                 615                 620
   Lys Leu Gln Leu Gln Ala Glu Glu Arg Gly Val Val Ser Ile Lys Gly
   625                 630                 635                 640
   Val Ser Ala Asn Arg Tyr Leu Ala Met Lys Glu Asp Gly Arg Leu Leu
                   645                 650                 655
   Ala Ser Lys Ser Val Thr Asp Glu Cys Phe Phe Phe Glu Arg Leu Glu
               660                 665                 670
   Ser Asn Asn Tyr Asn Thr Tyr Arg Ser Arg Lys Tyr Thr Ser Trp Tyr
           675                 680                 685
   Val Ala Leu Lys Arg Thr Gly Gln Tyr Lys Leu Gly Ser Lys Thr Gly
       690                 695                 700
   Pro Gly Gln Lys Ala Ile Leu Phe Leu Pro Met Ser Ala Lys Ser
   705                 710                 715
<210> 37
   <211> 2160
   <212> DNA
   <213> Homo sapiens
<400> 37
   atgggactcc aagcctgcct cctagggctc tttgccctca tcctctctgg caaatgcagt      60
   tacagcccgg agcccgacca gcggaggacg ctgcccccag gctgggtgtc cctgggccgt     120
   gcggaccctg aggaagagct gagtctcacc tttgccctga gacagcagaa tgtggaaaga     180
   ctctcggagc tggtgcaggc tgtgtcggat cccagctctc ctcaatacgg aaaatacctg     240
   accctagaga atgtggctga tctggtgagg ccatccccac tgaccctcca cacggtgcaa     300
   aaatggctct tggcagccgg agcccagaag tgccattctg tgatcacaca ggactttctg     360
   acttgctggc tgagcatccg acaagcagag ctgctgctcc ctggggctga gtttcatcac     420
   tatgtgggag gacctacgga aacccatgtt gtaaggtccc cacatcccta ccagcttcca     480
   caggccttgg ccccccatgt ggactttgtg gggggactgc accgttttcc cccaacatca     540
   tccctgaggc aacgtcctga gccgcaggtg acagggactg taggcctgca tctgggggta     600
   accccctctg tgatccgtaa gcgatacaac ttgacctcac aagacgtggg ctctggcacc     660
   agcaataaca gccaagcctg tgcccagttc ctggagcagt atttccatga ctcagacctg     720
   gctcagttca tgcgcctctt cggtggcaac tttgcacatc aggcatcagt agcccgtgtg     780
   gttggacaac agggccgggg ccgggccggg attgaggcca gtctagatgt gcagtacctg     840
   atgagtgctg gtgccaacat ctccacctgg gtctacagta gccctggccg gcatgaggga     900
   caggagccct tcctgcagtg gctcatgctg ctcagtaatg agtcagccct gccacatgtg     960
   catactgtga gctatggaga tgatgaggac tccctcagca gcgcctacat ccagcgggtc    1020
   aacactgagc tcatgaaggc tgccgctcgg ggtctcaccc tgctcttcgc ctcaggtgac    1080
   agtggggccg ggtgttggtc tgtctctgga agacaccagt tccgccctac cttccctgcc    1140
   tccagcccct atgtcaccac agtgggaggc acatccttcc aggaaccttt cctcatcaca    1200
   aatgaaattg ttgactatat cagtggtggt ggcttcagca atgtgttccc acggccttca    1260
   taccaggagg aagctgtaac gaagttcctg agctctagcc cccacctgcc accatccagt    1320
   tacttcaatg ccagtggccg tgcctaccca gatgtggctg cactttctga tggctactgg    1380
   gtggtcagca acagagtgcc cattccatgg gtgtccggaa cctcggcctc tactccagtg    1440
   tttgggggga tcctatcctt gatcaatgag cacaggatcc ttagtggccg cccccctctt    1500
   ggctttctca acccaaggct ctaccagcag catggggcag gactctttga tgtaacccgt    1560
   ggctgccatg agtcctgtct ggatgaagag gtagagggcc agggtttctg ctctggtcct    1620
   ggctgggatc ctgtaacagg ctggggaaca cccaacttcc cagctttgct gaagactcta    1680
   ctcaacccca gatccgtcga catcgaaggt agaggcattc ccgccttgcc cgaggatggc    1740
   ggcagcggcg ccttcccgcc cggccacttc aaggacccca agcggctgta ctgcaaaaac 1800
   gggggcttct tcctgcgcat ccaccccgac ggccgagttg acggggtccg ggagaagagc 1860
   gaccctcaca tcaagctaca acttcaagca gaagagagag gagttgtgtc tatcaaagga 1920
   gtgtctgcta accgttacct ggctatgaag gaagatggaa gattactggc ttctaaatct 1980
   gttacggatg agtgtttctt ttttgcacga ttggaatcta ataactacaa tacttaccgg 2040
   tcaaggaaat acaccagttg gtatgtggca ctgaaacgaa ctgggcagta taaacttggc 2100
   tccaaaacag gacctgggca gaaagctata ctttttcttc caatgtctgc taagagctga 2160
<210> 38
   <211> 719
   <212> PRT
   <213> Homo sapiens
<400> 38
   Met Gly Leu Gln Ala Cys Leu Leu Gly Leu Phe Ala Leu Ile Leu Ser
   1               5                   10                  15
   Gly Lys Cys Ser Tyr Ser Pro Glu Pro Asp Gln Arg Arg Thr Leu Pro
               20                  25                  30
   Pro Gly Trp Val Ser Leu Gly Arg Ala Asp Pro Glu Glu Glu Leu Ser
           35                  40                  45
   Leu Thr Phe Ala Leu Arg Gln Gln Asn Val Glu Arg Leu Ser Glu Leu
       50                  55                  60
   Val Gln Ala Val Ser Asp Pro Ser Ser Pro Gln Tyr Gly Lys Tyr Leu
   65                  70                  75                  80
   Thr Leu Glu Asn Val Ala Asp Leu Val Arg Pro Ser Pro Leu Thr Leu
                   85                  90                  95
   His Thr Val Gln Lys Trp Leu Leu Ala Ala Gly Ala Gln Lys Cys His
               100                 105                 110
   Ser Val Ile Thr Gln Asp Phe Leu Thr Cys Trp Leu Ser Ile Arg Gln
           115                 120                 125
   Ala Glu Leu Leu Leu Pro Gly Ala Glu Phe His His Tyr Val Gly Gly
       130                 135                 140
   Pro Thr Glu Thr His Val Val Arg Ser Pro His Pro Tyr Gln Leu Pro
   145                 150                 155                 160
   Gln Ala Leu Ala Pro His Val Asp Phe Val Gly Gly Leu His Arg Phe
                   165                 170                 175
   Pro Pro Thr Ser Ser Leu Arg Gln Arg Pro Glu Pro Gln Val Thr Gly
               180                 185                 190
   Thr Val Gly Leu His Leu Gly Val Thr Pro Ser Val Ile Arg Lys Arg
           195                 200                 205
   Tyr Asn Leu Thr Ser Gln Asp Val Gly Ser Gly Thr Ser Asn Asn Ser
       210                 215                 220
   Gln Ala Cys Ala Gln Phe Leu Glu Gln Tyr Phe His Asp Ser Asp Leu
   225                 230                 235                 240
   Ala Gln Phe Met Arg Leu Phe Gly Gly Asn Phe Ala His Gln Ala Ser
                   245                 250                 255
   Val Ala Arg Val Val Gly Gln Gln Gly Arg Gly Arg Ala Gly Ile Glu
               260                 265                 270
   Ala Ser Leu Asp Val Gln Tyr Leu Met Ser Ala Gly Ala Asn Ile Ser
           275                 280                 285
   Thr Trp Val Tyr Ser Ser Pro Gly Arg His Glu Gly Gln Glu Pro Phe
       290                 295                 300
   Leu Gln Trp Leu Met Leu Leu Ser Asn Glu Ser Ala Leu Pro His Val
   305                 310                 315                 320
   His Thr Val Ser Tyr Gly Asp Asp Glu Asp Ser Leu Ser Ser Ala Tyr
                   325                 330                 335
   Ile Gln Arg Val Asn Thr Glu Leu Met Lys Ala Ala Ala Arg Gly Leu
               340                 345                 350
   Thr Leu Leu Phe Ala Ser Gly Asp Ser Gly Ala Gly Cys Trp Ser Val
           355                 360                 365
   Ser Gly Arg His Gln Phe Arg Pro Thr Phe Pro Ala Ser Ser Pro Tyr
       370                 375                 380
   Val Thr Thr Val Gly Gly Thr Ser Phe Gln Glu Pro Phe Leu Ile Thr
   385                 390                 395                 400
   Asn Glu Ile Val Asp Tyr Ile Ser Gly Gly Gly Phe Ser Asn Val Phe
                   405                 410                 415
   Pro Arg Pro Ser Tyr Gln Glu Glu Ala Val Thr Lys Phe Leu Ser Ser
               420                 425                 430
   Ser Pro His Leu Pro Pro Ser Ser Tyr Phe Asn Ala Ser Gly Arg Ala
   435 440 445
   Tyr Pro Asp Val Ala Ala Leu Ser Asp Gly Tyr Trp Val Val Ser Asn
   450 455 460
   Arg Val Pro Ile Pro Trp Val Ser Gly Thr Ser Ala Ser Thr Pro Val
   465 470 475 480
   Phe Gly Gly Ile Leu Ser Leu Ile Asn Glu His Arg Ile Leu Ser Gly
   485 490 495
   Arg Pro Pro Leu Gly Phe Leu Asn Pro Arg Leu Tyr Gln Gln His Gly
   500 505 510
   Ala Gly Leu Phe Asp Val Thr Arg Gly Cys His Glu Ser Cys Leu Asp
   515 520 525
   Glu Glu Val Glu Gly Gln Gly Phe Cys Ser Gly Pro Gly Trp Asp Pro
   530 535 540
   Val Thr Gly Trp Gly Thr Pro Asn Phe Pro Ala Leu Leu Lys Thr Leu
   545 550 555 560
   Leu Asn Pro Arg Ser Val Asp Ile Glu Gly Arg Gly Ile Pro Ala Leu
   565 570 575
   Pro Glu Asp Gly Gly Ser Gly Ala Phe Pro Pro Gly His Phe Lys Asp
   580 585 590
   Pro Lys Arg Leu Tyr Cys Lys Asn Gly Gly Phe Phe Leu Arg Ile His
   595 600 605
   Pro Asp Gly Arg Val Asp Gly Val Arg Glu Lys Ser Asp Pro His Ile
   610 615 620
   Lys Leu Gln Leu Gln Ala Glu Glu Arg Gly Val Val Ser Ile Lys Gly
   625 630 635 640
   Val Ser Ala Asn Arg Tyr Leu Ala Met Lys Glu Asp Gly Arg Leu Leu
   645 650 655
   Ala Ser Lys Ser Val Thr Asp Glu Cys Phe Phe Phe Ala Arg Leu Glu
   660 665 670
   Ser Asn Asn Tyr Asn Thr Tyr Arg Ser Arg Lys Tyr Thr Ser Trp Tyr
   675 680 685
   Val Ala Leu Lys Arg Thr Gly Gln Tyr Lys Leu Gly Ser Lys Thr Gly
   690 695 700
   Pro Gly Gln Lys Ala Ile Leu Phe Leu Pro Met Ser Ala Lys Ser
   705 710 715
<210> 39
   <211> 2160
   <212> DNA
   <213> Homo sapiens
<400> 39
   atgggactcc aagcctgcct cctagggctc tttgccctca tcctctctgg caaatgcagt 60
   tacagcccgg agcccgacca gcggaggacg ctgcccccag gctgggtgtc cctgggccgt 120
   gcggaccctg aggaagagct gagtctcacc tttgccctga gacagcagaa tgtggaaaga 180
   ctctcggagc tggtgcaggc tgtgtcggat cccagctctc ctcaatacgg aaaatacctg 240
   accctagaga atgtggctga tctggtgagg ccatccccac tgaccctcca cacggtgcaa 300
   aaatggctct tggcagccgg agcccagaag tgccattctg tgatcacaca ggactttctg 360
   acttgctggc tgagcatccg acaagcagag ctgctgctcc ctggggctga gtttcatcac 420
   tatgtgggag gacctacgga aacccatgtt gtaaggtccc cacatcccta ccagcttcca 480
   caggccttgg ccccccatgt ggactttgtg gggggactgc accgttttcc cccaacatca 540
   tccctgaggc aacgtcctga gccgcaggtg acagggactg taggcctgca tctgggggta 600
   accccctctg tgatccgtaa gcgatacaac ttgacctcac aagacgtggg ctctggcacc 660
   agcaataaca gccaagcctg tgcccagttc ctggagcagt atttccatga ctcagacctg 720
   gctcagttca tgcgcctctt cggtggcaac tttgcacatc aggcatcagt agcccgtgtg 780
   gttggacaac agggccgggg ccgggccggg attgaggcca gtctagatgt gcagtacctg 840
   atgagtgctg gtgccaacat ctccacctgg gtctacagta gccctggccg gcatgaggga 900
   caggagccct tcctgcagtg gctcatgctg ctcagtaatg agtcagccct gccacatgtg 960
   catactgtga gctatggaga tgatgaggac tccctcagca gcgcctacat ccagcgggtc 1020
   aacactgagc tcatgaaggc tgccgctcgg ggtctcaccc tgctcttcgc ctcaggtgac 1080
   agtggggccg ggtgttggtc tgtctctgga agacaccagt tccgccctac cttccctgcc 1140
   tccagcccct atgtcaccac agtgggaggc acatccttcc aggaaccttt cctcatcaca 1200
   aatgaaattg ttgactatat cagtggtggt ggcttcagca atgtgttccc acggccttca 1260
   taccaggagg aagctgtaac gaagttcctg agctctagcc cccacctgcc accatccagt 1320
   tacttcaatg ccagtggccg tgcctaccca gatgtggctg cactttctga tggctactgg 1380
   gtggtcagca acagagtgcc cattccatgg gtgtccggaa cctcggcctc tactccagtg 1440
   tttgggggga tcctatcctt gatcaatgag cacaggatcc ttagtggccg cccccctctt 1500
   ggctttctca acccaaggct ctaccagcag catggggcag gactctttga tgtaacccgt 1560
   ggctgccatg agtcctgtct ggatgaagag gtagagggcc agggtttctg ctctggtcct 1620
   ggctgggatc ctgtaacagg ctggggaaca cccaacttcc cagctttgct gaagactcta 1680
   ctcaacccca gatccgtcga catcgaaggt agaggcattc ccgccttgcc cgaggatggc 1740
   ggcagcggcg ccttcccgcc cggccacttc aaggacccca agcggctgta ctgcaaaaac 1800
   gggggcttct tcctgcgcat ccaccccgac ggccgagttg acgggacaag ggacaggagc 1860
   gaccagcaca ttcagctgca gctcagtgca gaagagagag gagttgtgtc tatcaaagga 1920
   gtgtctgcta accgttacct ggctatgaag gaagatggaa gattactggc ttctaaatct 1980
   gttacggatg agtgtttctt ttttgaacga ttggaatcta ataactacaa tacttaccgg 2040
   tcaaggaaat acaccagttg gtatgtggca ctgaaacgaa ctgggcagta taaacttggc 2100
   tccaaaacag gacctgggca gaaagctata ctttttcttc caatgtctgc taagagctga 2160
<210> 40
   <211> 719
   <212> PRT
   <213> Homo sapiens
<400> 40
   Met Gly Leu Gln Ala Cys Leu Leu Gly Leu Phe Ala Leu Ile Leu Ser
   1               5                   10                  15
   Gly Lys Cys Ser Tyr Ser Pro Glu Pro Asp Gln Arg Arg Thr Leu Pro
               20                  25                  30
   Pro Gly Trp Val Ser Leu Gly Arg Ala Asp Pro Glu Glu Glu Leu Ser
           35                  40                  45
   Leu Thr Phe Ala Leu Arg Gln Gln Asn Val Glu Arg Leu Ser Glu Leu
       50                  55                  60
   Val Gln Ala Val Ser Asp Pro Ser Ser Pro Gln Tyr Gly Lys Tyr Leu
   65                  70                  75                  80
   Thr Leu Glu Asn Val Ala Asp Leu Val Arg Pro Ser Pro Leu Thr Leu
                   85                  90                  95
   His Thr Val Gln Lys Trp Leu Leu Ala Ala Gly Ala Gln Lys Cys His
               100                 105                 110
   Ser Val Ile Thr Gln Asp Phe Leu Thr Cys Trp Leu Ser Ile Arg Gln
           115                 120                 125
   Ala Glu Leu Leu Leu Pro Gly Ala Glu Phe His His Tyr Val Gly Gly
       130                 135                 140
   Pro Thr Glu Thr His Val Val Arg Ser Pro His Pro Tyr Gln Leu Pro
   145 150 155 160
   Gln Ala Leu Ala Pro His Val Asp Phe Val Gly Gly Leu His Arg Phe
   165 170 175
   Pro Pro Thr Ser Ser Leu Arg Gln Arg Pro Glu Pro Gln Val Thr Gly
   180 185 190
   Thr Val Gly Leu His Leu Gly Val Thr Pro Ser Val Ile Arg Lys Arg
   195 200 205
   Tyr Asn Leu Thr Ser Gln Asp Val Gly Ser Gly Thr Ser Asn Asn Ser
   210 215 220
   Gln Ala Cys Ala Gln Phe Leu Glu Gln Tyr Phe His Asp Ser Asp Leu
   225 230 235 240
   Ala Gln Phe Met Arg Leu Phe Gly Gly Asn Phe Ala His Gln Ala Ser
   245 250 255
   Val Ala Arg Val Val Gly Gln Gln Gly Arg Gly Arg Ala Gly Ile Glu
   260 265 270
   Ala Ser Leu Asp Val Gln Tyr Leu Met Ser Ala Gly Ala Asn Ile Ser
   275 280 285
   Thr Trp Val Tyr Ser Ser Pro Gly Arg His Glu Gly Gln Glu Pro Phe
   290 295 300
   Leu Gln Trp Leu Met Leu Leu Ser Asn Glu Ser Ala Leu Pro His Val
   305 310 315 320
   His Thr Val Ser Tyr Gly Asp Asp Glu Asp Ser Leu Ser Ser Ala Tyr
   325 330 335
   Ile Gln Arg Val Asn Thr Glu Leu Met Lys Ala Ala Ala Arg Gly Leu
   340 345 350
   Thr Leu Leu Phe Ala Ser Gly Asp Ser Gly Ala Gly Cys Trp Ser Val
   355 360 365
   Ser Gly Arg His Gln Phe Arg Pro Thr Phe Pro Ala Ser Ser Pro Tyr
   370 375 380
   Val Thr Thr Val Gly Gly Thr Ser Phe Gln Glu Pro Phe Leu Ile Thr
   385 390 395 400
   Asn Glu Ile Val Asp Tyr Ile Ser Gly Gly Gly Phe Ser Asn Val Phe
   405 410 415
   Pro Arg Pro Ser Tyr Gln Glu Glu Ala Val Thr Lys Phe Leu Ser Ser
   420 425 430
   Ser Pro His Leu Pro Pro Ser Ser Tyr Phe Asn Ala Ser Gly Arg Ala
   435 440 445
   Tyr Pro Asp Val Ala Ala Leu Ser Asp Gly Tyr Trp Val Val Ser Asn
   450 455 460
   Arg Val Pro Ile Pro Trp Val Ser Gly Thr Ser Ala Ser Thr Pro Val
   465 470 475 480
   Phe Gly Gly Ile Leu Ser Leu Ile Asn Glu His Arg Ile Leu Ser Gly
   485 490 495
   Arg Pro Pro Leu Gly Phe Leu Asn Pro Arg Leu Tyr Gln Gln His Gly
   500 505 510
   Ala Gly Leu Phe Asp Val Thr Arg Gly Cys His Glu Ser Cys Leu Asp
   515 520 525
   Glu Glu Val Glu Gly Gln Gly Phe Cys Ser Gly Pro Gly Trp Asp Pro
   530 535 540
   Val Thr Gly Trp Gly Thr Pro Asn Phe Pro Ala Leu Leu Lys Thr Leu
   545 550 555 560
   Leu Asn Pro Arg Ser Val Asp Ile Glu Gly Arg Gly Ile Pro Ala Leu
   565 570 575
   Pro Glu Asp Gly Gly Ser Gly Ala Phe Pro Pro Gly His Phe Lys Asp
   580 585 590
   Pro Lys Arg Leu Tyr Cys Lys Asn Gly Gly Phe Phe Leu Arg Ile His
   595 600 605
   Pro Asp Gly Arg Val Asp Gly Thr Arg Asp Arg Ser Asp Gln His Ile
   610 615 620
   Gln Leu Gln Leu Ser Ala Glu Glu Arg Gly Val Val Ser Ile Lys Gly
   625 630 635 640
   Val Ser Ala Asn Arg Tyr Leu Ala Met Lys Glu Asp Gly Arg Leu Leu
   645 650 655
   Ala Ser Lys Ser Val Thr Asp Glu Cys Phe Phe Phe Glu Arg Leu Glu
   660 665 670
   Ser Asn Asn Tyr Asn Thr Tyr Arg Ser Arg Lys Tyr Thr Ser Trp Tyr
           675                     680                     685
   Val Ala Leu Lys Arg Thr Gly Gln Tyr Lys Leu Gly Ser Lys Thr Gly
       690                     695                     700
   Pro Gly Gln Lys Ala Ile Leu Phe Leu Pro Met Ser Ala Lys Ser
   705                     710                     715
<210> 41
   <211> 2160
   <212> DNA
   <213> Homo sapiens
<400> 41
   atgggactcc aagcctgcct cctagggctc tttgccctca tcctctctgg caaatgcagt 60
   tacagcccgg agcccgacca gcggaggacg ctgcccccag gctgggtgtc cctgggccgt 120
   gcggaccctg aggaagagct gagtctcacc tttgccctga gacagcagaa tgtggaaaga 180
   ctctcggagc tggtgcaggc tgtgtcggat cccagctctc ctcaatacgg aaaatacctg 240
   accctagaga atgtggctga tctggtgagg ccatccccac tgaccctcca cacggtgcaa 300
   aaatggctct tggcagccgg agcccagaag tgccattctg tgatcacaca ggactttctg 360
   acttgctggc tgagcatccg acaagcagag ctgctgctcc ctggggctga gtttcatcac 420
   tatgtgggag gacctacgga aacccatgtt gtaaggtccc cacatcccta ccagcttcca 480
   caggccttgg ccccccatgt ggactttgtg gggggactgc accgttttcc cccaacatca 540
   tccctgaggc aacgtcctga gccgcaggtg acagggactg taggcctgca tctgggggta 600
   accccctctg tgatccgtaa gcgatacaac ttgacctcac aagacgtggg ctctggcacc 660
   agcaataaca gccaagcctg tgcccagttc ctggagcagt atttccatga ctcagacctg 720
   gctcagttca tgcgcctctt cggtggcaac tttgcacatc aggcatcagt agcccgtgtg 780
   gttggacaac agggccgggg ccgggccggg attgaggcca gtctagatgt gcagtacctg 840
   atgagtgctg gtgccaacat ctccacctgg gtctacagta gccctggccg gcatgaggga 900
   caggagccct tcctgcagtg gctcatgctg ctcagtaatg agtcagccct gccacatgtg 960
   catactgtga gctatggaga tgatgaggac tccctcagca gcgcctacat ccagcgggtc 1020
   aacactgagc tcatgaaggc tgccgctcgg ggtctcaccc tgctcttcgc ctcaggtgac 1080
   agtggggccg ggtgttggtc tgtctctgga agacaccagt tccgccctac cttccctgcc 1140
   tccagcccct atgtcaccac agtgggaggc acatccttcc aggaaccttt cctcatcaca 1200
   aatgaaattg ttgactatat cagtggtggt ggcttcagca atgtgttccc acggccttca 1260
   taccaggagg aagctgtaac gaagttcctg agctctagcc cccacctgcc accatccagt 1320
   tacttcaatg ccagtggccg tgcctaccca gatgtggctg cactttctga tggctactgg 1380
   gtggtcagca acagagtgcc cattccatgg gtgtccggaa cctcggcctc tactccagtg 1440
   tttgggggga tcctatcctt gatcaatgag cacaggatcc ttagtggccg cccccctctt 1500
   ggctttctca acccaaggct ctaccagcag catggggcag gactctttga tgtaacccgt 1560
   ggctgccatg agtcctgtct ggatgaagag gtagagggcc agggtttctg ctctggtcct 1620
   ggctgggatc ctgtaacagg ctggggaaca cccaacttcc cagctttgct gaagactcta 1680
   ctcaacccca gatccgtcga catcgaaggt agaggcattc ccgccttgcc cgaggatggc 1740
   ggcagcggcg ccttcccgcc cggccacttc aaggacccca agcggctgta ctgcaaaaac 1800
   gggggcttct tcctgcgcat ccaccccgac ggccgagttg acgggacaag ggacaggagc 1860
   gaccagcaca ttcagctgca gctcagtgca gaagagagag gagttgtgtc tatcaaagga 1920
   gtgtctgcta accgttacct ggctatgaag gaagatggaa gattactggc ttctaaatct 1980
   gttacggatg agtgtttctt ttttgcacga ttggaatcta ataactacaa tacttaccgg 2040
   tcaaggaaat acaccagttg gtatgtggca ctgaaacgaa ctgggcagta taaacttggc 2100
   tccaaaacag gacctgggca gaaagctata ctttttcttc caatgtctgc taagagctga 2160
<210> 42
   <211> 719
   <212> PRT
   <213> Homo sapiens
<400> 42
<210> 43
   <211> 276
   <212> DNA
   <213> Homo sapiens
<400> 43
   atgcagccct ccagccttct gccgctcgcc ctctgcctgc tggctgcacc cgccggatct 60
   tccaagccac aagcactggc cacaccaaac aaggaggagc acgggaaaag aaagaagaaa 120
   ggcaaggggc tagggaagaa gagggaccca tgtcttcgga aatacaagga cttctgcatc 180
   catggagaat gcaaatatgt gaaggagctc cgggctccct cctgcatctg ccacccgggt 240
   taccatggag agaggtgtca tgggctgagc ggatct 276
<210> 44
   <211> 141
   <212> DNA
   <213> Homo sapiens
<400> 44
   atgcagccct ccagccttct gccgctcgcc ctctgcctgc tggctgcacc cgccggatct 60
   gggaaaagaa agaagaaagg caaggggcta gggaagaaga gggacccatc tcttcggaaa 120
   tacaaggact tctccggatc t 141
<210> 45
   <211> 1725
   <212> DNA
   <213> Homo sapiens
<400> 45
   atgcagccct ccagccttct gccgctcgcc ctctgcctgc tggctgcacc cgccggatct      60
   tccaagccac aagcactggc cacaccaaac aaggaggagc acgggaaaag aaagaagaaa     120
   ggcaaggggc tagggaagaa gagggaccca tgtcttcgga aatacaagga cttctgcatc     180
   catggagaat gcaaatatgt gaaggagctc cgggctccct cctgcatctg ccacccgggt     240
   taccatggag agaggtgtca tgggctgagc ggatctcgtc cccggaacgc actgctgctc     300
   ctcgcggatg acggaggctt tgagagtggc gcgtacaaca acagcgccat cgccaccccg     360
   cacctggacg ccttggcccg ccgcagcctc ctctttcgca atgccttcac ctcggtcagc     420
   agctgctctc ccagccgcgc cagcctcctc actggcctgc cccagcatca gaatgggatg     480
   tacgggctgc accaggacgt gcaccacttc aactccttcg acaaggtgcg gagcctgccg     540
   ctgctgctca gccaagctgg tgtgcgcaca ggcatcatcg ggaagaagca cgtggggccg     600
   gagaccgtgt acccgtttga ctttgcgtac acggaggaga atggctccgt cctccaggtg     660
   gggcggaaca tcactagaat taagctgctc gtccggaaat tcctgcagac tcaggatgac     720
   cggcctttct tcctctacgt cgccttccac gacccccacc gctgtgggca ctcccagccc     780
   cagtacggaa ccttctgtga gaagtttggc aacggagaga gcggcatggg tcgtatccca     840
   gactggaccc cccaggccta cgacccactg gacgtgctgg tgccttactt cgtccccaac     900
   accccggcag cccgagccga cctggccgct cagtacacca ccgtaggccg catggaccaa     960
   ggagttggac tggtgctcca ggagctgcgt gacgccggtg tcctgaacga cacactggtg    1020
   atcttcacgt ccgacaacgg gatccccttc cccagcggca ggaccaacct gtactggccg    1080
   ggcactgctg aacccttact ggtgtcatcc ccggagcacc caaaacgctg gggccaagtc     1140
   agcgaggcct acgtgagcct cctagacctc acgcccacca tcttggattg gttctcgatc    1200
   ccgtacccca gctacgccat ctttggctcg aagaccatcc acctcactgg ccggtccctc    1260
   ctgccggcgc tggaggccga gcccctctgg gccaccgtct ttggcagcca gagccaccac    1320
   gaggtcacca tgtcctaccc catgcgctcc gtgcagcacc ggcacttccg cctcgtgcac    1380
   aacctcaact tcaagatgcc ctttcccatc gaccaggact tctacgtctc acccaccttc    1440
   caggacctcc tgaaccgcac tacagctggt cagcccacgg gctggtacaa ggacctccgt    1500
   cattactact accgggcgcg ctgggagctc tacgaccgga gccgggaccc ccacgagacc    1560
   cagaacctgg ccaccgaccc gcgctttgct cagcttctgg agatgcttcg ggaccagctg    1620
   gccaagtggc agtgggagac ccacgacccc tgggtgtgcg cccccgacgg cgtcctggag    1680
   gagaagctct ctccccagtg ccagcccctc cacaatgagc tgtaa                    1725
<210> 46
   <211> 574
   <212> PRT
   <213> Homo sapiens
<400> 46
   Met Gln Pro Ser Ser Leu Leu Pro Leu Ala Leu Cys Leu Leu Ala Ala
   1               5                   10                  15
   Pro Ala Gly Ser Ser Lys Pro Gln Ala Leu Ala Thr Pro Asn Lys Glu
               20                  25                  30
   Glu His Gly Lys Arg Lys Lys Lys Gly Lys Gly Leu Gly Lys Lys Arg
               35                  40                  45
   Asp Pro Cys Leu Arg Lys Tyr Lys Asp Phe Cys Ile His Gly Glu Cys
       50                  55                  60
   Lys Tyr Val Lys Glu Leu Arg Ala Pro Ser Cys Ile Cys His Pro Gly
   65                  70                  75                  80
   Tyr His Gly Glu Arg Cys His Gly Leu Ser Gly Ser Arg Pro Arg Asn
                   85                  90                  95
   Ala Leu Leu Leu Leu Ala Asp Asp Gly Gly Phe Glu Ser Gly Ala Tyr
               100                 105                 110
   Asn Asn Ser Ala Ile Ala Thr Pro His Leu Asp Ala Leu Ala Arg Arg
           115                 120                 125
   Ser Leu Leu Phe Arg Asn Ala Phe Thr Ser Val Ser Ser Cys Ser Pro
       130                 135                 140
   Ser Arg Ala Ser Leu Leu Thr Gly Leu Pro Gln His Gln Asn Gly Met
   145                 150                 155                 160
   Tyr Gly Leu His Gln Asp Val His His Phe Asn Ser Phe Asp Lys Val
                   165                 170                 175
   Arg Ser Leu Pro Leu Leu Leu Ser Gln Ala Gly Val Arg Thr Gly Ile
               180                 185                 190
   Ile Gly Lys Lys His Val Gly Pro Glu Thr Val Tyr Pro Phe Asp Phe
           195                 200                 205
   Ala Tyr Thr Glu Glu Asn Gly Ser Val Leu Gln Val Gly Arg Asn Ile
       210                 215                 220
   Thr Arg Ile Lys Leu Leu Val Arg Lys Phe Leu Gln Thr Gln Asp Asp
   225                 230                 235                 240
   Arg Pro Phe Phe Leu Tyr Val Ala Phe His Asp Pro His Arg Cys Gly
                   245                 250                 255
   His Ser Gln Pro Gln Tyr Gly Thr Phe Cys Glu Lys Phe Gly Asn Gly
               260                 265                 270
   Glu Ser Gly Met Gly Arg Ile Pro Asp Trp Thr Pro Gln Ala Tyr Asp
           275                 280                 285
   Pro Leu Asp Val Leu Val Pro Tyr Phe Val Pro Asn Thr Pro Ala Ala
       290                 295                 300
   Arg Ala Asp Leu Ala Ala Gln Tyr Thr Thr Val Gly Arg Met Asp Gln
   305 310 315 320
   Gly Val Gly Leu Val Leu Gln Glu Leu Arg Asp Ala Gly Val Leu Asn
   325 330 335
   Asp Thr Leu Val Ile Phe Thr Ser Asp Asn Gly Ile Pro Phe Pro Ser
   340 345 350
   Gly Arg Thr Asn Leu Tyr Trp Pro Gly Thr Ala Glu Pro Leu Leu Val
   355 360 365
   Ser Ser Pro Glu His Pro Lys Arg Trp Gly Gln Val Ser Glu Ala Tyr
   370 375 380
   Val Ser Leu Leu Asp Leu Thr Pro Thr Ile Leu Asp Trp Phe Ser Ile
   385 390 395 400
   Pro Tyr Pro Ser Tyr Ala Ile Phe Gly Ser Lys Thr Ile His Leu Thr
   405 410 415
   Gly Arg Ser Leu Leu Pro Ala Leu Glu Ala Glu Pro Leu Trp Ala Thr
   420 425 430
   Val Phe Gly Ser Gln Ser His His Glu Val Thr Met Ser Tyr Pro Met
   435 440 445
   Arg Ser Val Gln His Arg His Phe Arg Leu Val His Asn Leu Asn Phe
   450 455 460
   Lys Met Pro Phe Pro Ile Asp Gln Asp Phe Tyr Val Ser Pro Thr Phe
   465 470 475 480
   Gln Asp Leu Leu Asn Arg Thr Thr Ala Gly Gln Pro Thr Gly Trp Tyr
   485 490 495
   Lys Asp Leu Arg His Tyr Tyr Tyr Arg Ala Arg Trp Glu Leu Tyr Asp
   500 505 510
   Arg Ser Arg Asp Pro His Glu Thr Gln Asn Leu Ala Thr Asp Pro Arg
   515 520 525
   Phe Ala Gln Leu Leu Glu Met Leu Arg Asp Gln Leu Ala Lys Trp Gln
   530 535 540
   Trp Glu Thr His Asp Pro Trp Val Cys Ala Pro Asp Gly Val Leu Glu
   545 550 555 560
   Glu Lys Leu Ser Pro Gln Cys Gln Pro Leu His Asn Glu Leu
   565 570
<210> 47
   <211> 1590
   <212> DNA
   <213> Homo sapiens
<400> 47
   atgcagccct ccagccttct gccgctcgcc ctctgcctgc tggctgcacc cgccggatct      60
   gggaaaagaa agaagaaagg caaggggcta gggaagaaga gggacccatc tcttcggaaa     120
   tacaaggact tctccggatc tcgtccccgg aacgcactgc tgctcctcgc ggatgacgga     180
   ggctttgaga gtggcgcgta caacaacagc gccatcgcca ccccgcacct ggacgccttg     240
   gcccgccgca gcctcctctt tcgcaatgcc ttcacctcgg tcagcagctg ctctcccagc     300
   cgcgccagcc tcctcactgg cctgccccag catcagaatg ggatgtacgg gctgcaccag     360
   gacgtgcacc acttcaactc cttcgacaag gtgcggagcc tgccgctgct gctcagccaa     420
   gctggtgtgc gcacaggcat catcgggaag aagcacgtgg ggccggagac cgtgtacccg     480
   tttgactttg cgtacacgga ggagaatggc tccgtcctcc aggtggggcg gaacatcact     540
   agaattaagc tgctcgtccg gaaattcctg cagactcagg atgaccggcc tttcttcctc     600
   tacgtcgcct tccacgaccc ccaccgctgt gggcactccc agccccagta cggaaccttc     660
   tgtgagaagt ttggcaacgg agagagcggc atgggtcgta tcccagactg gaccccccag      720
   gcctacgacc cactggacgt gctggtgcct tacttcgtcc ccaacacccc ggcagcccga     780
   gccgacctgg ccgctcagta caccaccgta ggccgcatgg accaaggagt tggactggtg     840
   ctccaggagc tgcgtgacgc cggtgtcctg aacgacacac tggtgatctt cacgtccgac     900
   aacgggatcc ccttccccag cggcaggacc aacctgtact ggccgggcac tgctgaaccc     960
   ttactggtgt catccccgga gcacccaaaa cgctggggcc aagtcagcga ggcctacgtg    1020
   agcctcctag acctcacgcc caccatcttg gattggttct cgatcccgta ccccagctac    1080
   gccatctttg gctcgaagac catccacctc actggccggt ccctcctgcc ggcgctggag    1140
   gccgagcccc tctgggccac cgtctttggc agccagagcc accacgaggt caccatgtcc    1200
   taccccatgc gctccgtgca gcaccggcac ttccgcctcg tgcacaacct caacttcaag    1260
   atgccctttc ccatcgacca ggacttctac gtctcaccca ccttccagga cctcctgaac    1320
   cgcactacag ctggtcagcc cacgggctgg tacaaggacc tccgtcatta ctactaccgg    1380
   gcgcgctggg agctctacga ccggagccgg gacccccacg agacccagaa cctggccacc    1440
   gacccgcgct ttgctcagct tctggagatg cttcgggacc agctggccaa gtggcagtgg    1500
   gagacccacg acccctgggt gtgcgccccc gacggcgtcc tggaggagaa gctctctccc    1560
   cagtgccagc ccctccacaa tgagctgtaa                                     1590
<210> 48
   <211> 529
   <212> PRT
   <213> Homo sapiens
<400> 48
   Met Gln Pro Ser Ser Leu Leu Pro Leu Ala Leu Cys Leu Leu Ala Ala
   1               5                   10                  15
   Pro Ala Gly Ser Gly Lys Arg Lys Lys Lys Gly Lys Gly Leu Gly Lys
               20                  25                  30
   Lys Arg Asp Pro Ser Leu Arg Lys Tyr Lys Asp Phe Ser Gly Ser Arg
           35                  40                  45
   Pro Arg Asn Ala Leu Leu Leu Leu Ala Asp Asp Gly Gly Phe Glu Ser
       50                  55                  60
   Gly Ala Tyr Asn Asn Ser Ala Ile Ala Thr Pro His Leu Asp Ala Leu
   65                  70                  75                  80
   Ala Arg Arg Ser Leu Leu Phe Arg Asn Ala Phe Thr Ser Val Ser Ser
                   85                  90                  95
   Cys Ser Pro Ser Arg Ala Ser Leu Leu Thr Gly Leu Pro Gln His Gln
               100                 105                 110
   Asn Gly Met Tyr Gly Leu His Gln Asp Val His His Phe Asn Ser Phe
           115                 120                 125
   Asp Lys Val Arg Ser Leu Pro Leu Leu Leu Ser Gln Ala Gly Val Arg
       130                 135                 140
   Thr Gly Ile Ile Gly Lys Lys His Val Gly Pro Glu Thr Val Tyr Pro
   145                 150                 155                 160
   Phe Asp Phe Ala Tyr Thr Glu Glu Asn Gly Ser Val Leu Gln Val Gly
                   165                 170                 175
   Arg Asn Ile Thr Arg Ile Lys Leu Leu Val Arg Lys Phe Leu Gln Thr
               180                 185                 190
   Gln Asp Asp Arg Pro Phe Phe Leu Tyr Val Ala Phe His Asp Pro His
           195                 200                 205
   Arg Cys Gly His Ser Gln Pro Gln Tyr Gly Thr Phe Cys Glu Lys Phe
       210                 215                 220
   Gly Asn Gly Glu Ser Gly Met Gly Arg Ile Pro Asp Trp Thr Pro Gln
   225                 230                 235                 240
   Ala Tyr Asp Pro Leu Asp Val Leu Val Pro Tyr Phe Val Pro Asn Thr
                   245                 250                 255
   Pro Ala Ala Arg Ala Asp Leu Ala Ala Gln Tyr Thr Thr Val Gly Arg
               260                 265                 270
   Met Asp Gln Gly Val Gly Leu Val Leu Gln Glu Leu Arg Asp Ala Gly
           275                 280                 285
   Val Leu Asn Asp Thr Leu Val Ile Phe Thr Ser Asp Asn Gly Ile Pro
       290                 295                 300
   Phe Pro Ser Gly Arg Thr Asn Leu Tyr Trp Pro Gly Thr Ala Glu Pro
   305                 310                 315                 320
   Leu Leu Val Ser Ser Pro Glu His Pro Lys Arg Trp Gly Gln Val Ser
                   325                 330                 335
   Glu Ala Tyr Val Ser Leu Leu Asp Leu Thr Pro Thr Ile Leu Asp Trp
               340                 345                 350
   Phe Ser Ile Pro Tyr Pro Ser Tyr Ala Ile Phe Gly Ser Lys Thr Ile
           355                 360                 365
   His Leu Thr Gly Arg Ser Leu Leu Pro Ala Leu Glu Ala Glu Pro Leu
       370                 375                 380
   Trp Ala Thr Val Phe Gly Ser Gln Ser His His Glu Val Thr Met Ser
   385                 390                 395                 400
   Tyr Pro Met Arg Ser Val Gln His Arg His Phe Arg Leu Val His Asn
                   405                 410                 415
   Leu Asn Phe Lys Met Pro Phe Pro Ile Asp Gln Asp Phe Tyr Val Ser
               420                 425                 430
   Pro Thr Phe Gln Asp Leu Leu Asn Arg Thr Thr Ala Gly Gln Pro Thr
           435                 440                 445
   Gly Trp Tyr Lys Asp Leu Arg His Tyr Tyr Tyr Arg Ala Arg Trp Glu
       450                 455                 460
   Leu Tyr Asp Arg Ser Arg Asp Pro His Glu Thr Gln Asn Leu Ala Thr
   465                 470                 475                 480
   Asp Pro Arg Phe Ala Gln Leu Leu Glu Met Leu Arg Asp Gln Leu Ala
                   485                 490                 495
   Lys Trp Gln Trp Glu Thr His Asp Pro Trp Val Cys Ala Pro Asp Gly
   500 505 510
   Val Leu Glu Glu Lys Leu Ser Pro Gln Cys Gln Pro Leu His Asn Glu
   515 520 525
   Leu
<210> 49
   <211> 1650
   <212> DNA
   <213> Homo sapiens
<400> 49
   atgcagccct ccagccttct gccgctcgcc ctctgcctgc tggctgcacc cgccggatct 60
   gggaaaagaa agaagaaagg caaggggcta gggaagaaga gggacccatc tcttcggaaa 120
   tacaaggact tctccggatc tcgtccccgg aacgcactgc tgctcctcgc ggatgacgga 180
   ggctttgaga gtggcgcgta caacaacagc gccatcgcca ccccgcacct ggacgccttg 240
   gcccgccgca gcctcctctt tcgcaatgcc ttcacctcgg tcagcagctg ctctcccagc 300
   cgcgccagcc tcctcactgg cctgccccag catcagaatg ggatgtacgg gctgcaccag 360
   gacgtgcacc acttcaactc cttcgacaag gtgcggagcc tgccgctgct gctcagccaa 420
   gctggtgtgc gcacaggcat catcgggaag aagcacgtgg ggccggagac cgtgtacccg 480
   tttgactttg cgtacacgga ggagaatggc tccgtcctcc aggtggggcg gaacatcact 540
   agaattaagc tgctcgtccg gaaattcctg cagactcagg atgaccggcc tttcttcctc 600
   tacgtcgcct tccacgaccc ccaccgctgt gggcactccc agccccagta cggaaccttc 660
   tgtgagaagt ttggcaacgg agagagcggc atgggtcgta tcccagactg gaccccccag 720
   gcctacgacc cactggacgt gctggtgcct tacttcgtcc ccaacacccc ggcagcccga 780
   gccgacctgg ccgctcagta caccaccgta ggccgcatgg accaaggagt tggactggtg 840
   ctccaggagc tgcgtgacgc cggtgtcctg aacgacacac tggtgatctt cacgtccgac 900
   aacgggatcc ccttccccag cggcaggacc aacctgtact ggccgggcac tgctgaaccc 960
   ttactggtgt catccccgga gcacccaaaa cgctggggcc aagtcagcga ggcctacgtg 1020
   agcctcctag acctcacgcc caccatcttg gattggttct cgatcccgta ccccagctac 1080
   gccatctttg gctcgaagac catccacctc actggccggt ccctcctgcc ggcgctggag 1140
   gccgagcccc tctgggccac cgtctttggc agccagagcc accacgaggt caccatgtcc 1200
   taccccatgc gctccgtgca gcaccggcac ttccgcctcg tgcacaacct caacttcaag 1260
   atgccctttc ccatcgacca ggacttctac gtctcaccca ccttccagga cctcctgaac 1320
   cgcactacag ctggtcagcc cacgggctgg tacaaggacc tccgtcatta ctactaccgg 1380
   gcgcgctggg agctctacga ccggagccgg gacccccacg agacccagaa cctggccacc 1440
   gacccgcgct ttgctcagct tctggagatg cttcgggacc agctggccaa gtggcagtgg 1500
   gagacccacg acccctgggt gtgcgccccc gacggcgtcc tggaggagaa gctctctccc 1560
   cagtgccagc ccctccacaa tgagctgaga tcccccgggc gccagataaa gatttggttc 1620
   cagaatcggc gcatgaagtg gaagaagtaa 1650
<210> 50
   <211> 549
   <212> PRT
   <213> Homo sapiens
<400> 50
   Met Gln Pro Ser Ser Leu Leu Pro Leu Ala Leu Cys Leu Leu Ala Ala
   1               5                   10                  15
   Pro Ala Gly Ser Gly Lys Arg Lys Lys Lys Gly Lys Gly Leu Gly Lys
               20                  25                  30
   Lys Arg Asp Pro Ser Leu Arg Lys Tyr Lys Asp Phe Ser Gly Ser Arg
           35                  40                  45
   Pro Arg Asn Ala Leu Leu Leu Leu Ala Asp Asp Gly Gly Phe Glu Ser
       50                  55                  60
   Gly Ala Tyr Asn Asn Ser Ala Ile Ala Thr Pro His Leu Asp Ala Leu
   65                  70                  75                  80
   Ala Arg Arg Ser Leu Leu Phe Arg Asn Ala Phe Thr Ser Val Ser Ser
                   85                  90                  95
   Cys Ser Pro Ser Arg Ala Ser Leu Leu Thr Gly Leu Pro Gln His Gln
               100                 105                 110
   Asn Gly Met Tyr Gly Leu His Gln Asp Val His His Phe Asn Ser Phe
           115                 120                 125
   Asp Lys Val Arg Ser Leu Pro Leu Leu Leu Ser Gln Ala Gly Val Arg
       130                 135                 140
   Thr Gly Ile Ile Gly Lys Lys His Val Gly Pro Glu Thr Val Tyr Pro
   145                 150                 155                 160
   Phe Asp Phe Ala Tyr Thr Glu Glu Asn Gly Ser Val Leu Gln Val Gly
                   165                 170                 175
   Arg Asn Ile Thr Arg Ile Lys Leu Leu Val Arg Lys Phe Leu Gln Thr
               180                 185                 190
   Gln Asp Asp Arg Pro Phe Phe Leu Tyr Val Ala Phe His Asp Pro His
   195 200 205
   Arg Cys Gly His Ser Gln Pro Gln Tyr Gly Thr Phe Cys Glu Lys Phe
   210 215 220
   Gly Asn Gly Glu Ser Gly Met Gly Arg Ile Pro Asp Trp Thr Pro Gln
   225 230 235 240
   Ala Tyr Asp Pro Leu Asp Val Leu Val Pro Tyr Phe Val Pro Asn Thr
   245 250 255
   Pro Ala Ala Arg Ala Asp Leu Ala Ala Gln Tyr Thr Thr Val Gly Arg
   260 265 270
   Met Asp Gln Gly Val Gly Leu Val Leu Gln Glu Leu Arg Asp Ala Gly
   275 280 285
   Val Leu Asn Asp Thr Leu Val Ile Phe Thr Ser Asp Asn Gly Ile Pro
   290 295 300
   Phe Pro Ser Gly Arg Thr Asn Leu Tyr Trp Pro Gly Thr Ala Glu Pro
   305 310 315 320
   Leu Leu Val Ser Ser Pro Glu His Pro Lys Arg Trp Gly Gln Val Ser
   325 330 335
   Glu Ala Tyr Val Ser Leu Leu Asp Leu Thr Pro Thr Ile Leu Asp Trp
   340 345 350
   Phe Ser Ile Pro Tyr Pro Ser Tyr Ala Ile Phe Gly Ser Lys Thr Ile
   355 360 365
   His Leu Thr Gly Arg Ser Leu Leu Pro Ala Leu Glu Ala Glu Pro Leu
   370 375 380
   Trp Ala Thr Val Phe Gly Ser Gln Ser His His Glu Val Thr Met Ser
   385 390 395 400
   Tyr Pro Met Arg Ser Val Gln His Arg His Phe Arg Leu Val His Asn
   405 410 415
   Leu Asn Phe Lys Met Pro Phe Pro Ile Asp Gln Asp Phe Tyr Val Ser
   420 425 430
   Pro Thr Phe Gln Asp Leu Leu Asn Arg Thr Thr Ala Gly Gln Pro Thr
   435 440 445
   Gly Trp Tyr Lys Asp Leu Arg His Tyr Tyr Tyr Arg Ala Arg Trp Glu
       450                 455                 460
   Leu Tyr Asp Arg Ser Arg Asp Pro His Glu Thr Gln Asn Leu Ala Thr
   465                 470                 475                 480
   Asp Pro Arg Phe Ala Gln Leu Leu Glu Met Leu Arg Asp Gln Leu Ala
                   485                 490                 495
   Lys Trp Gln Trp Glu Thr His Asp Pro Trp Val Cys Ala Pro Asp Gly
               500                 505                 510
   Val Leu Glu Glu Lys Leu Ser Pro Gln Cys Gln Pro Leu His Asn Glu
           515                 520                 525
   Leu Arg Ser Pro Gly Arg Gln Ile Lys Ile Trp Phe Gln Asn Arg Arg
       530                 535                 540
   Met Lys Trp Lys Lys
   545
<210> 51
   <211> 1692
   <212> DNA
   <213> Homo sapiens
<400> 51
   atgggactcc aagcctgcct cctagggctc tttgccctca tcctctctgg caaatgcagt 60
   tacagcccgg agcccgacca gcggaggacg ctgcccccag gctgggtgtc cctgggccgt 120
   gcggaccctg aggaagagct gagtctcacc tttgccctga gacagcagaa tgtggaaaga 180
   ctctcggagc tggtgcaggc tgtgtcggat cccagctctc ctcaatacgg aaaatacctg 240
   accctagaga atgtggctga tctggtgagg ccatccccac tgaccctcca cacggtgcaa 300
   aaatggctct tggcagccgg agcccagaag tgccattctg tgatcacaca ggactttctg 360
   acttgctggc tgagcatccg acaagcagag ctgctgctcc ctggggctga gtttcatcac 420
   tatgtgggag gacctacgga aacccatgtt gtaaggtccc cacatcccta ccagcttcca 480
   caggccttgg ccccccatgt ggactttgtg gggggactgc accgttttcc cccaacatca 540
   tccctgaggc aacgtcctga gccgcaggtg acagggactg taggcctgca tctgggggta 600
   accccctctg tgatccgtaa gcgatacaac ttgacctcac aagacgtggg ctctggcacc 660
   agcaataaca gccaagcctg tgcccagttc ctggagcagt atttccatga ctcagacctg 720
   gctcagttca tgcgcctctt cggtggcaac tttgcacatc aggcatcagt agcccgtgtg 780
   gttggacaac agggccgggg ccgggccggg attgaggcca gtctagatgt gcagtacctg 840
   atgagtgctg gtgccaacat ctccacctgg gtctacagta gccctggccg gcatgaggga 900
   caggagccct tcctgcagtg gctcatgctg ctcagtaatg agtcagccct gccacatgtg 960
   catactgtga gctatggaga tgatgaggac tccctcagca gcgcctacat ccagcgggtc 1020
   aacactgagc tcatgaaggc tgccgctcgg ggtctcaccc tgctcttcgc ctcaggtgac 1080
   agtggggccg ggtgttggtc tgtctctgga agacaccagt tccgccctac cttccctgcc 1140
   tccagcccct atgtcaccac agtgggaggc acatccttcc aggaaccttt cctcatcaca 1200
   aatgaaattg ttgactatat cagtggtggt ggcttcagca atgtgttccc acggccttca 1260
   taccaggagg aagctgtaac gaagttcctg agctctagcc cccacctgcc accatccagt 1320
   tacttcaatg ccagtggccg tgcctaccca gatgtggctg cactttctga tggctactgg 1380
   gtggtcagca acagagtgcc cattccatgg gtgtccggaa cctcggcctc tactccagtg 1440
   tttgggggga tcctatcctt gatcaatgag cacaggatcc ttagtggccg cccccctctt 1500
   ggctttctca acccaaggct ctaccagcag catggggcag gactctttga tgtaacccgt 1560
   ggctgccatg agtcctgtct ggatgaagag gtagagggcc agggtttctg ctctggtcct 1620
   ggctgggatc ctgtaacagg ctggggaaca cccaacttcc cagctttgct gaagactcta 1680
   ctcaacccct ga 1692
<210> 52
   <211> 563
   <212> PRT
   <213> Homo sapiens
<400> 52
<210> 53
   <211> 1239
   <212> DNA
   <213> Homo sapiens
<400> 53
   atgcagccct ccagccttct gccgctcgcc ctctgcctgc tggctgcacc cgcctccgcg 60
   ctcgtcagga tcccgctgca caagttcacg tccatccgcc ggaccatgtc ggaggttggg 120
   ggctctgtgg aggacctgat tgccaaaggc cccgtctcaa agtactccca ggcggtgcca 180
   gccgtgaccg aggggcccat tcccgaggtg ctcaagaact acatggacgc ccagtactac 240
   ggggagattg gcatcgggac gcccccccag tgcttcacag tcgtcttcga cacgggctcc 300
   tccaacctgt gggtcccctc catccactgc aaactgctgg acatcgcttg ctggatccac      360
   cacaagtaca acagcgacaa gtccagcacc tacgtgaaga atggtacctc gtttgacatc      420
   cactatggct cgggcagcct ctccgggtac ctgagccagg acactgtgtc ggtgccctgc      480
   cagtcagcgt cgtcagcctc tgccctgggc ggtgtcaaag tggagaggca ggtctttggg      540
   gaggccacca agcagccagg catcaccttc atcgcagcca agttcgatgg catcctgggc      600
   atggcctacc cccgcatctc cgtcaacaac gtgctgcccg tcttcgacaa cctgatgcag      660
   cagaagctgg tggaccagaa catcttctcc ttctacctga gcagggaccc agatgcgcag      720
   cctgggggtg agctgatgct gggtggcaca gactccaagt attacaaggg ttctctgtcc      780
   tacctgaatg tcacccgcaa ggcctactgg caggtccacc tggaccaggt ggaggtggcc      840
   agcgggctga ccctgtgcaa ggagggctgt gaggccattg tggacacagg cacttccctc      900
   atggtgggcc cggtggatga ggtgcgcgag ctgcagaagg ccatcggggc cgtgccgctg      960
   attcagggcg agtacatgat cccctgtgag aaggtgtcca ccctgcccgc gatcacactg     1020
   aagctgggag gcaaaggcta caagctgtcc ccagaggact acacgctcaa ggtgtcgcag     1080
   gccgggaaga ccctctgcct gagcggcttc atgggcatgg acatcccgcc acccagcggg     1140
   ccactctgga tcctgggcga cgtcttcatc ggccgctact acactgtgtt tgaccgtgac     1200
   aacaacaggg tgggcttcgc cgaggctgcc cgcctctag                            1239
<210> 54
   <211> 412
   <212> PRT
   <213> Homo sapiens
<400> 54
   Met Gln Pro Ser Ser Leu Leu Pro Leu Ala Leu Cys Leu Leu Ala Ala
   1               5                   10                  15
   Pro Ala Ser Ala Leu Val Arg Ile Pro Leu His Lys Phe Thr Ser Ile
               20                  25                  30
   Arg Arg Thr Met Ser Glu Val Gly Gly Ser Val Glu Asp Leu Ile Ala
           35                  40                  45
   Lys Gly Pro Val Ser Lys Tyr Ser Gln Ala Val Pro Ala Val Thr Glu
       50                  55                  60
   Gly Pro Ile Pro Glu Val Leu Lys Asn Tyr Met Asp Ala Gln Tyr Tyr
   65                  70                  75                  80
   Gly Glu Ile Gly Ile Gly Thr Pro Pro Gln Cys Phe Thr Val Val Phe
                   85                  90                  95
   Asp Thr Gly Ser Ser Asn Leu Trp Val Pro Ser Ile His Cys Lys Leu
               100                 105                 110
   Leu Asp Ile Ala Cys Trp Ile His His Lys Tyr Asn Ser Asp Lys Ser
           115                 120                 125
   Ser Thr Tyr Val Lys Asn Gly Thr Ser Phe Asp Ile His Tyr Gly Ser
       130                 135                 140
   Gly Ser Leu Ser Gly Tyr Leu Ser Gln Asp Thr Val Ser Val Pro Cys
   145                 150                 155                 160
   Gln Ser Ala Ser Ser Ala Ser Ala Leu Gly Gly Val Lys Val Glu Arg
                   165                 170                 175
   Gln Val Phe Gly Glu Ala Thr Lys Gln Pro Gly Ile Thr Phe Ile Ala
               180                 185                 190
   Ala Lys Phe Asp Gly Ile Leu Gly Met Ala Tyr Pro Arg Ile Ser Val
           195                 200                 205
   Asn Asn Val Leu Pro Val Phe Asp Asn Leu Met Gln Gln Lys Leu Val
       210                 215                 220
   Asp Gln Asn Ile Phe Ser Phe Tyr Leu Ser Arg Asp Pro Asp Ala Gln
   225                 230                 235                 240
   Pro Gly Gly Glu Leu Met Leu Gly Gly Thr Asp Ser Lys Tyr Tyr Lys
                   245                 250                 255
   Gly Ser Leu Ser Tyr Leu Asn Val Thr Arg Lys Ala Tyr Trp Gln Val
               260                 265                 270
   His Leu Asp Gln Val Glu Val Ala Ser Gly Leu Thr Leu Cys Lys Glu
           275                 280                 285
   Gly Cys Glu Ala Ile Val Asp Thr Gly Thr Ser Leu Met Val Gly Pro
       290                 295                 300
   Val Asp Glu Val Arg Glu Leu Gln Lys Ala Ile Gly Ala Val Pro Leu
   305                 310                 315                 320
   Ile Gln Gly Glu Tyr Met Ile Pro Cys Glu Lys Val Ser Thr Leu Pro
                   325                 330                 335
   Ala Ile Thr Leu Lys Leu Gly Gly Lys Gly Tyr Lys Leu Ser Pro Glu
               340                 345                 350
   Asp Tyr Thr Leu Lys Val Ser Gln Ala Gly Lys Thr Leu Cys Leu Ser
   355 360 365
   Gly Phe Met Gly Met Asp Ile Pro Pro Pro Ser Gly Pro Leu Trp Ile
   370 375 380
   Leu Gly Asp Val Phe Ile Gly Arg Tyr Tyr Thr Val Phe Asp Arg Asp
   385 390 395 400
   Asn Asn Arg Val Gly Phe Ala Glu Ala Ala Arg Leu
   405 410
<210> 55
   <211> 921
   <212> DNA
   <213> Homo sapiens
<400> 55
   atggcgtcgc ccggctgcct gtggctcttg gctgtggctc tcctgccatg gacctgcgct 60
   tctcgggcgc tgcagcatct ggacccgccg gcgccgctgc cgttggtgat ctggcatggg 120
   atgggagaca gctgttgcaa tcccttaagc atgggtgcta ttaaaaaaat ggtggagaag 180
   aaaatacctg gaatttacgt cttatcttta gagattggga agaccctgat ggaggacgtg 240
   gagaacagct tcttcttgaa tgtcaattcc caagtaacaa cagtgtgtca ggcacttgct 300
   aaggatccta aattgcagca aggctacaat gctatgggat tctcccaggg aggccaattt 360
   ctgagggcag tggctcagag atgcccttca cctcccatga tcaatctgat ctcggttggg 420
   ggacaacatc aaggtgtttt tggactccct cgatgcccag gagagagctc tcacatctgt 480
   gacttcatcc gaaaaacact gaatgctggg gcgtactcca aagttgttca ggaacgcctc 540
   gtgcaagccg aatactggca tgaccccata aaggaggatg tgtatcgcaa ccacagcatc 600
   ttcttggcag atataaatca ggagcggggt atcaatgagt cctacaagaa aaacctgatg 660
   gccctgaaga agtttgtgat ggtgaaattc ctcaatgatt ccattgtgga ccctgtagat 720
   tcggagtggt ttggatttta cagaagtggc caagccaagg aaaccattcc cttacaggag 780
   acctccctgt acacacagga ccgcctgggg ctaaaggaaa tggacaatgc aggacagcta 840
   gtgtttctgg ctacagaagg ggaccatctt cagttgtctg aagaatggtt ttatgcccac 900
   atcataccat tccttggatg a 921
<210> 56
   <211> 306
   <212> PRT
   <213> Homo sapiens
<400> 56
<210> 57
   <211> 1509
   <212> DNA
   <213> Homo sapiens
<400> 57
   atgagctgcc ccgtgcccgc ctgctgcgcg ctgctgctag tcctggggct ctgccgggcg      60
   cgtccccgga acgcactgct gctcctcgcg gatgacggag gctttgagag tggcgcgtac     120
   aacaacagcg ccatcgccac cccgcacctg gacgccttgg cccgccgcag cctcctcttt     180
   cgcaatgcct tcacctcggt cagcagctgc tctcccagcc gcgccagcct cctcactggc     240
   ctgccccagc atcagaatgg gatgtacggg ctgcaccagg acgtgcacca cttcaactcc     300
   ttcgacaagg tgcggagcct gccgctgctg ctcagccaag ctggtgtgcg cacaggcatc     360
   atcgggaaga agcacgtggg gccggagacc gtgtacccgt ttgactttgc gtacacggag     420
   gagaatggct ccgtcctcca ggtggggcgg aacatcacta gaattaagct gctcgtccgg     480
   aaattcctgc agactcagga tgaccggcct ttcttcctct acgtcgcctt ccacgacccc     540
   caccgctgtg ggcactccca gccccagtac ggaaccttct gtgagaagtt tggcaacgga     600
   gagagcggca tgggtcgtat cccagactgg accccccagg cctacgaccc actggacgtg     660
   ctggtgcctt acttcgtccc caacaccccg gcagcccgag ccgacctggc cgctcagtac     720
   accaccgtag gccgcatgga ccaaggagtt ggactggtgc tccaggagct gcgtgacgcc     780
   ggtgtcctga acgacacact ggtgatcttc acgtccgaca acgggatccc cttccccagc     840
   ggcaggacca acctgtactg gccgggcact gctgaaccct tactggtgtc atccccggag     900
   cacccaaaac gctggggcca agtcagcgag gcctacgtga gcctcctaga cctcacgccc     960
   accatcttgg attggttctc gatcccgtac cccagctacg ccatctttgg ctcgaagacc    1020
   atccacctca ctggccggtc cctcctgccg gcgctggagg ccgagcccct ctgggccacc    1080
   gtctttggca gccagagcca ccacgaggtc accatgtcct accccatgcg ctccgtgcag    1140
   caccggcact tccgcctcgt gcacaacctc aacttcaaga tgccctttcc catcgaccag    1200
   gacttctacg tctcacccac cttccaggac ctcctgaacc gcactacagc tggtcagccc    1260
   acgggctggt acaaggacct ccgtcattac tactaccggg cgcgctggga gctctacgac    1320
   cggagccggg acccccacga gacccagaac ctggccaccg acccgcgctt tgctcagctt    1380
   ctggagatgc ttcgggacca gctggccaag tggcagtggg agacccacga cccctgggtg    1440
   tgcgcccccg acggcgtcct ggaggagaag ctctctcccc agtgccagcc cctccacaat    1500
   gagctgtga                                                            1509
<210> 58
   <211> 502
   <212> PRT
   <213> Homo sapiens
<400> 58
<210> 59
   <211> 1962
   <212> DNA
   <213> Homo sapiens
<400> 59
   atgcgtcccc tgcgcccccg cgccgcgctg ctggcgctcc tggcctcgct cctggccgcg      60
   cccccggtgg ccccggccga ggccccgcac ctggtgcatg tggacgcggc ccgcgcgctg     120
   tggcccctgc ggcgcttctg gaggagcaca ggcttctgcc ccccgctgcc acacagccag     180
   gctgaccagt acgtcctcag ctgggaccag cagctcaacc tcgcctatgt gggcgccgtc     240
   cctcaccgcg gcatcaagca ggtccggacc cactggctgc tggagcttgt caccaccagg     300
   gggtccactg gacggggcct gagctacaac ttcacccacc tggacgggta cctggacctt     360
   ctcagggaga accagctcct cccagggttt gagctgatgg gcagcgcctc gggccacttc     420
   actgactttg aggacaagca gcaggtgttt gagtggaagg acttggtctc cagcctggcc     480
   aggagataca tcggtaggta cggactggcg catgtttcca agtggaactt cgagacgtgg     540
   aatgagccag accaccacga ctttgacaac gtctccatga ccatgcaagg cttcctgaac     600
   tactacgatg cctgctcgga gggtctgcgc gccgccagcc ccgccctgcg gctgggaggc     660
   cccggcgact ccttccacac cccaccgcga tccccgctga gctggggcct cctgcgccac     720
   tgccacgacg gtaccaactt cttcactggg gaggcgggcg tgcggctgga ctacatctcc     780
   ctccacagga agggtgcgcg cagctccatc tccatcctgg agcaggagaa ggtcgtcgcg     840
   cagcagatcc ggcagctctt ccccaagttc gcggacaccc ccatttacaa cgacgaggcg     900
   gacccgctgg tgggctggtc cctgccacag ccgtggaggg cggacgtgac ctacgcggcc     960
   atggtggtga aggtcatcgc gcagcatcag aacctgctac tggccaacac cacctccgcc    1020
   ttcccctacg cgctcctgag caacgacaat gccttcctga gctaccaccc gcaccccttc    1080
   gcgcagcgca cgctcaccgc gcgcttccag gtcaacaaca cccgcccgcc gcacgtgcag    1140
   ctgttgcgca agccggtgct cacggccatg gggctgctgg cgctgctgga tgaggagcag    1200
   ctctgggccg aagtgtcgca ggccgggacc gtcctggaca gcaaccacac ggtgggcgtc    1260
   ctggccagcg cccaccgccc ccagggcccg gccgacgcct ggcgcgccgc ggtgctgatc    1320
   tacgcgagcg acgacacccg cgcccacccc aaccgcagcg tcgcggtgac cctgcggctg    1380
   cgcggggtgc cccccggccc gggcctggtc tacgtcacgc gctacctgga caacgggctc    1440
   tgcagccccg acggcgagtg gcggcgcctg ggccggcccg tcttccccac ggcagagcag    1500
   ttccggcgca tgcgcgcggc tgaggacccg gtggccgcgg cgccccgccc cttacccgcc 1560
   ggcggccgcc tgaccctgcg ccccgcgctg cggctgccgt cgcttttgct ggtgcacgtg 1620
   tgtgcgcgcc ccgagaagcc gcccgggcag gtcacgcggc tccgcgccct gcccctgacc 1680
   caagggcagc tggttctggt ctggtcggat gaacacgtgg gctccaagtg cctgtggaca 1740
   tacgagatcc agttctctca ggacggtaag gcgtacaccc cggtcagcag gaagccatcg 1800
   accttcaacc tctttgtgtt cagcccagac acaggtgctg tctctggctc ctaccgagtt 1860
   cgagccctgg actactgggc ccgaccaggc cccttctcgg accctgtgcc gtacctggag 1920
   gtccctgtgc caagagggcc cccatccccg ggcaatccat ga 1962
<210> 60
   <211> 653
   <212> PRT
   <213> Homo sapiens
<400> 60
<210> 61
   <211> 1653
   <212> DNA
   <213> Homo sapiens
<400> 61
   atgccgccac cccggaccgg ccgaggcctt ctctggctgg gtctggttct gagctccgtc      60
   tgcgtcgccc tcggatccga aacgcaggcc aactcgacca cagatgctct gaacgttctt     120
   ctcatcatcg tggatgacct gcgcccctcc ctgggctgtt atggggataa gctggtgagg     180
   tccccaaata ttgaccaact ggcatcccac agcctcctct tccagaatgc ctttgcgcag     240
   caagcagtgt gcgccccgag ccgcgtttct ttcctcactg gcaggagacc tgacaccacc     300
   cgcctgtacg acttcaactc ctactggagg gtgcacgctg gaaacttctc caccatcccc     360
   cagtacttca aggagaatgg ctatgtgacc atgtcggtgg gaaaagtctt tcaccctggg     420
   atatcttcta accataccga tgattctccg tatagctggt cttttccacc ttatcatcct     480
   tcctctgaga agtatgaaaa cactaagaca tgtcgagggc cagatggaga actccatgcc     540
   aacctgcttt gccctgtgga tgtgctggat gttcccgagg gcaccttgcc tgacaaacag     600
   agcactgagc aagccataca gttgttggaa aagatgaaaa cgtcagccag tcctttcttc     660
   ctggccgttg ggtatcataa gccacacatc cccttcagat accccaagga atttcagaag     720
   ttgtatccct tggagaacat caccctggcc cccgatcccg aggtccctga tggcctaccc     780
   cctgtggcct acaacccctg gatggacatc aggcaacggg aagacgtcca agccttaaac     840
   atcagtgtgc cgtatggtcc aattcctgtg gactttcagc ggaaaatccg ccagagctac     900
   tttgcctctg tgtcatattt ggatacacag gtcggccgcc tcttgagtgc tttggacgat     960
   cttcagctgg ccaacagcac catcattgca tttacctcgg atcatgggtg ggctctaggt    1020
   gaacatggag aatgggccaa atacagcaat tttgatgttg ctacccatgt tcccctgata    1080
   ttctatgttc ctggaaggac ggcttcactt ccggaggcag gcgagaagct tttcccttac     1140
   ctcgaccctt ttgattccgc ctcacagttg atggagccag gcaggcaatc catggacctt    1200
   gtggaacttg tgtctctttt tcccacgctg gctggacttg caggactgca ggttccacct    1260
   cgctgccccg ttccttcatt tcacgttgag ctgtgcagag aaggcaagaa ccttctgaag    1320
   cattttcgat tccgtgactt ggaagaggat ccgtacctcc ctggtaatcc ccgtgaactg    1380
   attgcctata gccagtatcc ccggccttca gacatccctc agtggaattc tgacaagccg    1440
   agtttaaaag atataaagat catgggctat tccatacgca ccatagacta taggtatact    1500
   gtgtgggttg gcttcaatcc tgatgaattt ctagctaact tttctgacat ccatgcaggg    1560
   gaactgtatt ttgtggattc tgacccattg caggatcaca atatgtataa tgattcccaa    1620
   ggtggagatc ttttccagtt gttgatgcct tga                                 1653
<210> 62
   <211> 550
   <212> PRT
   <213> Homo sapiens
<400> 62
   Met Pro Pro Pro Arg Thr Gly Arg Gly Leu Leu Trp Leu Gly Leu Val
   1 5 10 15
   Leu Ser Ser Val Cys Val Ala Leu Gly Ser Glu Thr Gln Ala Asn Ser
   20 25 30
   Thr Thr Asp Ala Leu Asn Val Leu Leu Ile Ile Val Asp Asp Leu Arg
   35 40 45
   Pro Ser Leu Gly Cys Tyr Gly Asp Lys Leu Val Arg Ser Pro Asn Ile
   50 55 60
   Asp Gln Leu Ala Ser His Ser Leu Leu Phe Gln Asn Ala Phe Ala Gln
   65 70 75 80
   Gln Ala Val Cys Ala Pro Ser Arg Val Ser Phe Leu Thr Gly Arg Arg
   85 90 95
   Pro Asp Thr Thr Arg Leu Tyr Asp Phe Asn Ser Tyr Trp Arg Val His
   100 105 110
   Ala Gly Asn Phe Ser Thr Ile Pro Gln Tyr Phe Lys Glu Asn Gly Tyr
   115 120 125
   Val Thr Met Ser Val Gly Lys Val Phe His Pro Gly Ile Ser Ser Asn
   130 135 140
   His Thr Asp Asp Ser Pro Tyr Ser Trp Ser Phe Pro Pro Tyr His Pro
   145 150 155 160
   Ser Ser Glu Lys Tyr Glu Asn Thr Lys Thr Cys Arg Gly Pro Asp Gly
   165 170 175
   Glu Leu His Ala Asn Leu Leu Cys Pro Val Asp Val Leu Asp Val Pro
   180 185 190
   Glu Gly Thr Leu Pro Asp Lys Gln Ser Thr Glu Gln Ala Ile Gln Leu
   195 200 205
   Leu Glu Lys Met Lys Thr Ser Ala Ser Pro Phe Phe Leu Ala Val Gly
   210 215 220
   Tyr His Lys Pro His Ile Pro Phe Arg Tyr Pro Lys Glu Phe Gln Lys
   225 230 235 240
   Leu Tyr Pro Leu Glu Asn Ile Thr Leu Ala Pro Asp Pro Glu Val Pro
   245 250 255
   Asp Gly Leu Pro Pro Val Ala Tyr Asn Pro Trp Met Asp Ile Arg Gln
   260 265 270
   Arg Glu Asp Val Gln Ala Leu Asn Ile Ser Val Pro Tyr Gly Pro Ile
   275 280 285
   Pro Val Asp Phe Gln Arg Lys Ile Arg Gln Ser Tyr Phe Ala Ser Val
   290 295 300
   Ser Tyr Leu Asp Thr Gln Val Gly Arg Leu Leu Ser Ala Leu Asp Asp
   305 310 315 320
   Leu Gln Leu Ala Asn Ser Thr Ile Ile Ala Phe Thr Ser Asp His Gly
   325 330 335
   Trp Ala Leu Gly Glu His Gly Glu Trp Ala Lys Tyr Ser Asn Phe Asp
   340 345 350
   Val Ala Thr His Val Pro Leu Ile Phe Tyr Val Pro Gly Arg Thr Ala
   355 360 365
   Ser Leu Pro Glu Ala Gly Glu Lys Leu Phe Pro Tyr Leu Asp Pro Phe
   370 375 380
   Asp Ser Ala Ser Gln Leu Met Glu Pro Gly Arg Gln Ser Met Asp Leu
   385 390 395 400
   Val Glu Leu Val Ser Leu Phe Pro Thr Leu Ala Gly Leu Ala Gly Leu
   405 410 415
   Gln Val Pro Pro Arg Cys Pro Val Pro Ser Phe His Val Glu Leu Cys
   420 425 430
   Arg Glu Gly Lys Asn Leu Leu Lys His Phe Arg Phe Arg Asp Leu Glu
   435 440 445
   Glu Asp Pro Tyr Leu Pro Gly Asn Pro Arg Glu Leu Ile Ala Tyr Ser
   450 455 460
   Gln Tyr Pro Arg Pro Ser Asp Ile Pro Gln Trp Asn Ser Asp Lys Pro
   465 470 475 480
   Ser Leu Lys Asp Ile Lys Ile Met Gly Tyr Ser Ile Arg Thr Ile Asp
   485 490 495
   Tyr Arg Tyr Thr Val Trp Val Gly Phe Asn Pro Asp Glu Phe Leu Ala
   500 505 510
   Asn Phe Ser Asp Ile His Ala Gly Glu Leu Tyr Phe Val Asp Ser Asp
   515 520 525
   Pro Leu Gln Asp His Asn Met Tyr Asn Asp Ser Gln Gly Gly Asp Leu
   530 535 540
   Phe Gln Leu Leu Met Pro
   545 550
<210> 63
   <211> 1524
   <212> DNA
   <213> Homo sapiens
<400> 63
   atgggggcac cgcggtccct cctcctggcc ctggctgctg gcctggccgt tgcccgtccg 60
   cccaacatcg tgctgatctt tgccgacgac ctcggctatg gggacctggg ctgctatggg 120
   caccccagct ctaccactcc caacctggac cagctggcgg cgggagggct gcggttcaca 180
   gacttctacg tgcctgtgtc tctgtgcaca ccctctaggg ccgccctcct gaccggccgg 240
   ctcccggttc ggatgggcat gtaccctggc gtcctggtgc ccagctcccg ggggggcctg 300
   cccctggagg aggtgaccgt ggccgaagtc ctggctgccc gaggctacct cacaggaatg 360
   gccggcaagt ggcaccttgg ggtggggcct gagggggcct tcctgccccc ccatcagggc 420
   ttccatcgat ttctaggcat cccgtactcc cacgaccagg gcccctgcca gaacctgacc 480
   tgcttcccgc cggccactcc ttgcgacggt ggctgtgacc agggcctggt ccccatccca 540
   ctgttggcca acctgtccgt ggaggcgcag cccccctggc tgcccggact agaggcccgc 600
   tacatggctt tcgcccatga cctcatggcc gacgcccagc gccaggatcg ccccttcttc 660
   ctgtactatg cctctcacca cacccactac cctcagttca gtgggcagag ctttgcagag 720
   cgttcaggcc gcgggccatt tggggactcc ctgatggagc tggatgcagc tgtggggacc 780
   ctgatgacag ccatagggga cctggggctg cttgaagaga cgctggtcat cttcactgca 840
   gacaatggac ctgagaccat gcgtatgtcc cgaggcggct gctccggtct cttgcggtgt 900
   ggaaagggaa cgacctacga gggcggtgtc cgagagcctg ccttggcctt ctggccaggt 960
   catatcgctc ccggcgtgac ccacgagctg gccagctccc tggacctgct gcctaccctg 1020
   gcagccctgg ctggggcccc actgcccaat gtcaccttgg atggctttga cctcagcccc 1080
   ctgctgctgg gcacaggcaa gagccctcgg cagtctctct tcttctaccc gtcctaccca 1140
   gacgaggtcc gtggggtttt tgctgtgcgg actggaaagt acaaggctca cttcttcacc 1200
   cagggctctg cccacagtga taccactgca gaccctgcct gccacgcctc cagctctctg 1260
   actgctcatg agcccccgct gctctatgac ctgtccaagg accctggtga gaactacaac 1320
   ctgctggggg gtgtggccgg ggccacccca gaggtgctgc aagccctgaa acagcttcag 1380
   ctgctcaagg cccagttaga cgcagctgtg accttcggcc ccagccaggt ggcccggggc 1440
   gaggaccccg ccctgcagat ctgctgtcat cctggctgca ccccccgccc agcttgctgc 1500
   cattgcccag atccccatgc ctga 1524
<210> 64
   <211> 507
   <212> PRT
   <213> Homo sapiens
<400> 64
   Met Gly Ala Pro Arg Ser Leu Leu Leu Ala Leu Ala Ala Gly Leu Ala
   1               5                   10                  15
   Val Ala Arg Pro Pro Asn Ile Val Leu Ile Phe Ala Asp Asp Leu Gly
               20                  25                  30
   Tyr Gly Asp Leu Gly Cys Tyr Gly His Pro Ser Ser Thr Thr Pro Asn
           35                  40                  45
   Leu Asp Gln Leu Ala Ala Gly Gly Leu Arg Phe Thr Asp Phe Tyr Val
       50                  55                  60
   Pro Val Ser Leu Cys Thr Pro Ser Arg Ala Ala Leu Leu Thr Gly Arg
   65                  70                  75                  80
   Leu Pro Val Arg Met Gly Met Tyr Pro Gly Val Leu Val Pro Ser Ser
                   85                  90                  95
   Arg Gly Gly Leu Pro Leu Glu Glu Val Thr Val Ala Glu Val Leu Ala
               100                 105                 110
   Ala Arg Gly Tyr Leu Thr Gly Met Ala Gly Lys Trp His Leu Gly Val
           115                 120                 125
   Gly Pro Glu Gly Ala Phe Leu Pro Pro His Gln Gly Phe His Arg Phe
       130                 135                 140
   Leu Gly Ile Pro Tyr Ser His Asp Gln Gly Pro Cys Gln Asn Leu Thr
   145                 150                 155                 160
   Cys Phe Pro Pro Ala Thr Pro Cys Asp Gly Gly Cys Asp Gln Gly Leu
                   165                 170                 175
   Val Pro Ile Pro Leu Leu Ala Asn Leu Ser Val Glu Ala Gln Pro Pro
               180                 185                 190
   Trp Leu Pro Gly Leu Glu Ala Arg Tyr Met Ala Phe Ala His Asp Leu
           195                 200                 205
   Met Ala Asp Ala Gln Arg Gln Asp Arg Pro Phe Phe Leu Tyr Tyr Ala
       210                 215                 220
   Ser His His Thr His Tyr Pro Gln Phe Ser Gly Gln Ser Phe Ala Glu
   225                 230                 235                 240
   Arg Ser Gly Arg Gly Pro Phe Gly Asp Ser Leu Met Glu Leu Asp Ala
                   245                 250                 255
   Ala Val Gly Thr Leu Met Thr Ala Ile Gly Asp Leu Gly Leu Leu Glu
               260                 265                 270
   Glu Thr Leu Val Ile Phe Thr Ala Asp Asn Gly Pro Glu Thr Met Arg
           275                 280                 285
   Met Ser Arg Gly Gly Cys Ser Gly Leu Leu Arg Cys Gly Lys Gly Thr
       290                 295                 300
   Thr Tyr Glu Gly Gly Val Arg Glu Pro Ala Leu Ala Phe Trp Pro Gly
   305                 310                 315                 320
   His Ile Ala Pro Gly Val Thr His Glu Leu Ala Ser Ser Leu Asp Leu
                   325                 330                 335
   Leu Pro Thr Leu Ala Ala Leu Ala Gly Ala Pro Leu Pro Asn Val Thr
               340                 345                 350
   Leu Asp Gly Phe Asp Leu Ser Pro Leu Leu Leu Gly Thr Gly Lys Ser
           355                 360                 365
   Pro Arg Gln Ser Leu Phe Phe Tyr Pro Ser Tyr Pro Asp Glu Val Arg
       370                 375                 380
   Gly Val Phe Ala Val Arg Thr Gly Lys Tyr Lys Ala His Phe Phe Thr
   385                 390                 395                 400
   Gln Gly Ser Ala His Ser Asp Thr Thr Ala Asp Pro Ala Cys His Ala
                   405                 410                 415
   Ser Ser Ser Leu Thr Ala His Glu Pro Pro Leu Leu Tyr Asp Leu Ser
               420                 425                 430
   Lys Asp Pro Gly Glu Asn Tyr Asn Leu Leu Gly Gly Val Ala Gly Ala
           435                 440                 445
   Thr Pro Glu Val Leu Gln Ala Leu Lys Gln Leu Gln Leu Leu Lys Ala
       450                 455                 460
   Gln Leu Asp Ala Ala Val Thr Phe Gly Pro Ser Gln Val Ala Arg Gly
   465                 470                 475                 480
   Glu Asp Pro Ala Leu Gln Ile Cys Cys His Pro Gly Cys Thr Pro Arg
   485 490 495
   Pro Ala Cys Cys His Cys Pro Asp Pro His Ala
   500 505
<210> 65
   <211> 2010
   <212> DNA
   <213> Homo sapiens
<400> 65
   atggctgcag ccgcgggttc ggcgggccgc gccgcggtgc ccttgctgct gtgtgcgctg 60
   ctggcgcccg gcggcgcgta cgtgctcgac gactccgacg ggctgggccg ggagttcgac 120
   ggcatcggcg cggtcagcgg cggcggggca acctcccgac ttctagtaaa ttacccagag 180
   ccctatcgtt ctcagatatt ggattatctc tttaagccga attttggtgc ctctttgcat 240
   attttaaaag tggaaatagg tggtgatggg cagacaacag atggcactga gccctcccac 300
   atgcattatg cactagatga gaattatttc cgaggatacg agtggtggtt gatgaaagaa 360
   gctaagaaga ggaatcccaa tattacactc attgggttgc catggtcatt ccctggatgg 420
   ctgggaaaag gtttcgactg gccttatgtc aatcttcagc tgactgccta ttatgtcgtg 480
   acctggattg tgggcgccaa gcgttaccat gatttggaca ttgattatat tggaatttgg 540
   aatgagaggt catataatgc caattatatt aagatattaa gaaaaatgct gaattatcaa 600
   ggtctccagc gagtgaaaat catagcaagt gataatctct gggagtccat ctctgcatcc 660
   atgctccttg atgccgaact cttcaaggtg gttgatgtta taggggctca ttatcctgga 720
   acccattcag caaaagatgc aaagttgact gggaagaagc tttggtcttc tgaagacttt 780
   agcactttaa atagtgacat gggtgcaggc tgctggggtc gcattttaaa tcagaattat 840
   atcaatggct atatgacttc cacaatcgca tggaatttag tggctagtta ctatgaacag 900
   ttgccttatg ggagatgcgg gttgatgacg gcccaggagc catggagtgg gcactacgtg 960
   gtagaatctc ctgtctgggt atcagctcat accactcagt ttactcaacc tggctggtat 1020
   tacctgaaga cagttggcca tttagagaaa ggaggaagct acgtagctct gactgatggc 1080
   ttagggaacc tcaccatcat cattgaaacc atgagtcata aacattctaa gtgcatacgg 1140
   ccatttcttc cttatttcaa tgtgtcacaa caatttgcca cctttgttct taagggatct 1200
   tttagtgaaa taccagagct acaggtatgg tataccaaac ttggaaaaac atccgaaaga 1260
   tttcttttta agcagctgga ttctctatgg ctccttgaca gcgatggcag tttcacactg 1320
   agcctgcatg aagatgagct gttcacactc accactctca ccactggtcg caaaggcagc 1380
   tacccgcttc ctccaaaatc ccagcccttc ccaagtacct ataaggatga tttcaatgtt 1440
   gattacccat tttttagtga agctccaaac tttgctgatc aaactggtgt atttgaatat 1500
   tttacaaata ttgaagaccc tggcgagcat cacttcacgc tacgccaagt tctcaaccag 1560
   agacccatta cgtgggctgc cgatgcatcc aacacaatca gtattatagg agactacaac 1620
   tggaccaatc tgactacaaa gtgtgatgtt tacatagaga cccctgacac aggaggtgtg 1680
   ttcattgcag gaagagtaaa taaaggtggt attttgatta gaagtgccag aggaattttc 1740
   ttctggattt ttgcaaatgg atcttacagg gttacaggtg atttagctgg atggattata 1800
   tatgctttag gacgtgttga agttacagca aaaaaatggt atacactcac gttaactatt 1860
   aagggtcatt tcgcctctgg catgctgaat gacaagtctc tgtggacaga catccctgtg 1920
   aattttccaa agaatggctg ggctgcaatt ggaactcact cctttgaatt tgcacagttt 1980
   gacaactttc ttgtggaagc cacacgctaa 2010
<210> 66
   <211> 669
   <212> PRT
   <213> Homo sapiens
<400> 66
   Met Ala Ala Ala Ala Gly Ser Ala Gly Arg Ala Ala Val Pro Leu Leu
   1 5 10 15
   Leu Cys Ala Leu Leu Ala Pro Gly Gly Ala Tyr Val Leu Asp Asp Ser
   20 25 30
   Asp Gly Leu Gly Arg Glu Phe Asp Gly Ile Gly Ala Val Ser Gly Gly
   35 40 45
   Gly Ala Thr Ser Arg Leu Leu Val Asn Tyr Pro Glu Pro Tyr Arg Ser
   50 55 60
   Gln Ile Leu Asp Tyr Leu Phe Lys Pro Asn Phe Gly Ala Ser Leu His
   65 70 75 80
   Ile Leu Lys Val Glu Ile Gly Gly Asp Gly Gln Thr Thr Asp Gly Thr
   85 90 95
   Glu Pro Ser His Met His Tyr Ala Leu Asp Glu Asn Tyr Phe Arg Gly
   100 105 110
   Tyr Glu Trp Trp Leu Met Lys Glu Ala Lys Lys Arg Asn Pro Asn Ile
   115 120 125
   Thr Leu Ile Gly Leu Pro Trp Ser Phe Pro Gly Trp Leu Gly Lys Gly
   130 135 140
   Phe Asp Trp Pro Tyr Val Asn Leu Gln Leu Thr Ala Tyr Tyr Val Val
   145 150 155 160
   Thr Trp Ile Val Gly Ala Lys Arg Tyr His Asp Leu Asp Ile Asp Tyr
   165 170 175
   Ile Gly Ile Trp Asn Glu Arg Ser Tyr Asn Ala Asn Tyr Ile Lys Ile
   180 185 190
   Leu Arg Lys Met Leu Asn Tyr Gln Gly Leu Gln Arg Val Lys Ile Ile
   195 200 205
   Ala Ser Asp Asn Leu Trp Glu Ser Ile Ser Ala Ser Met Leu Leu Asp
   210 215 220
   Ala Glu Leu Phe Lys Val Val Asp Val Ile Gly Ala His Tyr Pro Gly
   225 230 235 240
   Thr His Ser Ala Lys Asp Ala Lys Leu Thr Gly Lys Lys Leu Trp Ser
   245 250 255
   Ser Glu Asp Phe Ser Thr Leu Asn Ser Asp Met Gly Ala Gly Cys Trp
   260 265 270
   Gly Arg Ile Leu Asn Gln Asn Tyr Ile Asn Gly Tyr Met Thr Ser Thr
   275 280 285
   Ile Ala Trp Asn Leu Val Ala Ser Tyr Tyr Glu Gln Leu Pro Tyr Gly
   290 295 300
   Arg Cys Gly Leu Met Thr Ala Gln Glu Pro Trp Ser Gly His Tyr Val
   305 310 315 320
   Val Glu Ser Pro Val Trp Val Ser Ala His Thr Thr Gln Phe Thr Gln
   325 330 335
   Pro Gly Trp Tyr Tyr Leu Lys Thr Val Gly His Leu Glu Lys Gly Gly
   340 345 350
   Ser Tyr Val Ala Leu Thr Asp Gly Leu Gly Asn Leu Thr Ile Ile Ile
   355 360 365
   Glu Thr Met Ser His Lys His Ser Lys Cys Ile Arg Pro Phe Leu Pro
   370 375 380
   Tyr Phe Asn Val Ser Gln Gln Phe Ala Thr Phe Val Leu Lys Gly Ser
   385 390 395 400
   Phe Ser Glu Ile Pro Glu Leu Gln Val Trp Tyr Thr Lys Leu Gly Lys
   405 410 415
   Thr Ser Glu Arg Phe Leu Phe Lys Gln Leu Asp Ser Leu Trp Leu Leu
   420 425 430
   Asp Ser Asp Gly Ser Phe Thr Leu Ser Leu His Glu Asp Glu Leu Phe
   435 440 445
   Thr Leu Thr Thr Leu Thr Thr Gly Arg Lys Gly Ser Tyr Pro Leu Pro
   450 455 460
   Pro Lys Ser Gln Pro Phe Pro Ser Thr Tyr Lys Asp Asp Phe Asn Val
   465 470 475 480
   Asp Tyr Pro Phe Phe Ser Glu Ala Pro Asn Phe Ala Asp Gln Thr Gly
   485 490 495
   Val Phe Glu Tyr Phe Thr Asn Ile Glu Asp Pro Gly Glu His His Phe
   500 505 510
   Thr Leu Arg Gln Val Leu Asn Gln Arg Pro Ile Thr Trp Ala Ala Asp
   515 520 525
   Ala Ser Asn Thr Ile Ser Ile Ile Gly Asp Tyr Asn Trp Thr Asn Leu
   530 535 540
   Thr Thr Lys Cys Asp Val Tyr Ile Glu Thr Pro Asp Thr Gly Gly Val
   545 550 555 560
   Phe Ile Ala Gly Arg Val Asn Lys Gly Gly Ile Leu Ile Arg Ser Ala
   565 570 575
   Arg Gly Ile Phe Phe Trp Ile Phe Ala Asn Gly Ser Tyr Arg Val Thr
   580 585 590
   Gly Asp Leu Ala Gly Trp Ile Ile Tyr Ala Leu Gly Arg Val Glu Val
   595 600 605
   Thr Ala Lys Lys Trp Tyr Thr Leu Thr Leu Thr Ile Lys Gly His Phe
   610 615 620
   Ala Ser Gly Met Leu Asn Asp Lys Ser Leu Trp Thr Asp Ile Pro Val
   625 630 635 640
   Asn Phe Pro Lys Asn Gly Trp Ala Ala Ile Gly Thr His Ser Phe Glu
   645 650 655
   Phe Ala Gln Phe Asp Asn Phe Leu Val Glu Ala Thr Arg
   660 665
<210> 67
   <211> 1611
   <212> DNA
   <213> Homo sapiens
<400> 67
   atggagtttt caagtccttc cagagaggaa tgtcccaagc ctttgagtag ggtaagcatc      60
   atggctggca gcctcacagg attgcttcta cttcaggcag tgtcgtgggc atcaggtgcc     120
   cgcccctgca tccctaaaag cttcggctac agctcggtgg tgtgtgtctg caatgccaca     180
   tactgtgact cctttgaccc cccgaccttt cctgcccttg gtaccttcag ccgctatgag     240
   agtacacgca gtgggcgacg gatggagctg agtatggggc ccatccaggc taatcacacg     300
   ggcacaggcc tgctactgac cctgcagcca gaacagaagt tccagaaagt gaagggattt     360
   ggaggggcca tgacagatgc tgctgctctc aacatccttg ccctgtcacc ccctgcccaa     420
   aatttgctac ttaaatcgta cttctctgaa gaaggaatcg gatataacat catccgggta     480
   cccatggcca gcagcgactt ctccatccgc acctacacct atgcagacac ccctgatgat     540
   ttccagttgc acaacttcag cctcccagag gaagatacca agctcaagat acccctgatt     600
   caccgagccc tgcagttggc ccagcgtccc gtttcactcc ttgccagccc ctggacatca     660
   cccacttggc tcaagaccaa tggagcggtg aatgggaagg ggtcactcaa gggacagccc     720
   ggagacatct accaccagac ctgggccaga tactttgtga agttcctgga tgcctatgct     780
   gagcacaagt tacagttctg ggcagtgaca gctgaaaatg agccttctgc tgggctgttg     840
   agtggatacc ccttccagag cctgggcttc acccctgaac atcagcgaga cttcattgcc     900
   cgtgacctag gtcctaccct cgccaacagt actcaccaca atgtccgcct actcatgctg     960
   gatgaccaac gcttgctgct gccccactgg gcaaaggtgg tactgacaga cccagaagca    1020
   gctaaatatg ttcatggcat tgctgtacat tggtacctgg actttctggc tccagccaaa    1080
   gccaccctag gggagacaca ccgcctgttc cccaacacca tgctctttgc ctcagaggcc    1140
   agcgtgggct ccaagttctg ggagcagagt gtgcggctag gctcctggga tcgagggatg    1200
   cagtacagcc acagcatcat cacgaacctc ctgtaccatg tggtcggctg gaccgactgg    1260
   aaccttgccc tgaaccccga aggaggaccc aattgggtgc gtaactttgt cgacagtccc    1320
   atcattgtag acatcaccaa ggacacgttt tacaaacagc ccatgttcta ccaccttggc    1380
   cacttcagca agttcattcc tgagggctcc cagagagtgg ggctggttgc cagtcagaag    1440
   aacgacctgg acgcagtggc actgatgcat cccgatggct ctgctgttgt ggtcgtgcta    1500
   aaccgctcct ctaaggatgt gcctcttacc atcaaggatc ctgctgtggg cttcctggag    1560
   acaatctcac ctggctactc cattcacacc tacctgtggc gtcgccagtg a             1611
<210> 68
   <211> 538
   <212> PRT
   <213> Homo sapiens
<400> 68
   Ala Ser Met Glu Phe Ser Ser Pro Ser Arg Glu Glu Cys Pro Lys Pro
   1               5                   10                  15
   Leu Ser Arg Val Ser Ile Met Ala Gly Ser Leu Thr Gly Leu Leu Leu
               20                  25                  30
   Leu Gln Ala Val Ser Trp Ala Ser Gly Ala Arg Pro Cys Ile Pro Lys
           35                  40                  45
   Ser Phe Gly Tyr Ser Ser Val Val Cys Val Cys Asn Ala Thr Tyr Cys
       50                  55                  60
   Asp Ser Phe Asp Pro Pro Thr Phe Pro Ala Leu Gly Thr Phe Ser Arg
   65                  70                  75                  80
   Tyr Glu Ser Thr Arg Ser Gly Arg Arg Met Glu Leu Ser Met Gly Pro
                   85                  90                  95
   Ile Gln Ala Asn His Thr Gly Thr Gly Leu Leu Leu Thr Leu Gln Pro
               100                 105                 110
   Glu Gln Lys Phe Gln Lys Val Lys Gly Phe Gly Gly Ala Met Thr Asp
           115                 120                 125
   Ala Ala Ala Leu Asn Ile Leu Ala Leu Ser Pro Pro Ala Gln Asn Leu
       130                 135                 140
   Leu Leu Lys Ser Tyr Phe Ser Glu Glu Gly Ile Gly Tyr Asn Ile Ile
   145                 150                 155                 160
   Arg Val Pro Met Ala Ser Ser Asp Phe Ser Ile Arg Thr Tyr Thr Tyr
                   165                 170                 175
   Ala Asp Thr Pro Asp Asp Phe Gln Leu His Asn Phe Ser Leu Pro Glu
               180                 185                 190
   Glu Asp Thr Lys Leu Lys Ile Pro Leu Ile His Arg Ala Leu Gln Leu
           195                 200                 205
   Ala Gln Arg Pro Val Ser Leu Leu Ala Ser Pro Trp Thr Ser Pro Thr
       210                 215                 220
   Trp Leu Lys Thr Asn Gly Ala Val Asn Gly Lys Gly Ser Leu Lys Gly
   225                 230                 235                 240
   Gln Pro Gly Asp Ile Tyr His Gln Thr Trp Ala Arg Tyr Phe Val Lys
                   245                 250                 255
   Phe Leu Asp Ala Tyr Ala Glu His Lys Leu Gln Phe Trp Ala Val Thr
               260                 265                 270
   Ala Glu Asn Glu Pro Ser Ala Gly Leu Leu Ser Gly Tyr Pro Phe Gln
           275                 280                 285
   Ser Leu Gly Phe Thr Pro Glu His Gln Arg Asp Phe Ile Ala Arg Asp
       290                 295                 300
   Leu Gly Pro Thr Leu Ala Asn Ser Thr His His Asn Val Arg Leu Leu
   305                 310                 315                 320
   Met Leu Asp Asp Gln Arg Leu Leu Leu Pro His Trp Ala Lys Val Val
                   325                 330                 335
   Leu Thr Asp Pro Glu Ala Ala Lys Tyr Val His Gly Ile Ala Val His
               340                 345                 350
   Trp Tyr Leu Asp Phe Leu Ala Pro Ala Lys Ala Thr Leu Gly Glu Thr
           355                 360                 365
   His Arg Leu Phe Pro Asn Thr Met Leu Phe Ala Ser Glu Ala Ser Val
       370                 375                 380
   Gly Ser Lys Phe Trp Glu Gln Ser Val Arg Leu Gly Ser Trp Asp Arg
   385                 390                 395                 400
   Gly Met Gln Tyr Ser His Ser Ile Ile Thr Asn Leu Leu Tyr His Val
                   405                 410                 415
   Val Gly Trp Thr Asp Trp Asn Leu Ala Leu Asn Pro Glu Gly Gly Pro
               420                 425                 430
   Asn Trp Val Arg Asn Phe Val Asp Ser Pro Ile Ile Val Asp Ile Thr
           435                 440                 445
   Lys Asp Thr Phe Tyr Lys Gln Pro Met Phe Tyr His Leu Gly His Phe
       450                 455                 460
   Ser Lys Phe Ile Pro Glu Gly Ser Gln Arg Val Gly Leu Val Ala Ser
   465                 470                 475                 480
   Gln Lys Asn Asp Leu Asp Ala Val Ala Leu Met His Pro Asp Gly Ser
                   485                 490                 495
   Ala Val Val Val Val Leu Asn Arg Ser Ser Lys Asp Val Pro Leu Thr
               500                 505                 510
   Ile Lys Asp Pro Ala Val Gly Phe Leu Glu Thr Ile Ser Pro Gly Tyr
   515 520 525
   Ser Ile His Thr Tyr Leu Trp Arg Arg Gln
   530 535
<210> 69
   <211> 1320
   <212> DNA
   <213> Homo sapiens
<400> 69
   atgcagctga ggaacccaga actacatctg ggctgcgcgc ttgcgcttcg cttcctggcc      60
   ctcgtttcct gggacatccc tggggctaga gcactggaca atggattggc aaggacgcct     120
   accatgggct ggctgcactg ggagcgcttc atgtgcaacc ttgactgcca ggaagagcca     180
   gattcctgca tcagtgagaa gctcttcatg gagatggcag agctcatggt ctcagaaggc     240
   tggaaggatg caggttatga gtacctctgc attgatgact gttggatggc tccccaaaga     300
   gattcagaag gcagacttca ggcagaccct cagcgctttc ctcatgggat tcgccagcta     360
   gctaattatg ttcacagcaa aggactgaag ctagggattt atgcagatgt tgggaataaa     420
   acctgcgcag gcttccctgg gagttttgga tactacgaca ttgatgccca gacctttgct     480
   gactggggag tagatctgct aaaatttgat ggttgttact gtgacagttt ggaaaatttg     540
   gcagatggtt ataagcacat gtccttggcc ctgaatagga ctggcagaag cattgtgtac     600
   tcctgtgagt ggcctcttta tatgtggccc tttcaaaagc ccaattatac agaaatccga     660
   cagtactgca atcactggcg aaattttgct gacattgatg attcctggaa aagtataaag     720
   agtatcttgg actggacatc ttttaaccag gagagaattg ttgatgttgc tggaccaggg     780
   ggttggaatg acccagatat gttagtgatt ggcaactttg gcctcagctg gaatcagcaa     840
   gtaactcaga tggccctctg ggctatcatg gctgctcctt tattcatgtc taatgacctc     900
   cgacacatca gccctcaagc caaagctctc cttcaggata aggacgtaat tgccatcaat     960
   caggacccct tgggcaagca agggtaccag cttagacagg gagacaactt tgaagtgtgg    1020
   gaacgacctc tctcaggctt agcctgggct gtagctatga taaaccggca ggagattggt    1080
   ggacctcgct cttataccat cgcagttgct tccctgggta aaggagtggc ctgtaatcct    1140
   gcctgcttca tcacacagct cctccctgtg aaaaggaagc tagggttcta tgaatggact    1200
   tcaaggttaa gaagtcacat aaatcccaca ggcactgttt tgcttcagct agaaaataca    1260
   atgcagatgt cattaaaaga cttactttaa atgcagatgt cattaaaaga cttactttaa    1320
<210> 70
   <211> 429
   <212> PRT
   <213> Homo sapiens
<400> 70
<210> 71
   <211> 92
   <212> PRT
   <213> Homo sapiens
<400> 71
   Met Gln Pro Ser Ser Leu Leu Pro Leu Ala Leu Cys Leu Leu Ala Ala
   1 5 10 15
   Pro Ala Gly Ser Ser Lys Pro Gln Ala Leu Ala Thr Pro Asn Lys Glu
   20 25 30
   Glu His Gly Lys Arg Lys Lys Lys Gly Lys Gly Leu Gly Lys Lys Arg
   35 40 45
   Asp Pro Cys Leu Arg Lys Tyr Lys Asp Phe Cys Ile His Gly Glu Cys
   50 55 60
   Lys Tyr Val Lys Glu Leu Arg Ala Pro Ser Cys Ile Cys His Pro Gly
   65 70 75 80
   Tyr His Gly Glu Arg Cys His Gly Leu Ser Gly Ser
   85 90
<210> 72
   <211> 47
   <212> PRT
   <213> Homo sapiens
<400> 72
   Met Gln Pro Ser Ser Leu Leu Pro Leu Ala Leu Cys Leu Leu Ala Ala
   1 5 10 15
   Pro Ala Gly Ser Gly Lys Arg Lys Lys Lys Gly Lys Gly Leu Gly Lys
   20 25 30
   Lys Arg Asp Pro Ser Leu Arg Lys Tyr Lys Asp Phe Ser Gly Ser
   35 40 45
<210> 73
   <211> 30
   <212> DNA
   <213> Artificial Sequence
<220>
   <223> N-terminal Linker
<400> 73
   agatccgtcg acatcgaagg tagcggcatt 30
<210> 74
   <211> 30
   <212> DNA
   <213> Artificial Sequence
<220>
   <223> N-terminal Linker
<400> 74
   ggatccgtcg acatcgaagg tagcggcatt 30